# EUROPEAN PATENT APPLICATION

(11) **EP 3 673 732 A2**
(43) Date of publication of application: **01.07.2020**
(21) Application number: 20154422.8
(22) Date of filing: 27.10.2016
(51) Int. Cl.: A01K 67/027, C12N 15/09, C12N 5/10, C12N 5/12

(54) **ENGINEERING OF HUMANIZED CAR T-CELLS AND PLATELETS BY GENETIC COMPLEMENTATION**

(30) Priority: 27.10.2015 US 201562247114 P; 27.10.2015 US 201562247124 P
(62) Divisional of application: 16860819.8
(71) Applicant: Recombinetics, Inc., Saint Paul, MN 55104 (US)
(72) Inventor: FAHRENKRUG, Scott, C, Minneapolis, MN 55406 (US); COOPER, Laurence, Park City, UT 84060 (US); HACKETT, Perry, B, Shoreview, MN 55126 (US); CARLSON, Daniel, F, Woodbury, MN 55125 (US)
(74) Representative: Handley, Matthew Edward

(57) **Abstract**

Human or humanized tissues and organs suitable for transplant are disclosed herein. Gene editing of a host animal to knock out or disrupt genes responsible for the growth and/or differentiation of a target organ provides a niche for complementation of the missing genetic information by injecting that animal at an embryo stage with donor stem cells to complement the missing genetic information for the growth and development of the organ. The result is a chimeric animal in which the complemented tissue (human/humanized organ) matches the genotype and phenotype of the donor. Such organs may be made in a single generation and the stem cell may be taken or generated from the patient's own body. Multiple genes can be targeted for editing using targeted nucleases and homology directed repair (HOR) templates in vertebrate cells or embryos.

SEQUENCE LISTING

<110>  Fahrenkrug, Scott
       Cooper, Laurence
       Carlson, Daniel

<120>  ENGINEERING OF HUMANIZED CAR T-CELLS AND PLATELETS BY GENETIC
       COMPLEMENTATION

<130>  5054.52WO01

<140>  PCT/US201659191
<141>  2016-10-27

<150>  62/247,114
<151>  2015-10-27

<150>  62/247,124
<151>  2015-10-27

<160>  47

<170>  PatentIn version 3.5

<210>  1
<211>  90
<212>  DNA
<213>  ARTIFICIAL

<220>
<223>  HDR TEMPLATE FOR SUS SCROFA

<400>  1
ctcttttcgc aggcacaggt ctacgagctg gagcgacgct tctaagcttg cagcagcggt      60

acctgtcggc tcccgagcgt gaccagttgg                                      90


<210>  2
<211>  90
<212>  DNA
<213>  ARTIFICIAL

<220>
<223>  HDR TEMPLATE FOR SUS SCROFA

<400>  2
aaccctgtgt cgtttcccac ccagtagata tgtttgatga ctaagcttct cggaaggcag      60

agagtgtgtc aactgcgggg ccatgtccac                                      90


<210>  3
<211>  60
<212>  DNA
<213>  ARTIFICIAL

<220>
<223>  HDR TEMPLATE FOR SUS SCROFA

<400>  3
tgcggttgct cccccgcctc gtccccgtaa gcttgactcc tggtgcagcg ccccggccag      60

<210>  4
<211>  36
<212>  PRT
<213>  ARTIFICIAL

<220>
<223>  TALEN

<400>  4

His Asp His Asp His Asp Asn Ile Asn Ile Asn Ile Asn Asn Asn Asn
1               5                   10                  15

Asn Gly Asn Gly His Asp Asn Ile Asn Asn Asn Gly Asn Asn Asn Gly
            20                  25                  30

Asn Gly Asn Gly
        35

<210>  5
<211>  40
<212>  PRT
<213>  ARTIFICIAL

<220>
<223>  TALEN

<400>  5

His Asp His Asp Asn Ile Asn Ile Asn Asn Asn Gly Asn Asn His Asp
1               5                   10                  15

Asn Ile Asn Ile Asn Gly Asn Gly His Asp Asn Ile Asn Gly Asn Asn
            20                  25                  30

Asn Gly Asn Ile His Asp Asn Gly
        35                  40

<210>  6
<211>  36
<212>  PRT
<213>  ARTIFICIAL

<220>
<223>  TALEN

<400>  6

Asn Ile His Asp His Asp Asn Gly Asn Gly His Asp His Asp Asn Gly
1               5                   10                  15

His Asp His Asp Asn Gly His Asp Asn Gly His Asp His Asp Asn Asn
            20                  25                  30

His Asp Asn Gly
        35

<210>  7
<211>  34
<212>  PRT
<213>  ARTIFICIAL

<220>
<223>  SEQ ID NO 8

<400>  7

His Asp Asn Gly Asn Ile Asn Ile Asn Asn His Asp Asn Gly Asn Asn
1               5                   10                  15

His Asp Asn Gly Asn Gly Asn Gly Asn Gly Asn Asn Asn Ile Asn Ile
            20                  25                  30

Asn Gly

<210>  8
<211>  36
<212>  PRT
<213>  ARTIFICIAL

<220>
<223>  TALEN

<400>  8

Asn Asn Asn Asn Asn Ile Asn Ile Asn Asn Asn Ile Asn Ile Asn Asn
1               5                   10                  15

Asn Gly Asn Ile Asn Gly His Asp Asn Ile Asn Asn His Asp His Asp
            20                  25                  30

Asn Ile Asn Gly
        35

<210>  9
<211>  32
<212>  PRT
<213>  ARTIFICIAL

<220>
<223>  TALEN

<400>  9

Asn Asn Asn Ile His Asp His Asp His Asp Asn Ile Asn Asn Asn Ile
1               5                   10                  15

Asn Ile Asn Gly Asn Gly Asn Gly His Asp Asn Gly Asn Asn Asn Gly

20                  25                  30

<210>  10
<211>  34
<212>  PRT
<213>  ARTIFICIAL

<220>
<223>  SEQ ID NO 11

<400>  10

Asn Asn Asn Asn His Asp Asn Ile His Asp His Asp His Asp Asn Asn
1               5                   10                  15

Asn Gly Asn Asn Asn Gly His Asp His Asp Asn Asn His Asp Asn Asn
            20                  25                  30

His Asp

<210>  11
<211>  30
<212>  PRT
<213>  ARTIFICIAL

<220>
<223>  TALEN

<400>  11

His Asp Asn Ile Asn Gly Asn Asn Asn Gly Asn Ile His Asp Asn Gly
1               5                   10                  15

His Asp Asn Gly Asn Asn Asn Ile His Asp Asn Gly Asn Gly
            20                  25                  30

<210>  12
<211>  30
<212>  PRT
<213>  ARTIFICIAL

<220>
<223>  TALEN

<400>  12

Asn Asn His Asp Asn Gly His Asp Asn Gly Asn Ile His Asp Asn Gly
1               5                   10                  15

His Asp Asn Gly Asn Ile His Asp His Asp His Asp His Asp
            20                  25                  30

<210>  13
<211>  34

<212>  PRT
<213>  ARTIFICIAL

<220>
<223>  TALEN

<400>  13

Asn Asn His Asp Asn Ile His Asp Asn Ile Asn Gly Asn Asn Asn Ile
1               5                   10                  15

Asn Ile Asn Asn Asn Gly His Asp Asn Asn His Asp His Asp His Asp
            20                  25                  30

Asn Ile

<210>  14
<211>  36
<212>  PRT
<213>  ARTIFICIAL

<220>
<223>  TALEN

<400>  14

His Asp His Asp Asn Asn Asn Gly His Asp His Asp Asn Gly Asn Gly
1               5                   10                  15

Asn Gly Asn Asn His Asp His Asp Asn Ile Asn Ile His Asp His Asp
            20                  25                  30

Asn Gly Asn Gly
        35

<210>  15
<211>  36
<212>  PRT
<213>  ARTIFICIAL

<220>
<223>  TALEN

<400>  15

Asn Gly Asn Asn Asn Asn Asn Asn Asn Asn Asn Asn His Asp His Asp
1               5                   10                  15

His Asp Asn Ile His Asp Asn Asn Asn Asn Asn Gly Asn Gly Asn Asn
            20                  25                  30

His Asp Asn Gly
        35

<210>  16
<211>  34
<212>  PRT
<213>  ARTIFICIAL

<220>
<223>  TALEN

<400>  16

His Asp Asn Gly His Asp His Asp Asn Gly Asn Ile His Asp Asn Ile
1               5                   10                  15

Asn Ile Asn Asn Asn Gly Asn Asn Asn Asn Asn Ile Asn Gly Asn Gly
            20                  25                  30

Asn Gly

<210>  17
<211>  36
<212>  PRT
<213>  ARTIFICIAL

<220>
<223>  TALEN

<400>  17

His Asp Asn Asn Asn Asn Asn Ile His Asp His Asp His Asp Asn Asn
1               5                   10                  15

Asn Gly His Asp His Asp Asn Gly Asn Gly Asn Asn His Asp Asn Ile
            20                  25                  30

His Asp Asn Gly
        35

<210>  18
<211>  30
<212>  PRT
<213>  ARTIFICIAL

<220>
<223>  TALEN

<400>  18

Asn Asn Asn Asn Asn Ile His Asp Asn Gly Asn Asn Asn Ile His Asp
1               5                   10                  15

His Asp Asn Ile His Asp Asn Gly Asn Ile Asn Gly Asn Gly
            20                  25                  30

<210>  19
<211>  34
<212>  PRT
<213>  ARTIFICIAL

<220>
<223>  TALEN

<400>  19

Asn Ile Asn Asn Asn Ile Asn Asn Asn Ile Asn Ile Asn Gly Asn Asn
1               5                   10                  15

Asn Gly Asn Asn Asn Asn Asn Gly His Asp His Asp Asn Ile Asn Asn
            20                  25                  30

His Asp

<210>  20
<211>  34
<212>  PRT
<213>  ARTIFICIAL

<220>
<223>  TALEN

<400>  20

His Asp Asn Asn His Asp Asn Ile Asn Asn Asn Asn His Asp Asn Ile
1               5                   10                  15

His Asp Asn Ile Asn Asn Asn Asn Asn Gly His Asp Asn Gly Asn Ile
            20                  25                  30

His Asp

<210>  21
<211>  30
<212>  PRT
<213>  ARTIFICIAL

<220>
<223>  TALEN

<400>  21

Asn Ile His Asp His Asp Asn Asn His Asp Asn Gly Asn Asn His Asp
1               5                   10                  15

Asn Gly Asn Asn His Asp Asn Gly Asn Gly Asn Asn Asn Ile
            20                  25                  30

<210>  22

<211>  34
<212>  PRT
<213>  ARTIFICIAL

<220>
<223>  TALEN

<400>  22

Asn Asn His Asp Asn Asn Asn Asn Asn Gly Asn Gly Asn Asn His Asp
1               5                   10                  15

Asn Gly His Asp His Asp His Asp His Asp His Asp Asn Asn His Asp
            20                  25                  30

His Asp

<210>  23
<211>  34
<212>  PRT
<213>  ARTIFICIAL

<220>
<223>  TALEN

<400>  23

Asn Asn Asn Asn His Asp His Asp Asn Asn Asn Asn Asn Asn Asn Asn
1               5                   10                  15

His Asp Asn Asn His Asp Asn Gly Asn Asn His Asp Asn Ile His Asp
            20                  25                  30

His Asp

<210>  24
<211>  30
<212>  PRT
<213>  ARTIFICIAL

<220>
<223>  TALEN

<400>  24

Asn Ile Asn Gly Asn Asn Asn Gly Asn Gly Asn Gly Asn Asn Asn Ile
1               5                   10                  15

Asn Gly Asn Asn Asn Ile His Asp Asn Gly Asn Gly His Asp
            20                  25                  30

<210>  25
<211>  34

<212>  PRT
<213>  ARTIFICIAL

<220>
<223>  TALEN

<400>  25

Asn Asn Asn Asn His Asp His Asp His Asp His Asp Asn Asn His Asp
1               5                   10                  15

Asn Ile Asn Asn Asn Gly Asn Gly Asn Asn Asn Ile His Asp Asn Ile
            20                  25                  30

His Asp

<210>  26
<211>  90
<212>  DNA
<213>  ARTIFICIAL

<220>
<223>  HDR TEMPLATE

<400>  26
ttccactcta ccccccccaa aggttcagtg ttttgtgtaa gcttcaatgt tgagtacatg      60

aattgcactt gggacagcag ctctgagctc                                      90

<210>  27
<211>  90
<212>  DNA
<213>  ARTIFICIAL

<220>
<223>  HDR TEMPLATE

<400>  27
ctctaaggat tcctgccacc ttcctcctct ccgctaccca gactaagctt tgcacattca      60

aaagcagctt agggtctgaa aaacatcagt                                      90

<210>  28
<211>  90
<212>  DNA
<213>  ARTIFICIAL

<220>
<223>  HDR TEMPLATE

<400>  28
ccagatcgcc aaagtcacgg aagaagtatc agccattcat ccctcccagt gaagcttaca      60

gaaattctgg gtcgaccacg gagttgcact                                      90

<210>  29

<211>  90
<212>  DNA
<213>  ARTIFICIAL

<220>
<223>  HDR TEMPLATE

<400>  29
agctcgccac ccccgccggg cacccgtgtc cgcgccatgg ccatctaagc ttaaagaagt      60

cagagtacat gcccgaggtg gtgaggcgct                                        90


<210>  30
<211>  90
<212>  DNA
<213>  ARTIFICIAL

<220>
<223>  HDR TEMPLATE

<400>  30
gtgctgcgtg ccctttactg ctctactcta ccccctacca gcctaagctt gtgctgggcg      60

acttcatgtg caagttcctc aactacatcc                                        90


<210>  31
<211>  90
<212>  DNA
<213>  ARTIFICIAL

<220>
<223>  HDR TEMPLATE

<400>  31
cccagacttc actccgtcct ttgccgactt cggccgacca gcccttagca accgtgggcc      60

cccaaggggt gggccaggtg gggagctgcc                                        90


<210>  32
<211>  90
<212>  DNA
<213>  ARTIFICIAL

<220>
<223>  HDR TEMPLATE

<400>  32
ccgagacggg aaatgcacct cctacaagtg gatttgtgat ggatccgaac accgagtgca      60

aggacgggtc cgctgagtcc ctggagacgt                                        90


<210>  33
<211>  90
<212>  DNA
<213>  ARTIFICIAL

<220>
<223>  HDR TEMPLATE

<400>  33
aaagtggcct ggcccaaccc ctggactgac cactcgagta ttgaagcacg taagtatgct      60

ggaccacatt ctctatggct gtagacattc                                        90


<210>  34
<211>  90
<212>  DNA
<213>  ARTIFICIAL

<220>
<223>  HDR TEMPLATE

<400>  34
ctcttttcgc aggcacaggt ctacgagctg gagcgacgct tctaagcttg cagcagcggt      60

acctgtcggc tcccgagcgt gaccagttgg                                        90


<210>  35
<211>  60
<212>  DNA
<213>  ARTIFICIAL

<220>
<223>  HDR TEMPLATE

<400>  35
tgcggttgct cccccgcctc gtccccgtaa gcttgactcc tggtgcagcg ccccggccag      60


<210>  36
<211>  90
<212>  DNA
<213>  ARTIFICIAL

<220>
<223>  HDR TEMPLATE

<400>  36
aaccctgtgt cgtttcccac ccagtagata tgtttgatga ctaagcttct cggaaggcag      60

agagtgtgtc aactgcgggg ccatgtccac                                        90


<210>  37
<211>  90
<212>  DNA
<213>  ARTIFICIAL

<220>
<223>  HDR TEMPLATE

<400>  37
gctcccactc ccctgggggc ctctgggctc accaacggtc tcctcccggg ggacgaagac      60

ttctcctcca ttgcggacat ggacttctca                                        90


<210>  38
<211>  23
<212>  DNA

<213>  ARTIFICIAL

<220>
<223>  Cas9/CRISPR, sgRNA

<400>  38
cgtcaccaac ggtctcctct cgg                                              23


<210>  39
<211>  60
<212>  DNA
<213>  Artificial Sequence

<220>
<223>  Synthetic TALEN

<400>  39
gccgctgtct actgtgggcc tgcaaggcgt gcaaacgcaa gaccactaac gccgaccgcc      60


<210>  40
<211>  60
<212>  DNA
<213>  Artificial Sequence

<220>
<223>  Sythetic TALEN

<400>  40
gccactgcct catgtgggcc tgcaaagcgt gcaagaggaa atccaccacc atggatcggc      60


<210>  41
<211>  59
<212>  DNA
<213>  Artificial Sequence

<220>
<223>  Synthetic TALEN

<400>  41
cccctgccag gaccaaatgc ccccggaagc tgggagcgac agcagtggag aggaacatg       59


<210>  42
<211>  50
<212>  DNA
<213>  Artificial Sequence

<220>
<223>  Synthetic Sequence

<400>  42
ccgctgtcta ctgtgggcct gcaaggcgtg caaacgcaag accactaacg                 50


<210>  43
<211>  55
<212>  DNA
<213>  Artificial Sequence

<220>

<223>  Sythetic Sequence

<400>  43
ccgctgtcta ctgtgggcct gctaagcttg gcgtgcaaac gcaagaccac taacg          55


<210>  44
<211>  39
<212>  DNA
<213>  Artificial Sequence

<220>
<223>  Synthetic Sequence

<400>  44
atgtgggcct gcaaagcgtg caagaggaaa tccaccacc                            39


<210>  45
<211>  31
<212>  DNA
<213>  Artificial Sequence

<220>
<223>  Synthetic Sequence

<400>  45
atgtgggcct gcaaagagga aatccaccac c                                    31


<210>  46
<211>  42
<212>  DNA
<213>  Artificial Sequence

<220>
<223>  Synthetic Sequence

<400>  46
gatggtatcc tgtccccctg ccaggaccaa atgcccccgg aa                         42


<210>  47
<211>  46
<212>  DNA
<213>  Artificial Sequence

<220>
<223>  Synthetic Sequence

<400>  47
gatggtatcc tgtcccccta agcttcagga ccaaatgccc ccggaa                    46

## Description

### CROSS REFERENCE TO RELATED APPLICATIONS

This application claims priority to U.S. Provisional application Nos. 62/247,114 and 62/247,124 each filed October 27, 2015 and both hereby incorporated by reference in their entirety.

### TECHNICAL FIELD

The technical field relates to engineering and production of humanized organs and tissues in animals by genetic complementation.

### BACKGROUND

In the past 100 years scientists and physicians have been spectacularly effective in keeping people alive and healthy, at least until the last decades of their lives when a panoply of old-age diseases and disorders set in. Over $1 trillion dollars are spent annually in the United States for treatment of these diseases. Organ transplant can be effective but there are far too few and in many cases immunological mismatches lead to problems. For example, over 7,000 Americans died while awaiting an organ transplant since 2003.

Genetic complementation of animal somatic cells by various stem cells allows for the engineering and production of humanized tissues and organs for use in therapy, transplant and regenerative medicine. Currently the source of organs for transplantation are either mechanical or biological coming from human donors, cadavers and in limited cases are xenotranplants from other species of mammals most particularly swine and all are subject to rejection by the host body or may elicit other side effects.

### SUMMARY OF THE INVENTION

It would be useful to make human or humanized tissues and organs personalized to each recipient's immune complex. As disclosed herein, it is possible to do so by using a large animal as a host editing its genome to knock out or debilitate genes responsible for the growth and/or differentiation of a target organ and inoculating that animal at a blastocyst or zygote stage with donor stem cells to complement the missing genetic information for the growth and development of the organ. The result is a chimeric animal in which the complemented tissue (human/humanized organ) matches the genotype and phenotype of the donor. Such organs may be made in a single generation and the stem cell may be taken or generated from the patient's own body. As disclosed herein, it is possible to do so by simultaneously editing multiple genes in a cell or embryo creating a "niche" for the complemented tissue. Multiple genes can be targeted for editing using targeted nucleases and homology directed repair (HDR) templates in vertebrate cells or embryos.

In one exemplary embodiment, the disclosure provides a method of produced humanized tissues in a non-human host animal comprising: i) genetically editing one or more genes responsible for a desired tissue or organ's growth and/or development in a cell or embryo, of the host; ii) complementing the host's lost genetic information by injecting an effective amount of stem cells from a donor into the cell, embryo, zygote or blastocyst to create a chimeric animal; wherein the chimeric tissues occupy a niche afforded by the genetic editing; and wherein the niche comprises a human or humanized tissue or organ.

### BRIEF DESCRIPTION OF THE DRAWINGS

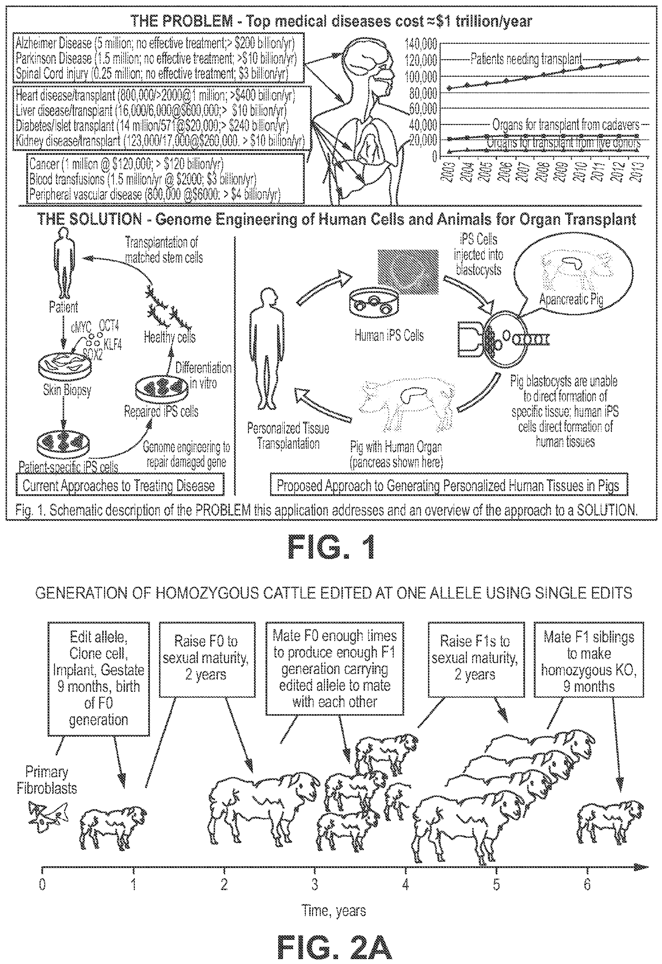

FIG. 1 is a schematic diagram illustrating the problem of tissue/organ transplantation and the solution provided by genome engineering of Human Cells and Animals for Organ Transplant.
FIG. 2A depicts a process for making animals homozygous for two knockouts using single edits.

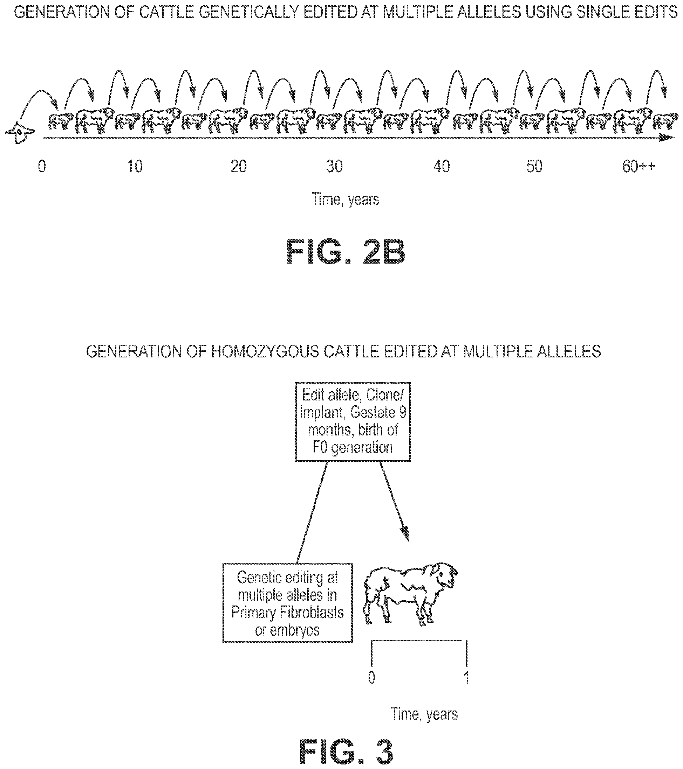

FIG. 2B depicts a hypothetical process of making animals with multiple edits by making of a single edit at a time.
FIG. 3 depicts multiplex gene edits used to establish founders at generation F0

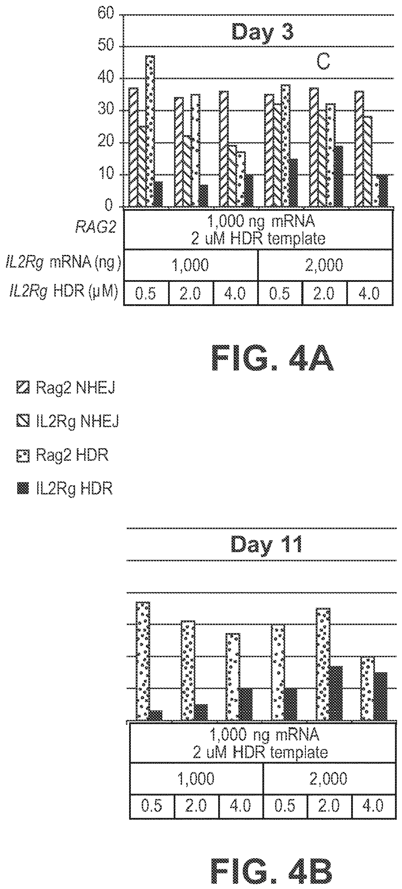

FIGs. 4A-4D Multiplex gene editing of swine *RAG2* and *IL2Rγ* (or IL2Rg). 4A) Surveyor and RFLP analysis to determine the efficiency of non-homologous end joining (NHEJ) and homology depended repair HDR on cell populations 3 days post transfection. 4B) RFLP analysis for homology dependent repair on cell populations 11 days post transfection. 4C) Percentage of colonies positive for HDR at *IL2Rγ, RAG2* or both. Cells were plated from the population indicated by a "C" in 4A. 4D) Colony analysis from cells transfected with TALEN mRNA quantities of 2 and 1 µg for *IL2Rγ* and *RAG2* and HDR template at 1 µM for each. Distribution of colony genotypes is shown below. In present application, *IL2Rγ* and *IL2Rg* are used interchangeably.

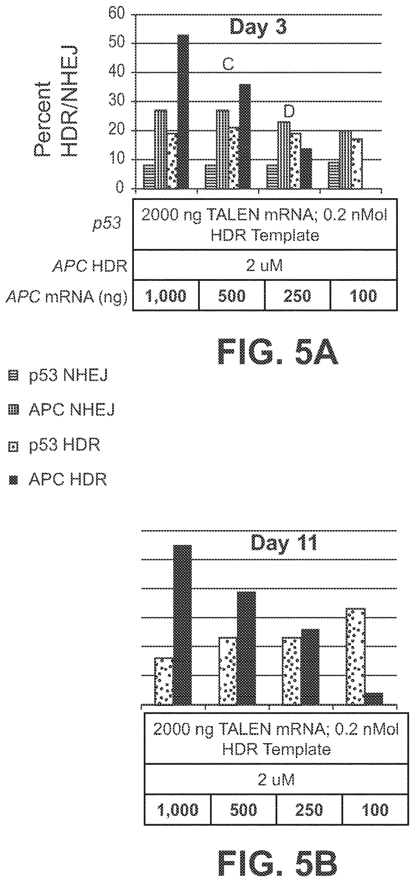

FIG. 5A-5D Multiplex gene editing of swine *APC* and *p53.* 5A) Surveyor and RFLP analysis to determine the efficiency of non-homologous end joining (NHEJ) and homology depended repair HDR on cell populations 3 days post transfection. 5B). RFLP analysis for homology dependent repair on cell populations 11 days post transfection. 5C and 5D) Percentage of colonies positive derived from the indicated cell population (indicated in 5A, "5C" and "5D") for HDR at *APC, p53* or both. Colonies with 3 or more HDR alleles are listed below.

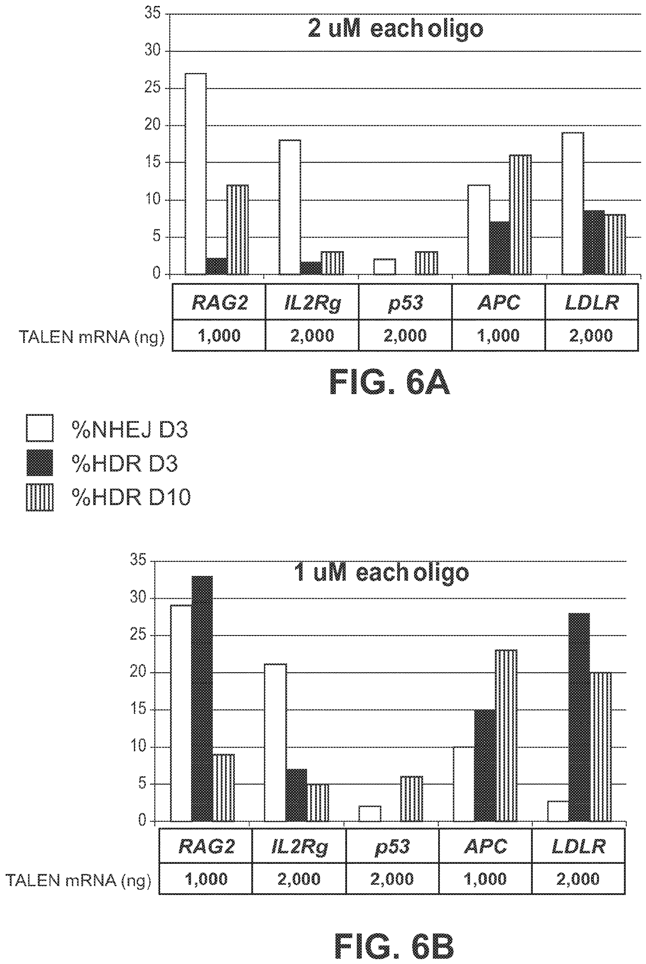

FIG. 6 Effect of Oligonucleotide HDR template concentration on Five-gene multiplex HDR efficiency. Indicated amounts of TALEN mRNA directed to swine *RAG2, IL2Rg, p53, APC* and *LDLR* were co-transfected into pig fibroblasts along with 2 uM (6A) or 1 uM (6B) of each cognate HDR template. Percent NHEJ and HDR were measured by Surveyor and RFLP assay.

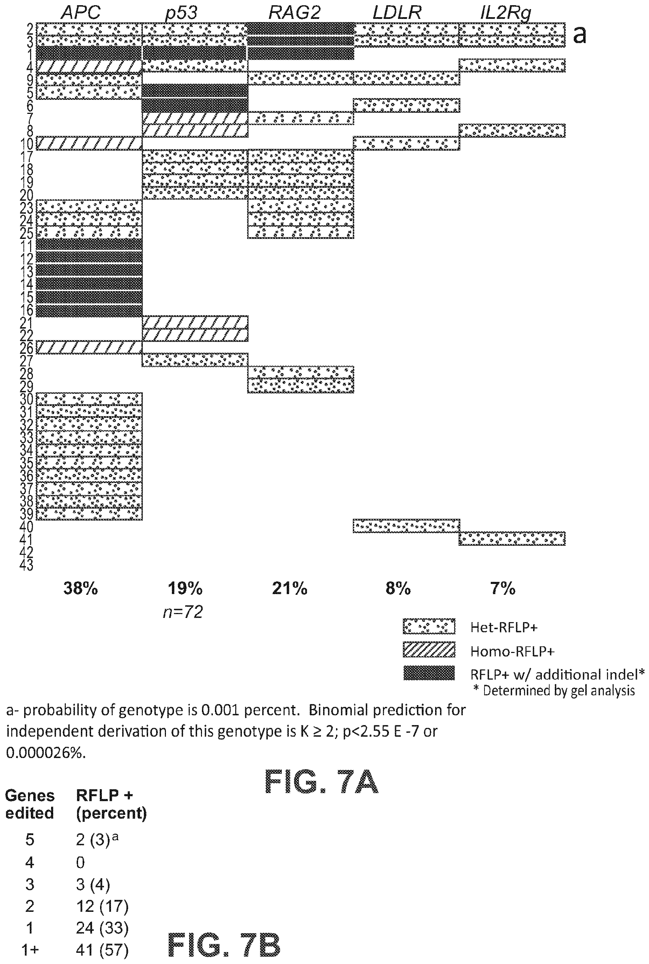

FIG. 7 is a five-gene multiplex data set that shows plots of experimental data for the effect of oligonucleotide HDR template concentration on 5-gene multiplex HDR efficiency. Indicated amounts of TALEN mRNA directed to swine *RAG2, IL2Rg, p53, APC* and *LDLR* were co-transfected into pig fibroblasts along with 2 uM (7A) or 1 uM (7B) of each cognate HDR template. Percent NHEJ and HDR were measured by Surveyor and RFLP assay. Colony genotypes from 5-gene multiplex HDR: Colony genotypes were evaluated by RFLP analysis. 7A) Each line represents the genotype of one colony at each specified locus. Three genotypes could be identified; those with the expected RFLP genotype of heterozygous or homozygous HDR as well as those with an RFLP positive fragment, plus a second allele that has a visible shift in size indicative of an insertion or deletion (indel) allele. The percentage of colonies with an edit at the specified locus Bis indicated below each column. 7B) A tally of the number of colonies edited at 0-5 loci.

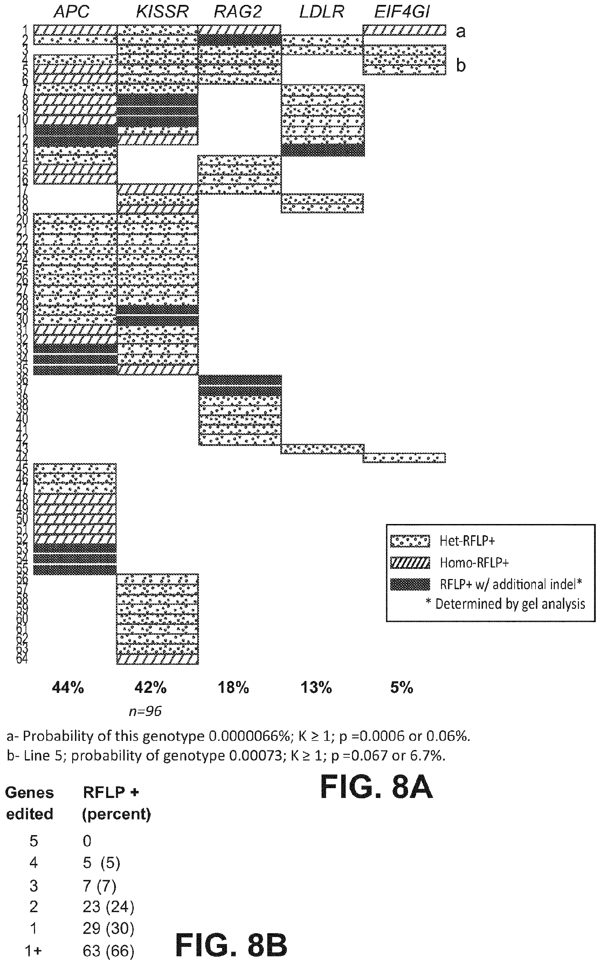

FIG. 8 is another five-gene multiplex data set that shows plots of experimental data for a second experiment involving the effect of oligonucleotide HDR template concentration on Five-gene multiplex HDR efficiency. Colony genotypes of a second 5-gene multiplex trial. 8A) Each line represents the genotype of one colony at each specified locus. Three genotypes could be identified; those with the expected RFLP genotype of heterozygous or homozygous HDR as well as those with an RFLP positive fragment, plus a second allele that has a visible shift in size indicative of an insertion or deletion (indel) allele. The percentage of colonies with an edit at the specified locus is indicated below each column. 8B) A tally of the number of colonies edited at 0-5 loci.

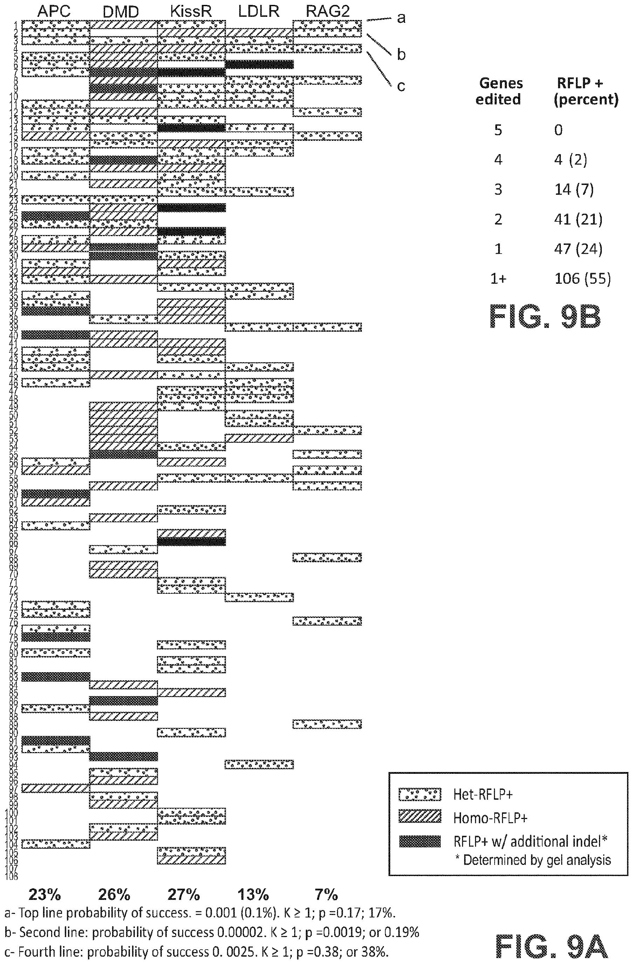

FIG. 9 is another five-gene multiplex trial data set that shows colony genotypes. 9A) Each line represents the genotype of one colony at each specified locus. Three genotypes could be identified; those with the expected RFLP genotype of heterozygous or homozygous HDR as well as those with an RFLP positive fragment, plus a second allele that has a visible shift in size indicative of an insertion or deletion (indel) allele. The percentage of colonies with an edit at the specified locus is indicated below each column. 9B) A tally of the number of colonies edited at 0-5 loci.

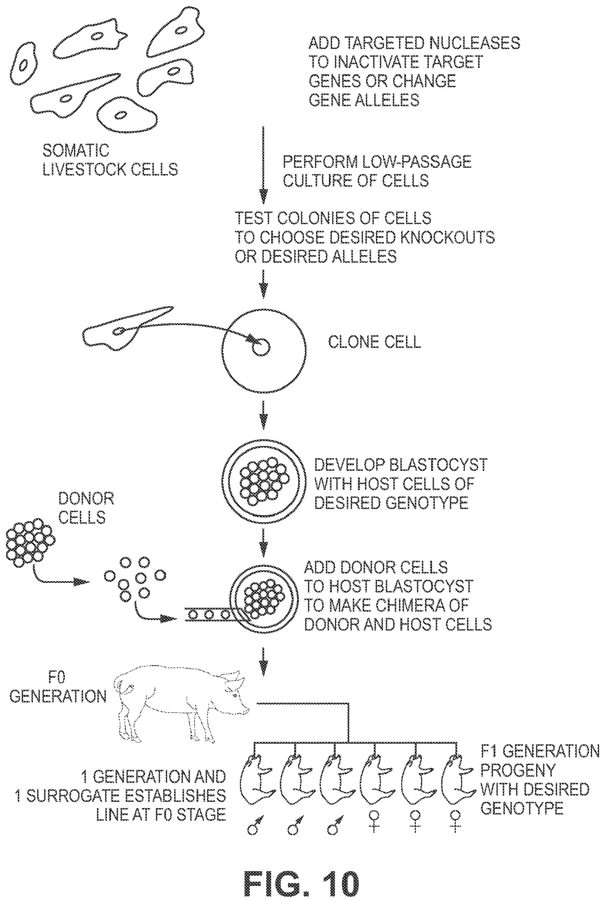

FIG. 10 depicts a process of making an F0 generation chimera with targeted nucleases that produce a desired gene knockout or choice of alleles.

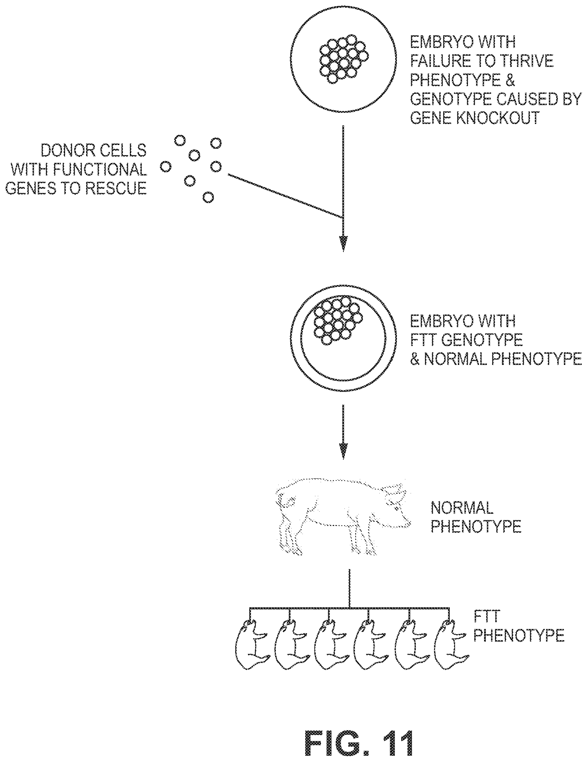

FIG. 11 depicts establishment of an F0 generation animal with a normal phenotype and progeny with a failure to thrive (FTT) phenotype and genotype.

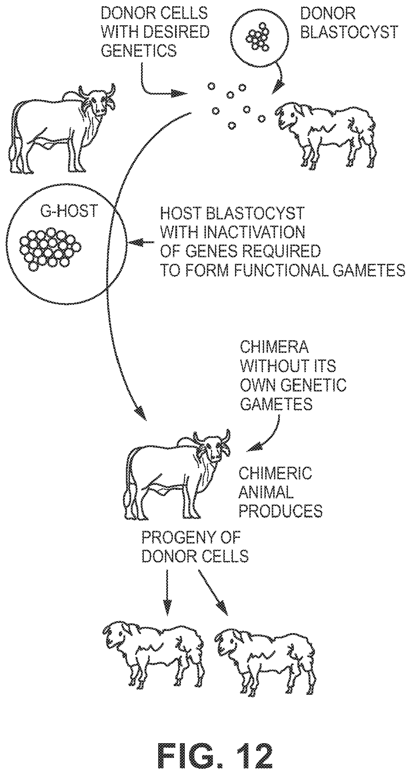

FIG. 12 depicts a process for making chimeric animals with gametes having the genetics of the donor embryo.

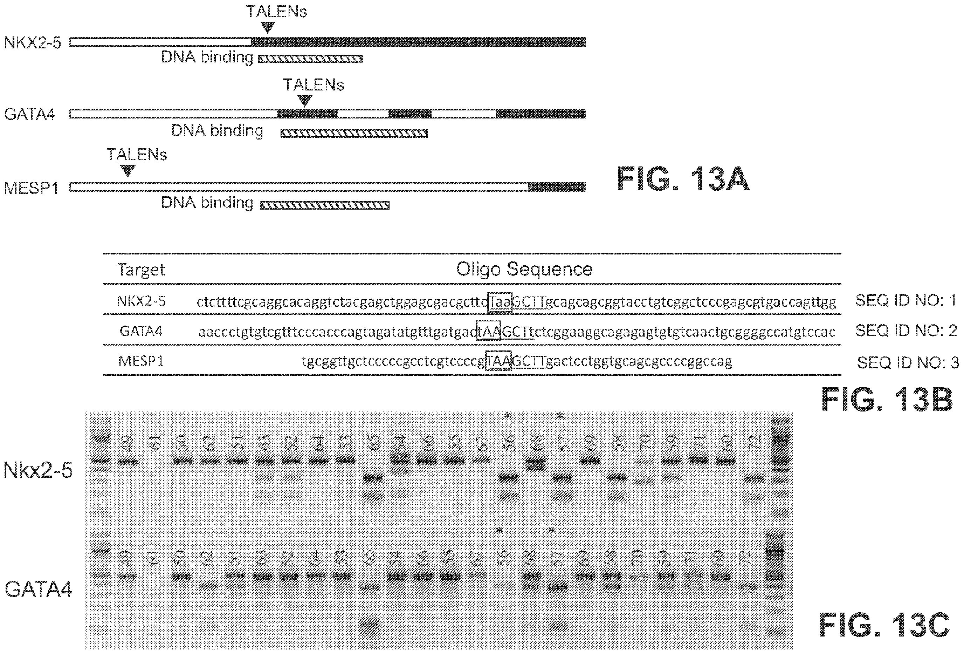

FIG. 13 depicts multiplex editing at three targeted loci of NKX-2, GATA4, and MESP1. 13A) is a schematic of the experiment, 13B) shows the targeting of the genes, with the NKX2-5, GATA4, and MESP1 listed as SEQ ID NOs: 1-3, respectively. 13C) depicts the results of an assay for the experiments. Oligo sequences for each target gene. Novel nucleotides are represented by capital letters. The PTC is represented by light color letters in boxes and the novel HindIII RFLP site is underlined.

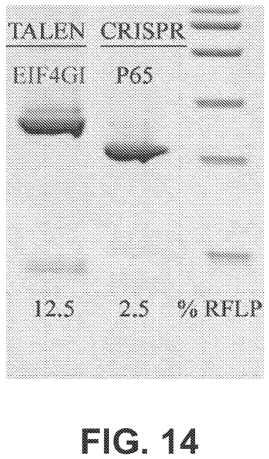

FIG. 14 depicts multiplex gene-editing using a combination of TALENs and RGENs; assay of transfected cells evaluated by RFLP revealed HDR at both sites.

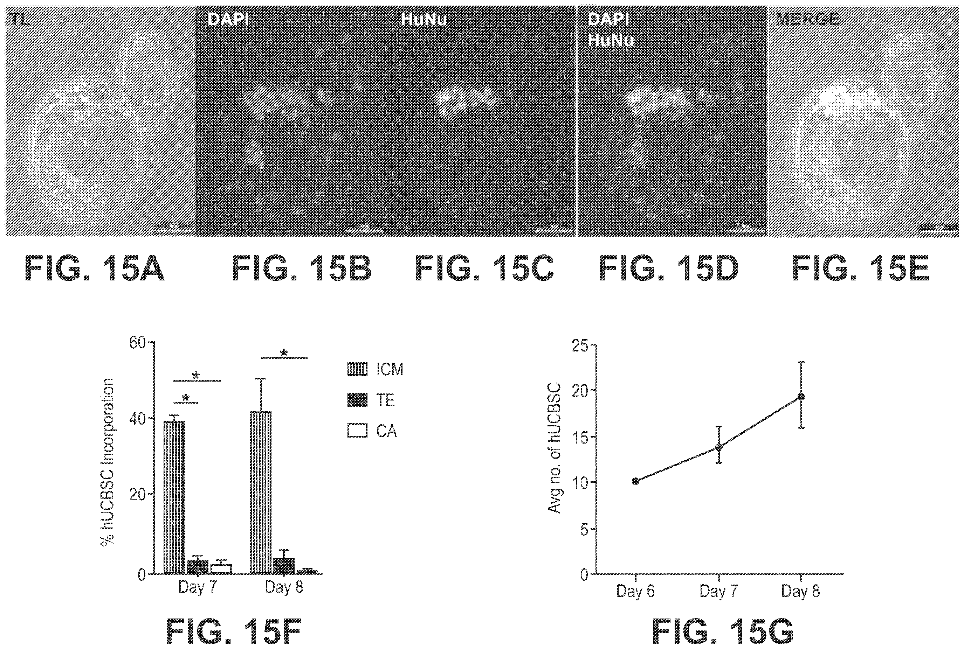

FIG. 15 Incorporation of human cord blood stem cells (hUCBSC) into parthenogenetic porcine blastocyst. 15A) Phase contrast image of blastocyst. (15B) DAPI image of cells within the blastocyst. (15C) Human nuclear antigen (HNA) staining. (15D) Merged DAPI and HuNu image. (15E) Merged image of panels 15A, 15B, and 15C. (15F) Quantification of HuNu cells in the inner cell mass (ICM), trophectoderm (TE), or blastocoel cavity (CA). (15G) Proliferation of HNA cells at days 6, 7, and 8 after activation of oocyte. Injection of hUCBSC was at day 6.

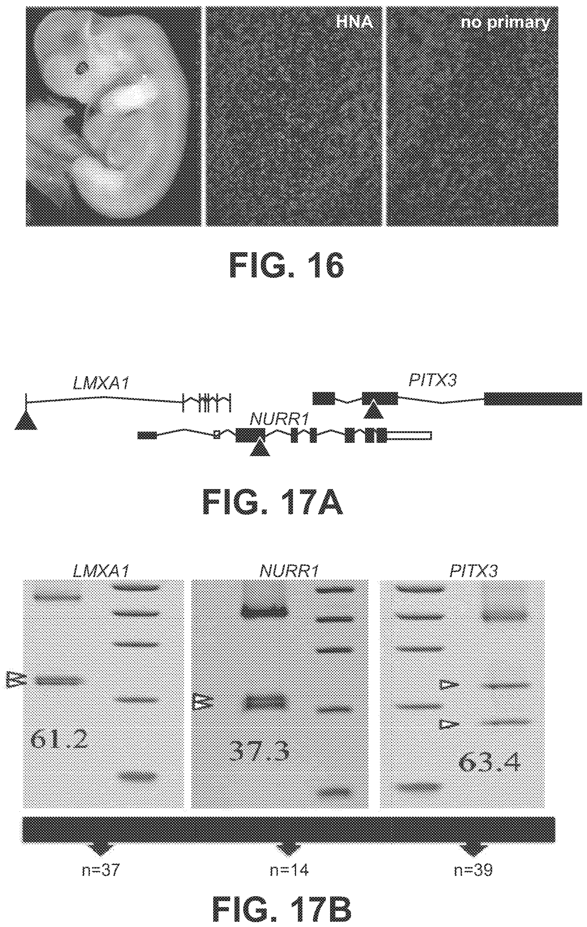

FIG. 16 Chimeric human-porcine fetus. Left panel - chimeric fetus at 28 days in gestation following injection of hUCBSCs into parthenogenetic porcine blastocysts. Center panel - staining for human nuclear antigen (HNA) in red and DAPI in blue. Right panel - control section with no primary antibody.
FIG. 17 TALEN mediated knockout of porcine genes. (17A) Cleavage sites for *LMXA1, NURR1,* and *PITX3.* (17B) TALEN cleavage products as indicated by double arrows.

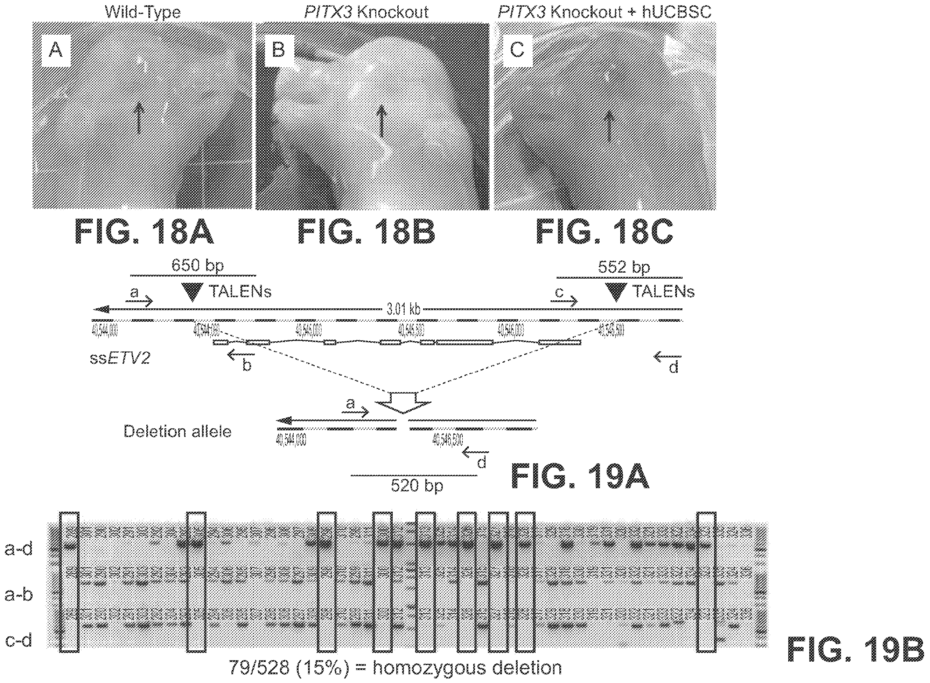

FIG. 18 Ocular effects of complementation of *PITX3* knockout in porcine blastocysts with human umbilical cord blood stem cells. 18A. Wild fetal pig. 18B. Fetal pig with *PITX3* knockout. 18C. Fetal pig with *PITX3* knockout and complemented with hUCBSCs. Arrow points to the location of the eye for each fetus.
FIG. 19 TALEN-mediated knockout of *ETV2.* (19A) Three-tiered PCR assay utilized to detect gene editing. Amplification from primers a-d indicated a deletion allele was present. To distinguish between heterozygous and homozygous clones, primers a-b and c-d were used to amplify the wild type allele. Only when the a-d product is present and both a-b, c-d products are absent is the clone considered homozygous for the deletion allele. (19B) Clones fitting these criteria are enclosed by a green box.

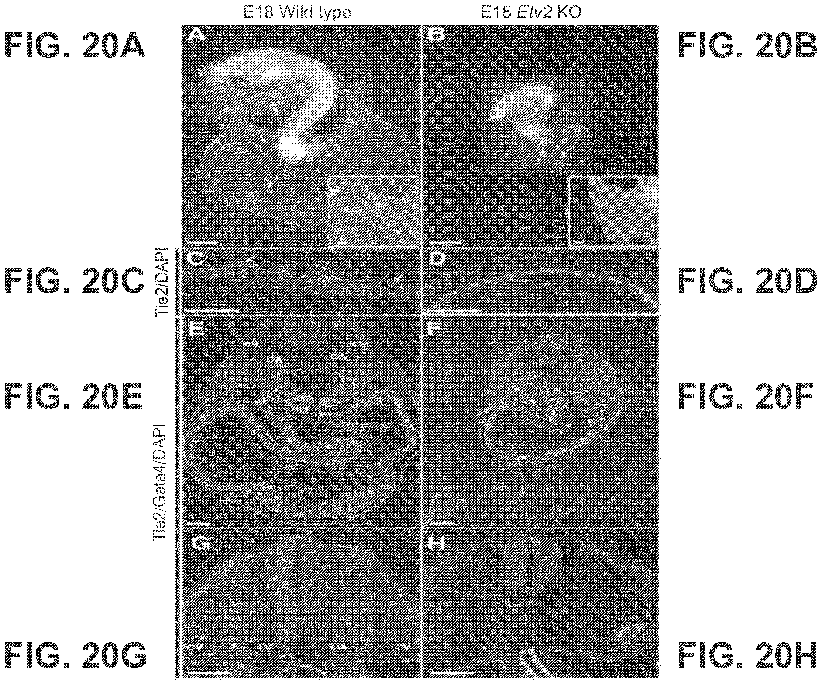

FIG. 20. Loss of porcine *ETV2* recapitulated the mouse *Etv2* mutant phenotype. Wild-type E18.0 pig embryo (20A) and (20B) *ETV2* knockout embryo at the same developmental stage. Insets show enlarged views of the allantois. Note an abnormal overall morphology with lack of vascular plexus formation in the mutant (inset). (20C-20H) Sections through the allantois (20C, 20D), the heart level (20E, 20F) and the trunk level (20G, 20H) of the embryos shown in 20A and 20B, respectively, were stained for Tie2, an endothelial marker; Gata4, a cardiac lineage marker; and 4',6-diamidino-2-phenylindole (DAPI), a nuclear counterstain. The wild-type allantois was highly vascularized with Tie2 positive endothelial lining and contained blood (20C, arrows), whereas, the mutant lacked these populations (20D). The endocardium, cardinal veins (CV), and dorsal aortae (DA) are clearly visible in the wild-type embryo (20E, 20G). In contrast, *ETV2* null embryos completely lacked these structures although the heart progenitors and gut marked by Gata4 (green) were present (20F and 20H, respectively). Scale bars: 1000 µm (20A, 20B), 200 µm (insets in 20A, 20B), 100 µm (20C-20H).

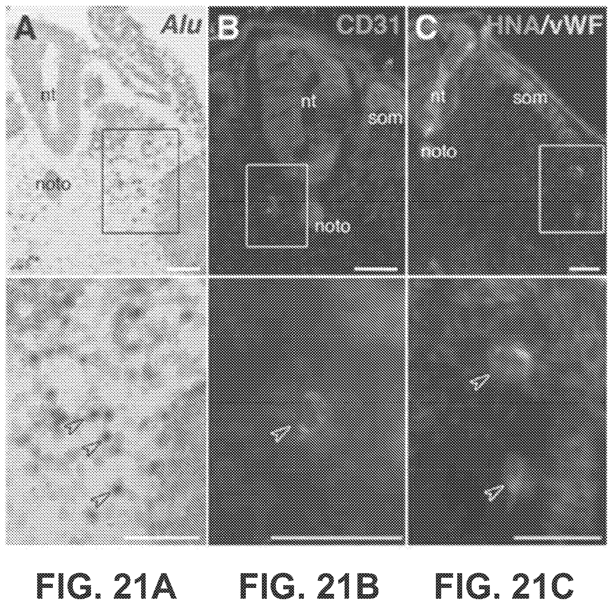

FIG. 21 Complementation of *ETV2* mutant porcine embryos with hiPSCs. *ETV2* mutant blastocysts were generated by SCNT, and injected with ten hiPSCs at the morula stage and subsequently transferred into hormonally synchronized gilts. (21A) *In situ* hybridization using the human specific *Alu* sequence. (21B, 21C) Immunohistochemistry against human CD31 (21B), HNA (21C, red), and human vWF (21C, green). Boxed areas are enlarged in panels below. Arrowheads point to positive cells. Note formation of vessel-like structures. All scale bars indicate 50 microns. nt: neural tube, noto: notochord, som: somite.

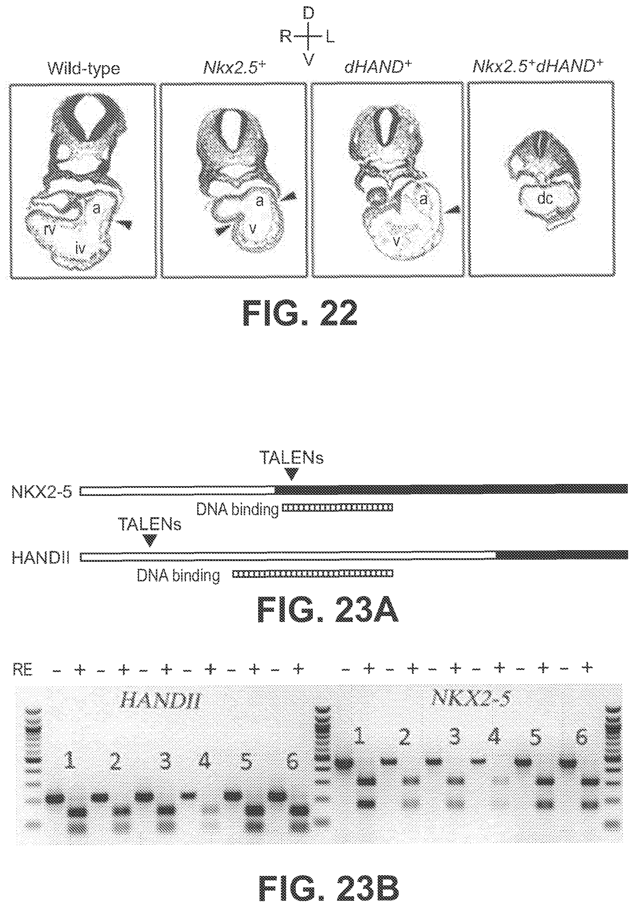

FIG. 22 Nkx2-5 and HandII (also known as dHand) double knockouts lack both ventricles (rv and lv) and have a single, small primitive atrium (dc).
FIG. 23 Double knockout of *NKX2-5* and *HANDII* in swine fibroblasts. 23A) Schematics of the coding sequence for each gene are shown; alternating colors indicate exon boundaries, the blue region (below) indicates the DNA binding domain of each transcription factor, and the triangles indicate the location TALENs binding sites. 23B) RFLP analysis of fibroblast colonies for bialleic KO of *HANDII* and *NKX2-5.*

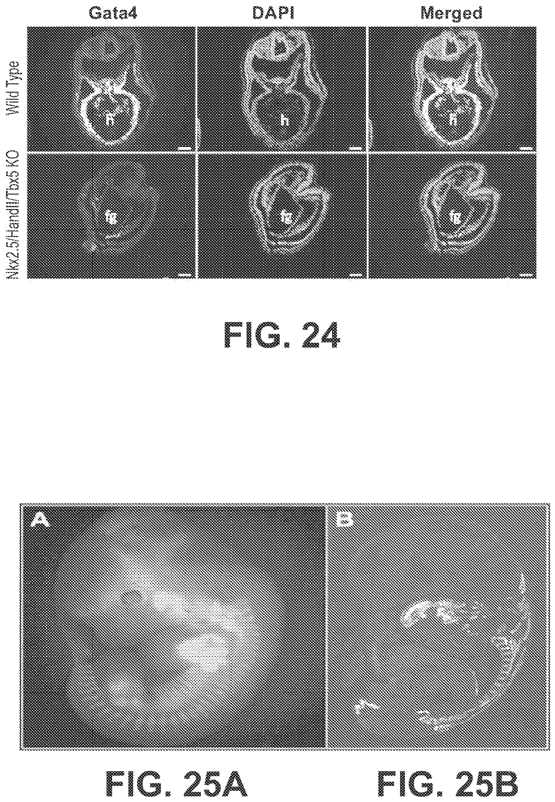

FIG. 24 Nkx2-5/HANDII/TBX5 triple knockout porcine embryos have acardia. Triple knockout porcine embryos lack a heart with essentially no Gata4 immunohistochemically positive cells (marking the heart) at E18.0 (h, heart and fg, foregut).
FIG. 25 Myf5, Myod and Mrf4 are master regulators of skeletal muscle and are restricted to skeletal muscle in development and in the adult. Shown here is Myod-GFP transgenic expression which is restricted to the somites, diaphragm and established skeletal muscle at E11.5 (25A). In 25B, in situ hybridization of a parasagittal section of an E13.5 (mid-gestation) mouse embryo using a 35S-labeled MyoD riboprobe. Note expression in back, intercostal and limb muscle groups.

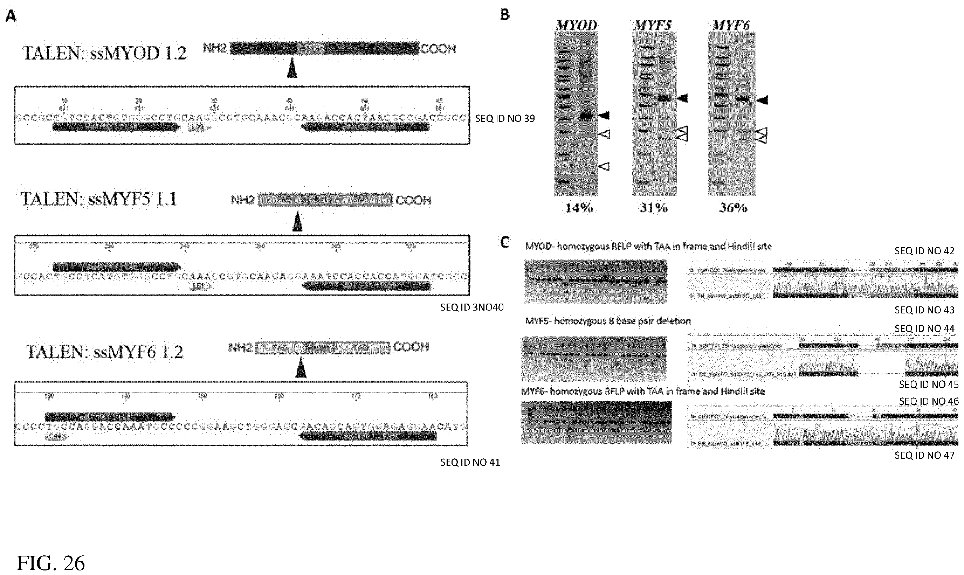

FIG. 26A. TALEN pairs were designed for swine *MYOD, MYF5,* and *MYF6 (aka MRF4)* genes. TALEN binding sites (denoted by red arrow heads) were upstream the critical basic (+) helix-loop-helix (HLH) domain for each gene. The TALEN binding sites are shown below (denoted by red arrows) and the amino acid that was targeted for a premature STOP codon by homology dependent repair (HDR) are denoted by yellow arrows. 26B. HDR templates were designed to introduce the premature STOP codon and a novel restriction enzyme recognition site (HindIII) to allow facile analysis of HDR events. The region of interest for each gene was amplified by PCR and restriction fragment length polymorphism (RFLP) was assessed for the population of transfected cells. The closed arrow heads denote the uncut or wild type alleles, while the open arrow heads denote the HDR alleles. The percent of alleles positive for HDR for *MYOD, MYF5,* and *MYF6* were 14%, 31%, and 36%, respectively. 26C. These populations were plated out for individual colony isolation. 38 out of 768 (4.9%) colonies demonstrated 4 or more RFLP events and were further analyzed by sequencing. 5 clones were identified to be homozygous knockout for all three genes by either HDR incorporating the premature STOP codon or in/dels that would result in a frameshift and subsequent premature STOP codon. An example of the RFLP analysis and sequencing of a clone that is a triple knockout for *MYOD*/*MYF5*/*MYF6* is shown.

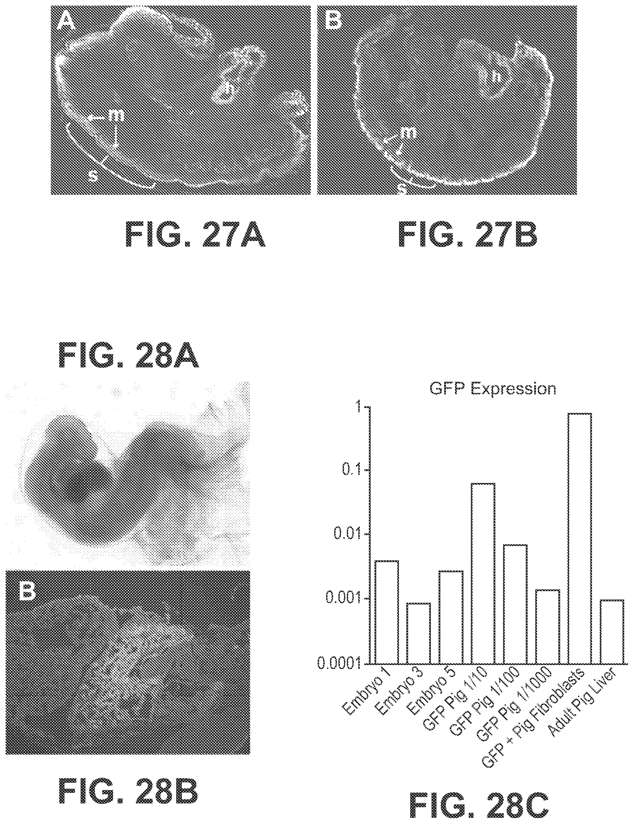

FIG. 27 At E18.0, wild-type (Wt) embryos had well defined somites(s), desmin positive (red) myotomes (m) and developing musculature (Fig. 27A). In addition, the developing heart tube demonstrated strong desmin signal (h). In contrast, *MYF5*/*MYOD*/*MRF4* KO embryos showed a lack of myotome formation while the heart remained desmin positive (Fig. 27B).
FIG. 28A. E20 porcine *MYF5*/*MYOD*/*MRF4* null embryos complemented with GFP labeled blastomeres. Native GFP is observed in the liver and yolk sac of the embryo. 28B. Section of porcine liver from *MYF5*/*MYOD*/*MRF4* null embryos (E20) complemented with GFP labeled blastomeres. Native GFP is visible in the sinusoids of the liver. 28C. PCR of yolk sac from E20 porcine *MYF5*/*MYOD*/*MRF4* null embryos complemented with GFP labeled blastomeres (Embryos 1[shown in 28A and 28B], 3, 5). GFP-labeled pig fibroblasts is positive control while WT pig liver is negative control.

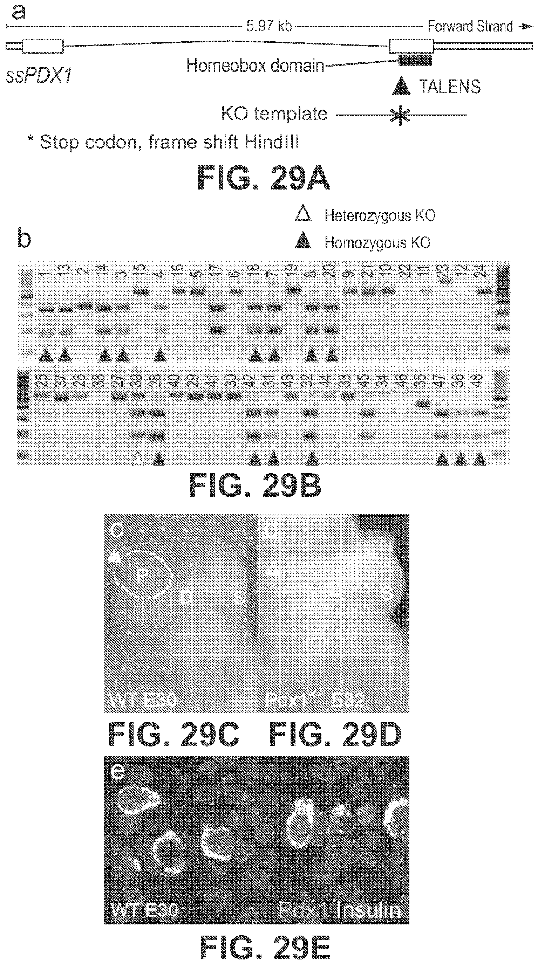

FIG. 29 Generating *PDX1-*/*-* pigs (a) TALEN gene editing of the pig *PDX1* locus. (b) RFLP analysis identified unmodified, heterozygous knockouts (open arrowhead) or homozygous knockouts (closed arrowhead). 41% of the clones were homozygous knockouts for *PDX1.* (d) Pancreas ablation (Δ) in cloned E32 *Pdx1-*/*-* pig embryos compared to the pancreas in WT E30 embryos (c) containing nascent b cells (e) P pancreas, S stomach, D duodenum of Wt E30 fetus.

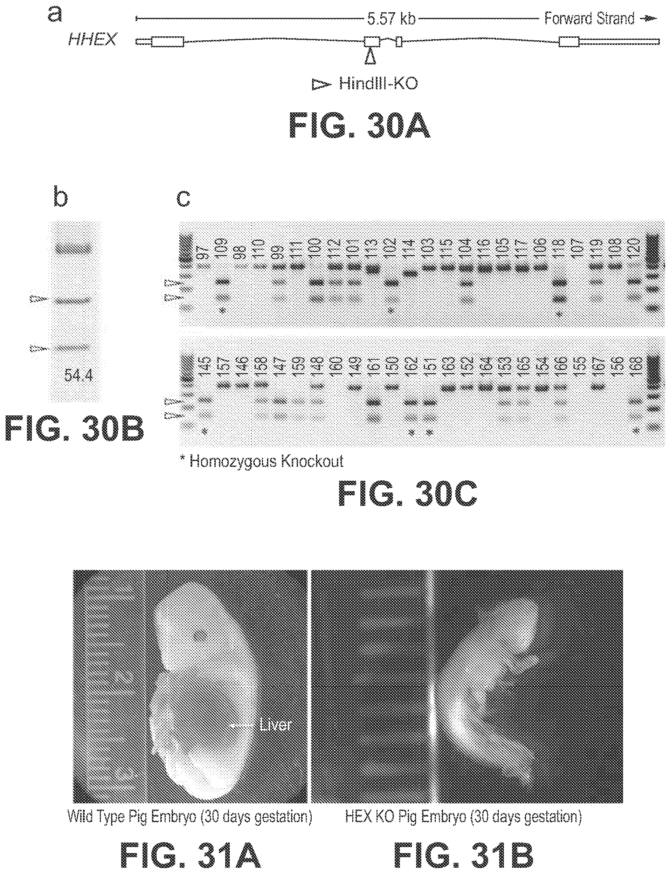

FIG. 30, Generation of *HHEX* KOs by gene-editing. (30A) The *HHEX* gene is comprised of 4 exons. The *Hind*III KO allele was inserted into exon 2 of the *HHEX* gene by gene-editing. (30B) The efficiency of gene-editing was measured on the transfected population by a *Hind*III RFLP assay. The proportion of chromosomes with the novel *Hind*III KO allele (indicated by cleavage products, open triangles) is indicated on the gel. (30C) Fibroblast clones were also screened using the *Hind*III RFLP assay. Homozygous KO clones are indicated with an asterisk.
FIG. 31, Liver development in wild-type and HHEX KO pig embryos at 30 days in gestation. Note absence of liver development in HHEX KO specimen on the right panel. Wild-type control at the same gestational age is shown on the left panel.

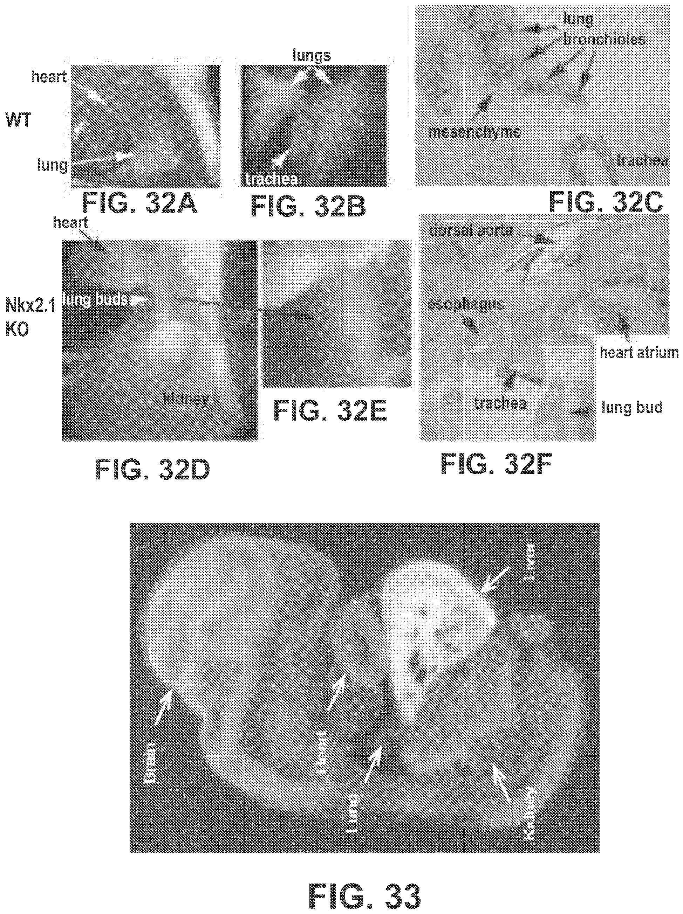

FIG. 32, Knockout of NKX2.1 results in loss of fetal lung. Upper panels - wild type lungs. Low panels - NKX2.1 knockout lungs.
FIG. 33, MR imaging of fetal pig at 16.4T showing internal organs. Pig gestational age is 30 days when crown-rump length is approximately 20 mm.

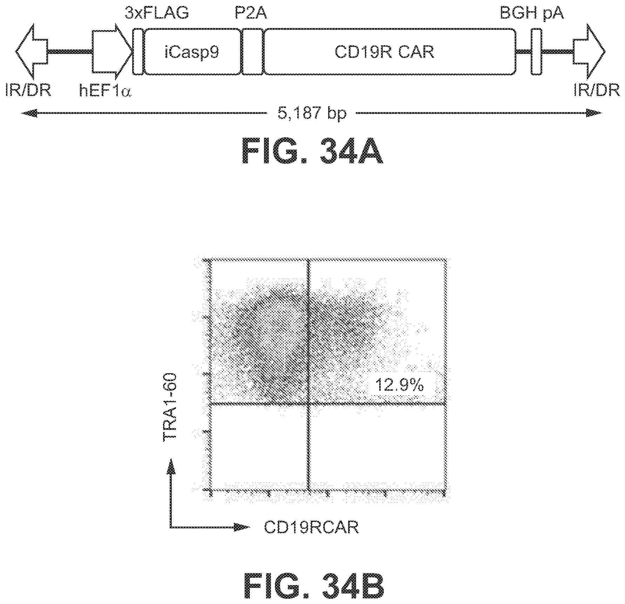

FIGs. 34A-34B, 34A is a cartoon illustrating a strategy for utilizing sleeping Beauty (SB) transposons that drives expression of both CD19 target CAR and iCasp9. 34B FACS analysis demonstrating that SB system can successfully introduce CAR gene into iPSCs 10 days after transfection.

### DETAILED DESCRIPTION

The present disclosure provides methods to engineer and to produce viable authentic human organs such as hearts, livers, kidneys, lungs, pancreases, and skeletal muscle; and cells such as neurons and oligodendrocytes, immune cells, and endothelial cells for making blood vessels. The strategy to achieve this goal is to interrogate key genes that are critical for the development of specific organs. These genes are evaluated using gene editing technology to knockout specific genes to determine which genes alone or in combination will give rise to specific organs or cell types when knocked out in murine and porcine blastocysts. The gene knockouts in blastocysts will create a niche in which normal syngeneic or xenogeneic stem cells should occupy to contribute to the development of the desired organ or cell (Fig. 1). Novel gene editing and gene modulation technologies using TALENS, CRISPR, and synthetic porcine artificial chromosomes are used to knockout desired target genes and to enhance the function of other genes that can minimize off-target effects. Human stem cells are vetted to determine which type of stem cell gives rise to a robust replication of specific human organs and cells. The inventors address this issue by evaluating the contribution of various human stem cells to the inner cell mass of porcine blastocysts and to the developing chimeric fetus. The interactions among these three technical areas are critical to the successful achievement of creating authentic human organs and cells.

Classically, genetic complementation, refers to the production of a wild-type phenotype when two different mutations are combined in a diploid or a heterokaryon. However, modern techniques of chimera production can now rely on stem cell complementation, whereby cells of more than one embryonic origin are combined to make one genetically mixed animal. In this case, complementation does not involve any change in the genotypes of individual chromosomes; rather it represents the mixing of gene products. Complementation occurs during the time that two cell types are in the same embryo and can each supply a function. Afterward, each respective chromosome remains unaltered. In the case of chimeras, complementation occurs when two different sets of chromosomes, are active in the same embryo. However, progeny that result from this complementation will carry cells of each genotype. In embryonic complementation, genes of the host embryo are edited to produce a knock out or otherwise make a non-functional gene. When human stem cells are injected into the gene edited blastocyst, they will rescue or "complement" the defects of the host (edited) genome. When the gene or genes that are knocked out support the growth of a particular organ or tissue, the resulting complementation produced tissue will be the result of the growth and differentiation of the non-edited, e.g., stem cell derived genotype. When human stem cells are used to complement the host-edited genome, the resulting tissue or organ will be composed of human cells. In this way, fully human organs can be produced, in vivo, using another animal as a host for the complementation produced organ.

Because multiple genes may be responsible for the growth and differentiation of any particular organ or tissue, processes for multiplex gene edits are also described. Multiple genes can be modified or knocked out in a cell or embryo that may be used for research or to make whole chimeric animals. These embodiments include the complementation of cell or organ loss by selective depopulation of host niches. These inventions provide for rapid creation of animals to serve as models, food, and as sources of cellular and acellular products for industry and medicine.

FIG. 1 provides a schematic description of the problem and the proposed approach for providing personalized human organs and tissues to those in need using swine as a host animal. Those of skill in the art will appreciate that the technology which allows for the production of induced pluripotent stem cells (IPSC) allows for a patient to provide her or his own stem cells for complementation of the edited genes and production of human or humanized "self" organs or tissues.

The use of multiplex gene editing is essential for producing a host animal with multiple edited genes in need of complementation. FIG. 2A has a timeline that illustrates why it takes several years using single edits to make livestock that have only two edited alleles, with the time being about six years for cattle. Edited, in this context, refers to choosing gene and altering it. First, a gene of interest has to be edited, for instance knocked out (KO), in cultured somatic cells that are cloned to create a heterozygous calf with a targeted KO. The heterozygotes would be raised to maturity for breeding, about 2 years old for cattle, to generate first-generation (F1) male and female heterozygous calves, which would be bred with each other to generate a homozygous knockout calf (F2). Generating homozygotes with respect to multiple targeted mutations using a conventional approach in cattle would be impractical. The number of required years and the number of required animals to make further edits increases in an approximately exponential fashion, depending on the particular scheme that is used, as illustrated in FIG. 2B. Among the vertebrates, even those animals that have larger numbers of offspring per generation and have shorter gestational times than cattle nonetheless would require overly long times to achieve multiple edits. Swine, for example, have a larger number of offspring per mating and a gestational time that is roughly half that of cattle but the time to make multiple edits can require many years. Moreover, schemes that minimize time with aggressive inbreeding may not be reasonably possible for multiple edits. Also, serial cloning is undesirable from a process and an outcome standpoint, especially if the animals are to be useful as livestock or laboratory models.

An opportunity presented by the invention is illustrated in FIG. 3, which shows multiple edits being made in a first-generation animal (F0). Embryos are prepared directly or by cloning with two or more edits independently chosen to be heterozygotes or homozygotes and placed in surrogate females to gestate. The resultant animals are F0 generation founders. A plurality of embryos may be prepared and placed in one or more surrogates to produce progeny of both genders, or well-known techniques of embryo-splitting may be used to make a plurality of clonal embryos. Livestock such as pigs that typically produce a litter with both genders may be crossed and propagated.

Multiple alleles can be disrupted or otherwise edited as described herein in a cell or embryo using targeted endonucleases and homology directed repair (HDR). An embodiment is a method of making genetic edits in a vertebrate cell or embryo at a plurality of target chromosomal DNA sites comprising introducing into a vertebrate cell or embryo: a first targeted endonuclease directed to a first target chromosomal DNA site and a first homology directed repair (HDR) template homologous to the first target site sequence; and a second targeted endonuclease directed to a second target chromosomal DNA site and a second HDR template homologous to the second target site sequence, with the first HDR template sequence replacing the native chromosomal DNA sequence at the first target site and the second HDR template sequence replacing the native chromosomal DNA sequence at the second target site sequence.

It was an unexpected and surprising, and not predictable, result to learn that multiple edits such as knockouts or replacements could be obtained. One theorized mechanism is that there are a minority of cells that are receptive to multiple edits because they are at a particular stage in the cell cycle. When exposed to endonucleases and HDR templates, they respond readily. A related theory of operation is that the HDR templating process lends itself to multiple substitutions because activation of cellular repair machinery for one targeted site favors repair, or HDR templating, at other sites as well. HDR has historically been a low efficiency process so that multiple HDR edits were apparently not attempted, observed, or recognized.

Heretofore, previous experiments with xenogeneic complementation have only been done on single edit genomes. However, the disclosed platform for multiplex gene editing now provides for a host blastocyst having multiple edited genes allowing for the complementation of those edits by human stem cells and the production of those organs and tissues arising therefrom.

Results herein show that too much or too little endonuclease and/or HDR template can have a negative effect, which may have confounded prior research in this area. In fact, the inventors have observed that targeted endonucleases can be designed and made correctly but nonetheless fail because they are too efficient. Further, the population of successfully modified cells often does not improve over time. Artisans modifying cells normally look for longevity of the cell and modification as an indicator of stability and health for successful cloning or other uses. But that expectation has often not been helpful in the multiplexing processes herein. Moreover, the inventors have observed that homologous recombination (HR) introgression efficiencies are variable in the multiplex approach as compared to a single-locus introgression. Some loci were very sensitive but others had large drops in efficiency. There is apparently interference between the endonucleases but the net effect cannot be explained simply, for instance by positing that the endonucleases are competing for common resource.

There are various well-known techniques to insert many genes randomly or imprecisely into a plurality of locations in chromosomal DNA, or to make many random edits that disrupt a plurality of genes. As is evident, random or imprecise processes are not going to assist the scientist that needs to edit a plurality of specifically targeted genes to achieve an effect. Accordingly, HDR processes taught herein may be readily distinguished by the edits, and resultant organisms, being made only at the intended target sites. One difference is that the inventive HDR editing embodiments can be performed free of insertion of extra gene copies and/or free of disruption of genes other than those targeted by the endonucleases. And the specific edits are made at one location because the HDR template sequence is not copied into sites without appropriate homology. Embodiments include organisms and processes wherein an exogenous allele is copied into chromosomal DNA only at the site of its cognate allele.

An advantage of HDR-based editing is that the edits can be chosen. In contrast, other attempts, by non-homologous end joining (NHEJ) processes, can make indels at multiple positions such that the indels cancel each other out without making a frame shift. This problem becomes significant when multiplexing is involved. But successful use of HDR provides that the edits can be made to ensure that, if desired, the target gene has an intended frame shift. Moreover, allelic replacement requires HDR and cannot be accomplished by NHEJ, vector-driven insertion of nucleic acids, transposon insertions, and the like. Moreover, choosing organism that are free of unwanted edits further increases the degree of difficulty.

It is generally believed, however, that multiplex edits as described herein have not been previously achieved at targeted sites in cells or animals relevant to livestock or large vertebrates. It is well known that cloning animals from high-passage cells creates animals with so much genetic damage that they are not useful as F0 founders of laboratory models or livestock.

And gene editing is a stochastic process; as a result, the field has traditionally emphasized various screening techniques to identify the few percent of cells that have successfully been edited. Since it is a stochastic process, the difficulty of making a plurality of edits can be expected by the artisan to increase in an exponential fashion as the number of intended edits increases.

An embodiment of the invention provides processes for creating multiple targeted gene knockouts or other edits in a single cell or embryo, a process referred to herein as multiplex gene knockouts or editing. The term targeted gene refers to a site of chromosomal DNA that is selected for endonuclease attack by design of the endonuclease system, e.g., a TALENs or CRISPR. The term knockout, inactivated, and disrupted are used interchangeably herein to mean that the targeted site is changed so that the gene expression product is eliminated or greatly reduced so that the gene's expression no longer has a significant impact on the animal as a whole. These terms are sometimes used elsewhere to refer to observably reducing the role of a gene without essentially eliminating its role.

Gene editing, as that term is used herein, refers to choosing a gene and altering it. Random insertions, gene trapping, and the like are not gene editing. Examples of gene edits are, at targeted sites, gene knockouts, adding nucleic acids, removing nucleic acids, elimination of all function, introgression of an allele, a hypermorphic alteration, a hypomorphic alteration, and a replacement of one or more alleles.

A replacement of an allele refers to a non-meiotic process of copying an exogenous allele over an endogenous allele. The term replacement of an allele means the change is made from the native allele to the exogenous allele without indels or other changes except for, in some cases, degenerate substitutions. The term degenerate substitution means that a base in a codon is changed to another base without changing the amino acid that is coded. The degenerate substitution may be chosen to be in an exon or in an intron. One use for a degenerate substitution is to create a restriction site for easy testing of a presence of the introgressed sequence. The endogenous allele is also referred to herein as the native allele. The term gene is broad and refers to chromosomal DNA that is expressed to make a functional product. Genes have alleles. Genotypes are homozygous if there are two identical alleles at a particular locus and as heterozygous if the two alleles differ. Alleles are alternative forms of a gene (one member of a pair) that are located at a specific position on a specific chromosome. Alleles determine distinct traits. Alleles have basepair (bp) differences at specific positions in their DNA sequences (distinguishing positions or bp) that give rise to the distinct trait and distinguish them from each another, these distinguishing positions serve as allelic markers. Alleles are commonly described, and are described herein, as being identical if they have the same bases at distinguishing positions; animals naturally have certain variations at other bp in other positions. Artisans routinely accommodate natural variations when comparing alleles. The term exactly identical is used herein to mean absolutely no bp differences or indels in a DNA alignment.

A similar test for allelic identity is to align the chromosomal DNA in the altered organism with the chromosomal DNA of the exogenous allele as it is recognized in nature. The exogenous allele will have one or more allelic markers. The DNA alignment upstream and downstream of the markers will be identical for a certain distance. Depending on the desired test, this distance may be from, e.g., 10 to 4000 bp. While an HDR template can be expected to create a sequence that has exactly identical, the bases on either side of the templated area will, of course, have some natural variation. Artisans routinely distinguish alleles despite the presence of natural variations. Artisans will immediately appreciate that all ranges and values between the explicitly stated bounds are contemplated, with any of the following distances being available as an upper or lower limit: 15, 25, 50, 100, 200, 300, 400, 500, 600, 800, 1000, 1200, 1400, 1600, 1800, 2000, 4000.

Artisans are also able to distinguish gene edits to an allele that are a result of gene editing as opposed to sexual reproduction. It is trivial when the allele is from another species that cannot sexually reproduce to mix alleles. And many edits are simply not found in nature. Edits can be also be readily distinguished when alleles are migrated from one breed to the next, even when a replacement is made that exactly duplicates an allele naturally found in another breed. Alleles are stably located on DNA most of the time. But meiosis during gamete formation causes male and female DNAs to occasionally swap alleles, an event called a crossover. Crossover frequencies and genetic maps have been extensively studied and developed. In the case of livestock, the pedigree of an animal can be traced in great detail for many generations. In genetics, a centimorgan (cM, also called a map unit (m.u.)) is a unit that measures genetic linkage. It is defined as the distance between chromosome positions (loci or markers of loci) for which the expected average number of intervening chromosomal crossovers in a single generation is 0.01. Genes that are close to each other have a lower chance of crossing over compared to genes that are distant from each other on the chromosome. Crossing over is a very rare event when two genes are right next to each other on the chromosome. Crossing over of a single allele relative to its two neighboring alleles is so improbable that such an event must be the product of genetic engineering. Even in the case where animals of the same breeds are involved, natural versus engineered allele replacement can be readily determined when the parents are known. And parentage can be determined with a high degree of accuracy by genotyping potential parents. Parent determination is routine in herds and humans.

Embodiments include multiplex gene editing methods that are simultaneous. The term simultaneous is in contrast to a hypothetical process of treating cells multiple times to achieve multiple edits, as in serial knockouts or serial cloning or intervening cycles of animal breeding. Simultaneous means being present at a useful concentration at the same time, for instance multiple targeted endonucleases being present. The processes can be applied to zygotes and embryos to make organisms wherein all cells or essentially all cells have edited alleles or knockouts. Essentially all cells, in the context of a knockout for instance, refers to knocking the gene out of so many cells that the gene is, for practical purposes, absent because its gene products are ineffective for the organism's function. The processes modify cells, and cells in embryos, over a minimal number cell divisions, preferably about zero to about two divisions. Embodiments include a quick process or a process that takes place over various times or numbers of cell divisions is contemplated, for instance: from 0 to 20 replications (cell divisions). Artisans will immediately appreciate that all values and ranges within the expressly stated limits are contemplated, e.g., about 0 to about 2 replications, about 0 to about 3 replications, no more than about 4 replications, from about 0 to about 10 replications, 10-17; less than about 7 days, less than about 1, about 2, about 3, about 4, about 5, or about 6 days, from about 0.5 to about 18 days, and the like. The term low-passage refers to primary cells that have undergone no more than about 20 replications.

Elsewhere, the inventors have shown that, in a single embryo, maternal, paternal or both alleles can be edited in bovine and porcine embryos, and that template editing of both alleles can therefore occur using HDR in the embryo. These edits were made at the same locus. Specifically introgression from sister chromatids was detected. Carlson et al., PNAS 43(109):17382-17387, 2012.

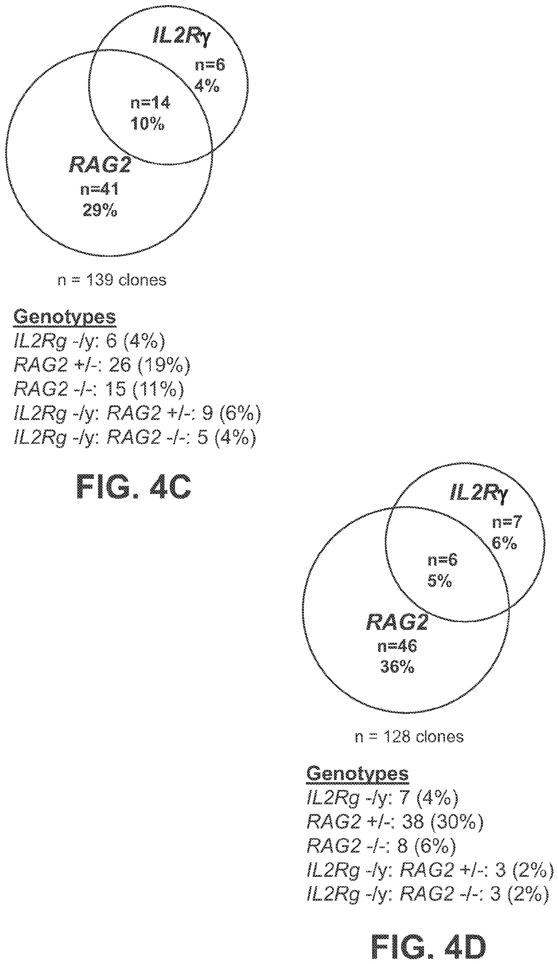

Example 1, see FIG. 4, describes experiments that attempted, successfully, to use HDR editing to knockout two genes at once and, further, to be able to select cells that are homozygous for both knockouts or heterozygous for each knockout. The term select is used to refer to the ability to identify and isolate the cells for further use; there were no expressible reporter genes anywhere in the process, which is a highly significant advantage that distinguishes this process from many other approaches. Cells were treated to introduce a first and a second targeted endonuclease (each being a TALENs pair) directed to, respectively, a first gene (Recombination Activating Gene 2, RAG2) and a second gene target (Interleukin Receptor 2, gamma, IL2Rg or ILR2γ). The TALENs had to be designed to target intended sites and made in adequate amounts. The treatment of the cells took less than five minutes. Electroporation was used but there are many other suitable protein or DNA introducing-processes described herein. The cells were then cultured so that they formed individual colonies of cells that each descended from a single treated cell. Cells from the various colonies were tested after 3 days or 11 days. The rate of knockout of RAG2 was about six times higher than the rate of knockout of IL2Rg; apparently some genes are more difficult to knockout than others. The efficiency of knocking out both genes was high and cells heterozygous or homozygous for both knockouts were successfully identified. Significantly, dosage of TALEN mRNA and HDR template had specific and non-specific effects. An increase in TALEN mRNA for IL2Rg led to an increase in both NHEJ and HDR for IL2Rg while NHEJ levels for RAG2 were unchanged. An increase in IL2Rg HDR template reduced HDR at the RAG2 locus suggesting a nonspecific inhibition of homology directed repair by escalation of the concentration of oligonucleotide. This dose sensitivity, particularly at these low doses, has possibly lead others away from pursuit of multiplex processes. Cells from Example 1 have been cloned and, at the time of filing, two animals are pregnant with embryos derived from the same.

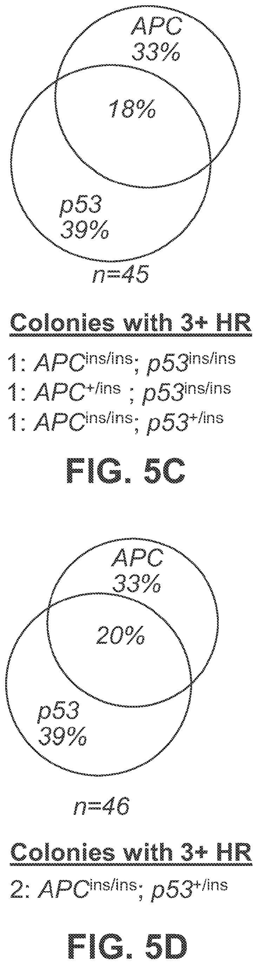

Example 2, see FIG. 5, describes experiments that had the same goal of multiplex HDR editing but for different genes. The first gene target was Adenomatous polyposis coli (APC). The second gene target was p53 (the TP53 gene). Cells homozygous for both knockouts and cells heterozygous for both knockouts were detected and isolated.

Example 3, see FIGs. 6-9, describes multiplex HDR editing to knockout 2-5 genes. There were three experiments, with the number of cell colonies tested for genotype ranging from 72-192 for each experiment. Cells were treated for multiplex knockout of various combinations the genes APC, p53, RAG2, Low Density Lipoprotein Receptor (LDLR), IL2Rg, Kisspeptin Receptor (KISSR or GPR54), and Eukaryotic Translation Initiation Factor 4GI (EIF4GI). The gene LDLR was consistently less amenable to modification than the other genes. As is evident from the results, multiple alleles can be disrupted simultaneously using the TALEN-specified, homology directed repair (HDR). Five TALEN pairs that each resulted in more than 20% HDR/site and their cognate HDR templates were simultaneously co-transfected in three combinations (Table A). A proportion of colonies from each replicate were positive for HDR events in at least four genes and two colonies from replicate-A had HDR events in all five genes. Although simultaneous indel formation in five genes has been demonstrated by Cas9/CRISPR-stimulated NHEJ in mouse ES cells, the precise modification of 5 genes (up to 7 alleles) by targeted nuclease-stimulated HDR is unexpected, surprising, and unrivaled. When the TALENs of replicate were replaced Cas9/CRISPRs (vectors were introduced into cells to express), modification levels were below detection (data not shown); however, other data points to RGEN multiplex, e.g., Example 9 below. Four genes were found to be edited in all experiments and five genes in one experiment.

The speed and efficiency of this process lends itself to scaling-up such that the multiplex knockout of more than 5 genes is achievable without changing the nature of the process. Referring to Table A, about 72 to 192 cells were tested; now that this process has been established it is not unreasonable to increase the number of tests to a very much larger number of cells such that multiplex of larger numbers of genes/alleles can be expected. A number of multiplex genes or alleles may be from 2-25; artisans will immediately appreciate that all ranges and values between the explicitly stated bounds are contemplated, with any of the following being available as an upper or lower limit in combination with each other: 2, 4, 5, 6, 7, 8, 9, 10, 11, 12, 14, 16, 18, 20, 25.

| **Table A: Multiplex HDR in pig fibroblasts** | | | |
|---|---|---|---|
| Genes edited | Rep A # (percent) | Rep B # (percent) | Rep C # (percent) |
| 5 | 2 (3) | 0 | 0 |
| 4 | 0 | 5 (5) | 4 (2) |
| 3 | 3 (4) | 7 (7) | 14 (7) |
| 2 | 12 (17) | 23 (24) | 41 (21) |
| 1 | 24 (33) | 29 (30) | 47 (24) |
| 1+ | 41 (57) | 63 (66) | 106 (55) |
| Genes targeted in each replicate: | | | |
| *A.* | *APC, LDLR, RAG2, IL2Rg, p53.* | | |
| *B.* | *APC, LDLR, RAG2, KISSR, EIF4G1* | | |
| C. | *APC, LDLR, RAG2, KISSR, DMD* | | |

As is evident, cells and embryos with multiplex knockouts are embodiments of the invention, as well as animals made thereby.

Example 4 describes some detailed processes for making various animals and refers to certain genes by way of example. Example 5 describes examples of CRISPR/Cas9 design and production.

Example 6 provides further examples of multiplex gene editing with targeted nucleases driving HDR processes. GATA binding protein 4 (GATA4); homeobox protein NKX2-5 (NKX2-5) and Mesoderm Posterior Protein 1 (MESP1) were simultaneously targeted with TALENs and HDR templates to direct frame-shift mutations and premature stop-codons into each gene. The objective was to create biallelic knockouts for each gene for use in complementation studies. The process was about 0.5% efficient as 2 clones had the intended biallelic HDR at each gene. The given genes knocked out singly or in combination genes will cause a failure to thrive genotype and early embryonic lethality without complementation. Artisans will appreciate that knockout of these genes individually and interbreeding of heterozygotes to obtain triple knockouts (about 1/66 chance) for FTT and complementation studies is not feasible in livestock.

Example 7 provides data that TALENs and Cas9/CRISPR can be mixed to perform multiplex editing of genes. Some genes/alleles are more readily targeted by a TALEN, or Cas9/CRISPR and that the situation may arise that multiplexing must be done with a combination of these tools. In this example, the Eukaryotic Translation Initiation Factor 4GI (EIF4GI) was targeted by TALENs and the p65 (RELA) gene was targeted by Cas9/CRISPR. The cells were analyzed by RFLP assay, indicative of HDR events, and HDR was evident at both sites. Accordingly, TALENs and RGENs may be used together or separately for multiplexing Combinations including, for example, 1, 2, 3 4, 5, 6, 7, 8, 9 or 10 TALENs with 1, 2, 3 4, 5, 6, 7, 8, 9 or 10 RGEN reagents, in any combination.

### Chimeras

Chimeras can be made by preparing a host blastocyst and adding a donor cell from a donor animal. The resultant animal will be a chimera that has cells that originate from both the host and the donor. Some genes are required for the embryo to create certain kinds of cells and cell lineages. When such a gene is knocked out in the host cells, the introduction of a donor cell that has the missing gene can result in those cells and cell lineages being restored to the host embryo; the restored cells have the donor genotype. Such a process is referred to as a complementation process.

Matsunari et al., PNAS 110:4557-4562, 2013, described a complementation process for making a donor-derived pig pancreas. They made a host pig blastocyst that was altered to prevent formation of a functional pancreas. They made the host blastocyst by somatic cell cloning. The somatic cell had been modified to overexpress Hes1 under the promoter of Pdx1 (pancreatic and duodenal homeobox 1), which was known to inhibit pancreatic development. The added donor cells to the host blastocyst that did not have this modification; the donor cells supplied the cell lineages needed to make the pancreas. They had already demonstrated elsewhere that functional organs can be generated from pluripotent stem cells (PSCs) in vivo by blastocyst complementation in organogenesis-disabled mouse embryos. They proposed future research using xenogenic pluripotent stem cells (PSCs), including human induced PSCs. Indeed, xenotransplantation has been considered a potential solution to the organ/tissue shortage for greater than 40 years. The fact that no genes were knocked out to disable the formation of the pancreas is significant.

Knocking out even one gene in a large vertebrate is a significant investment of resources using conventional processes. In contrast, overexpression of a gene product in a cell is readily achieved using the present state of the art, for instance, with a plasmid or a vector that places multiple gene cassette copies into the genome. Adding expression of a gene is easier than targeting a gene and knocking it out. The ability to prevent organogenesis by overexpression of a gene product is believed to be unusual at this time. In fact, limitations in the ability to engineer large animal genomes can be significant. Nonetheless, the pig is the preferred donor animal for xenotransplantation due to its similarity in size and physiology to humans as well as its high fecundity and growth rate.

FIG 10 depicts a multiplex process used herein to make gene knockouts or other gene edits as applied in the context of chimeras. Low-passage primary somatic cells are made with gene knockouts. Cells with exactly the desired distribution of heterozygosity and homozygosity for the knockouts are isolated. These cells are used in cloning to make an embryo that is allowed to develop as a host blastocyst. The term blastocyst is used broadly herein to refer to embryos from two cells to about three weeks. The term embryo is used broadly to refer to animals from zygote to live birth. A donor embryo is established and used as a source of donor cells that provide genes to populate the niche created by the knockouts. The donor cells are introduced into the host blastocyst and reproduce with the host cells to form a chimera having both host and donor cells. The embryo is transferred to a surrogate female and gestated. The progeny of the chimera have host genotypes when the host cells form the gametes. Chimeras have their gender determined by their host blastocyst.

FIG 11 illustrates a failure to thrive phenotype (FTT) complementation process. FTT refers to animals that are not expected to live to an age of sexual maturity. A host embryo is provided with an FTT genotype and phenotype. Multiplex processes are ideal because the FTTs available by knockout of just one gene are limited and are not known for some organs and tissues. The donor cells provide the genes missing in the FTT and provide the missing cell types. The embryo can be a large vertebrate animal and the knockouts can be multiplex, e.g., 2-25 genes. Moreover, targeted endonucleases can be used to achieve a knockout. In an immunodeficiency embodiment, an IL2Rg-/y RAG2-/- knockout is the FTT because the host is essentially missing immune functions. But the donor cells do not have those genes missing and the resultant chimera has an essentially normal phenotype for purposes of being able to raise and maintain the animal. But the progeny has the FTT phenotype. The animals can thus be maintained and FTT animals conveniently produced. The chimeras can be any combination of heterozygous and homozygous for the knockouts. Processes for making chimera are thus described that are F0 generation animals that produce failure to thrive (FTT) phenotypes when other processes require an additional generation, or more.

Chimera normally pass on the genetics of the host cells. Disclosed herein, however, are alternative chimeras that pass the donor cell genetics to their progeny and not the host cell genetics. It turns out that switching the genetic inheritance can create some useful opportunities. Referring to FIG. 12, an embryo labeled as G⁻ host is depicted. The embryo has been prepared with nonfunctional gametes. A donor blastocyst is prepared and used as a source of donor cells. The donor cells provide the genes and cell lineages that are needed to make donor gametes. The resultant chimera has the gametes of the donor cells and creates progeny having donor cell genetics. In the illustration, the host embryo is a male Brahman bull. The donor cells are from a double-muscled bull. The chimera has a Brahman bull phenotype but its progeny are double muscled. The host and donors may be from the same or different breeds or same or different species. The host has been prepared to be sterile, meaning that it cannot sexually reproduce. Some sterile animals may be used to make gametes that are nonfunctional, e.g., immotile sperm, or not make gametes at all, e.g., with early gametogenesis being disrupted. The donor cells may be, for instance, wild-type cells, cells from animal breeds having desirable traits, or genetically modified cells.

Embodiments of the invention include chimeric sterile animals, such as chimeric livestock, that have a genetic modification to a chromosome that prevents gametogenesis or spermatogenesis. The chromosome may be an X chromosome, a Y chromosome, or an autosome. The modification may include a disruption of an existing gene. The disruption may be created by altering an existing chromosomal gene so that it cannot be expressed, or by genetically expressing factors that will inhibit the transcription or translation of a gene. The term gametogenesis means the production of haploid sex cells (ova and spermatozoa) that each carry one-half the genetic compliment of the parents from the germ cell line of each parent. The production of spermatozoa is spermatogenesis. The fusion of spermatozoa and ova during fertilization results in a zygote cell that has a diploid genome. The term gametogenic cell refers to a progenitor to an ovum or sperm, typically a germ cell or a spermatogonial cell. One embodiment is a knockout of spermatogonial stem cells (SSC) in the host. The animal may be made with donor cells that have desirable genetics and supplies SSC cells that make gametes with the donor genotype. Some genes are disrupted in combination to produce one or more effects that cause infertility, for instance, combinations of: Acr/H1.1/Smcp, Acr/Tnp2/Smcp, Tnp2/H1.1/Smcp, Acr/H1t/Smcp, Tnp2/H1t/Smcp (Nayernia K; Drabent B; Meinhardt A; Adham IM; Schwandt I; Muller C; Sancken U; Kleene KC; Engel W Triple knockouts reveal gene interactions affecting fertility of male mice. Mol. Reprod. Dev 70(4):406-16, 2005). Embodiments include a first line of animals with a knockout of a first gene or genes and a second line of animals with a knockout of a second gene or genes so that male progeny of the lines are infertile.

"Humanized" as used herein refers to an organ or tissue harvested from a non-human animal whose protein sequences and genetic complement are more similar to those of humans than the non-human host.

"Organ" as used herein refers to a collection of tissues joined in a structural unit to serve a common function. "Tissue" as used herein refers to a collection of similar cells from the same origin that together carry out a specific function.

The use of genetic engineering to create genetically modified large vertebrates will accelerate the creation of animals with desirable traits. Traditional livestock breeding is an expensive and time consuming process that involves careful selection of genetic traits and lengthy waits for generational reproduction. Even with careful trait selection, the variations of sexual reproduction present a considerable challenge in cultivating and passing on desirable trait combinations. But creation of chimeras that pass on donor traits creates methods of animal reproduction that allow for rapid dissemination of desirable genetic traits, as well as for protection of the proprietary control of the traits. Embodiments include the production of genetically and genomically sterile animals that can serve as hosts for donated genetic material. Sexual intercourse by the host will lead to reproduction of the donor's genetic material. A group of genetically sterile animals can be used to disseminate identical genes from a single donor by sexual reproduction so that many donor progeny may be rapidly generated. Embodiments include animals that are modified to produce only one gender of animal so that users receiving the animals will not be able to easily breed the animals with the traits.

Embodiments include making a genetic modification to cells or embryos to inactivate a gene or plurality of genes selective for gametogenesis or spermatozoa activity. One process of genetic modification involves introduction of a targeted nuclease, e.g., a Cas9/CRISPR or mRNA for a TALEN pair that specifically binds to the gene. An animal is cloned from the cells or the modified embryo is directly raised in a surrogate mother. The animal may be a livestock animal or other animal. Gametogenesis may be blocked at an early stage. Or spermatozoa activity may be disrupted that is essential for fertility but is not otherwise essential to the animal. The animal is thus sterile because it cannot sexually reproduce: however, ARTs may be used to create progeny from the modified sperm. A donor animal that has desirable genetic traits (as a result of breeding and/or genetic engineering) is selected.

### Rapid establishment of F0 generation founder animal lines with two or more knockouts

With multiplex, two, three, or more genes (2-25) may be simultaneously knocked out to produce an F0 generation with the desired combination of alleles. If homozygosity for all of the knockouts creates an FTT, then one option is to make the founders homozygous for all of the knockouts except for one - or whatever the minimum heterozygosity should be for that situation. The one heterozygote gene can allow for a non-FTT phenotype. Alternatively, the multiplex knockouts can be used in combination with complementation to make thriving chimera that have FTT progeny. This process can eliminate generations in the creation of a multiple knockout animal.

In either case, the advantages are large and move many processes into the realm of actually being achievable. Producing animals with knockouts of two loci by conventional breeding is cost prohibitive as only ∼6% of offspring would have the desired phenotype in the F2 generation (Table B). In contrast, the multiplex approach enables production of the desired genotype in the F0 generation, a large advantage over conventional knockouts and breeding. It should be stressed that the saving of time and animals is not theoretical: it is an advance that makes some kinds of modifications possible because success is expected instead of failure. Furthermore, to continue the example, breeding between one or two chimeric RG-KO parents would significantly increase the production rate of RG-KO offspring to 25 and 100 percent respectively (Table B).

| **Table B: Breeding advantage of chimeric pigs.** | | | |
|---|---|---|---|
| **Male** | | **Female** | **% RG-KO** |
| *Chimera-IL2Rg*^{y/*-*}; *RAG2*^{*-*/*-*} | X | *Chimera-IL2Rg*^{*-*/*-*}; *RAG2*^{*-*/*-*} | 100% |
| *Chimera- IL2Rg*^{y/*-*}; *RAG2*^{*-*/*-*} | X | *IL2Rg*^{+/-}; *RAG2*^{+/-} | 25% |
| *IL2Rg*^{y/+}; *RAG2*^{+/-} | X | *IL2Rg*^{+/*-*}; *RAG2*^{+/*-*} | 6.3% |

### Immunodeficient animals

One group of embodiments relates to immunodeficient pigs or other livestock and processes of making them. These embodiments are examples of multiplex edits, e.g., knockouts that take advantage of the opportunity to manage selection of homozygous and heterozygous knockout genotypes. These demonstrate the power of multiplex to rapidly establish founder lines. They also include further aspects of the inventions that involve making chimeras.

The pig is the most relevant, non-primate animal model that mimics the size and physiology of humans. Unfortunately, fully immunodeficient pigs are not widely available because (1) multiple gene knockouts (KOs) are required, (2) intercrossing to create multi-locus null animals is extremely costly and depending on the number of Kos may be possible, and (3) only small scale germ-free facilities are available for pigs. Herein, embodiments include large vertebrate animals with a knockout of both *RAG2* and *IL2Rg* (i.e., RG-KO). The term large vertebrate refers to simians, livestock, dogs, and cats. The term livestock refers to animals customarily raised for food, such as cattle, sheep, goats, avian (chicken, turkey), pigs, buffalo, and fish. The genes can be knocked out of somatic cells that are then used for cloning to produce a whole animal. Alternatively, embryos can be treated to knockout the genes, with the animals being derived directly from the embryos. The multiplex gene-targeting platform can simultaneously disrupt of T, B and NK cell development in the pig. Accordingly, animals made without such cells can be made directly with the methods herein, as F0 founders, but the phenotype is FTT.

### Agricultural Targets for Multiplex Edits

The editing of food animal genomes can be greatly accelerated by editing numerous loci at the same time, saving generations of animal breeding that would be required to bring together alleles that are generated instead one at a time. In addition, some agricultural traits are complex, meaning that they are manifest as a result of the influence of alleles at more than one gene (from 2 to hundreds). For example, polymorphisms at DGAT, ABCG2, and a polymorphism on chromosome 18 together account for a large portion of the variation in Net Dairy Merit in dairy cattle. Livestock cells or embryos can be subjected to multiplex editing of numerous genes, including various agricultural targets: one or more of ACAN, AMELY, BLG, BMP 1B (FecB), DAZL, DGAT, Eif4GI, GDF8, Horn-poll locus, IGF2, CWC15, KissR/GRP54, OFD1Y, p65, PRLR, Prmd14, PRNP, Rosa, Socs2, SRY, ZFY, β-lactoglobulin, CLPG.

### Disease Modeling Targets For Multiplexing:

Some traits, like cancer, are caused on the basis of mutations at multiple genes (see APC/p53). In addition numerous disease traits are so-called Complex traits that manifest as a result of the influence of alleles at more than one gene. For example, diabetes, metabolism, heart disease, and neurological diseases are considered complex traits. Embodiments include animal models that are heterozygous and homozygous for individual alleles, or in combination with alleles at other genes, in different combinations. For example mature onset diabetes of the young (MODY) loci cause diabetes individually and additively, including; MODY 1 (HNF4α), MODY 2 (GCK), MODY 3 (HNF1α), MODY 4 (Pdx1), MODY 5 (HNF-1β), MODY 6 (eurogenic differentiation 1), MODY 7 (KLF11), MODY 8 (CEL), MODY 9 (PAX4), MODY 10 (INS), MODY 11 (BLK). Livestock cells or embryos can be subjected to multiplex editing of numerous genes for animal modelling, including various disease modeling targets: APC, ApoE, DMD, GHRHR, HR, HSD11B2, LDLR, NF1, NPPA, NR3C2, p53, PKD1, Rbm20, SCNN1G, tP53, DAZL, FAH, HBB, IL2RG, PDX1, PITX3, Runx1, RAG2, GGTA. Embodiments include cells, embryos, and animals with one or more of the above targets being edited, e.g., KO.

Genes in one species consistently have orthologs in other species. Humans and mice genes consistently have orthologs in livestock, particularly among cows, pigs, sheep, goats, chicken, and rabbits. Genetic orthologs between these species and fish is often consistent, depending upon the gene's function. Biologists are familiar with processes for finding gene orthologs so genes may be described herein in terms of one of the species without listing orthologs of the other species. Embodiments describing the disruption of a gene thus include disruption of orthologs that have the same or different names in other species. There are general genetic databases as well as databases that are specialized to identification of genetic orthologs. Moreover, artisans are familiar with the commonly used abbreviations for genes and using the context to identify which gene is being referred to in case there is more than one abbreviation for a gene or two genes are referred to by the same abbreviation.

Spermatogonial stem cells offer a second method genetic modification of livestock. Genetic modification or gene edits can be executed in vitro in spermatogonial stem cells isolated from donor testes. Modified cells are transplanted into germ cell-depleted testes of a recipient. Implanted spermatogonial stem cells produce sperm that carry the genetic modification(s) that can be used for breeding via artificial insemination or in vitro fertilization (IVF) to derive founder animals.

### Complementation of nullomorphic cell or organ loss by selective depopulation of host niches.

Multiplex editing can be used to purposefully ablate cells or organs from a specific embryonic or animal niche, creating an environment conducive to better donor cell integration, proliferation, and differentiation, enhancing their contribution by complementation of orthologous cells, tissues or organs in the embryo, fetus or animal. The animal with the empty niche is a deficiency carrier because it has been created to have a deficiency that can be filled by donor cells and genes. Specific examples include the recipient-elimination, and donor-rescue of gametogenic cell lineages (DAZL, VASA, MIWI, PIWI, and so forth.).

In another embodiment multiplex gene editing can be used to induce congenital alopecia, providing opportunity for donor derived cells to participate in hair folliculogenesis. The genes considered for multiplex gene editing to cause alopecia include those identified in OMIM and thru Human Phenotype Ontology database; DCAF17, VDR, PNPLA1, HRAS, Telomerase-vert, DSP, SNRPE, RPL21, LAMA3, UROD, EDAR, OFD1, PEX7, COL3A1, ALOX12B, HLCS, NIPAL4, CERS3, ANTXR1, B3GALT6, DSG4, UBR1, CTC1, MBTPS2 ,UROS, ABHD5, NOP10, ALMS1, LAMB3, EOGT, SAT1, RBPJ, ARHGAP31, ACVR1, IKBKG, LPAR6, HR, ATR, HTRA1, AIRE, BCS1L, MCCC2, DKC1, PORCN, EBP, SLITRK1, BTK, DOCK6, APCDD1, ZIP4, CASR, TERT, EDARADD, ATP6V0A2, PVRL1, MGP, KRT85, RAG2, RAG-1, ROR2, CLAUDIN1, ABCA12, SLA-DRA1, B4GALT7, COL7A1, NHP2, GNA11, WNT5A, USB1, LMNA, EPS8L3, NSDHL, TRPV3, KRAS, TINF2, TGM1, DCLRE1C, PKP1, WRAP53, KDM5C, ECM1, TP63, KRT14, RIPK4. Chimerism with donor cells that have folliculogenic potential may be used to grow human hair follicles. The ablation of organs or tissues in pigs or other vertebrates and growth of organs or tissues from human origins is particularly useful as a source of medical organs or tissues.

Further complementation targets for multiplexing are: PRKDC, BCL11a, BMI1, CCR5, CXCR4, DKK1, ETV2, FLI1, FLK1, GATA2, GATA4, HHEX, KIT, LMX1A, MYF5, MYOD1, MYOG, NKX2-5, NR4A2, PAX3, PDX1, PITX3, Runx1, RAG2, GGTA, HR, HANDII, TBX5.

Embodiments include targeting one, two, or more (2-25) of the above targets in a multiplex approach or by other approaches.

### Edited Genes

The methods and inventions described herein with respect to particular targets and targeted endonucleases are broadly applicable. The inventors have prepared primary livestock cells suitable for cloning with edits with all of the following genes..

| Table C: Primary livestock cells suitable for cloning, produced in swine and/or bovine fibroblasts by targeted endonucleases (TALENs) and HDR knockout. | | |
|---|---|---|
| Gene ID | Gene Name | Species |
| | | S: Swine |
| | | B: Bovine |
| ETV2 | Ets Variant 2 | S |
| PDX1 | Pancreatic and duodenal homeobox 1 | S |
| TBX4 | T-box transcription factor TBX4 | S |
| ID2 | DNA-binding protein inhibitor | S |
| SOX2 | SRY (sex determining region Y)-box 2 | S |
| TTF1/NKX2-1 | thyroid transcription factor 1/NK2 homeobox 1 | S |
| MESP1 | mesoderm posterior 1 homolog | S |
| GATA4 | GATA binding protein 4 | S |
| NKX2-5 | NK2 homeobox 5 | S |
| FAH | Fumarylacetoacetate Hydrolase | S |
| PRKDC | protein kinase, DNA-activated, catalytic polypeptide | S |
| RUNX1 | Runt-related transcription factor 1 | S |
| FLI1 | Friend leukemia integration 1 transcription factor | S |
| PITX3 | Pituitary homeobox 3 | S |
| LMX1A | LIM homeobox transcription factor 1, alpha | S |
| DKK1 | Dickkopf-related protein 1 | S |
| NR4A2/NURR1 | Nuclear receptor subfamily 4, group A, member 2/Nuclear receptor related 1 protein | S |
| FLK1 | Fetal Liver Kinase 1 | S |
| HHEX1 | Hematopoietically-expressed homeobox protein | S |
| BCL11A | B-cell lymphoma/leukemia 11A | S |
| RAG2 | Recombination activating gene 2 | S |
| RAG1 | Recombination activating gene 1 | S |
| IL2RG | Interleukin 2 receptor, gamma | S |
| c-KIT/SCFR | Mast/stem cell growth factor receptor | S |
| BMI1 | polycomb ring finger oncogene | S |
| HANDII | Heart- and neural crest derivatives-expressed protein 2 | S |
| TBX5 | T-box transcription factor 5 | S |
| GATA2 | GATA binding protein 2 | S |
| DAZL | Deleted in AZoospermia like | S, B |
| OLIG1 | oligodendrocyte transcription factor 1 | S |
| OLIG2 | oligodendrocyte transcription factor 2 | S |

### Genetically Modified Animals

Animals may be made that are mono-allelic or bi-allelic for a chromosomal modification, using methods that either leave a genetically expressible marker in place, allow for it to be bred out of an animal, or by methods that do not place such a marker in the animal. For instance, the inventors have used methods of homologous dependent recombination (HDR) to make changes to, or insertion of exogenous genes into, chromosomes of animals. Tools such as TALENs and recombinase fusion proteins, as well as conventional methods, are discussed elsewhere herein. Some of the experimental data supporting genetic modifications disclosed herein is summarized as follows. The inventors have previously demonstrated exceptional cloning efficiency when cloning from polygenic populations of modified cells, and advocated for this approach to avoid variation in cloning efficiency by somatic cell nuclear transfer (SCNT) for isolated colonies (Carlson et al., 2011). Additionally, however, TALEN-mediated genome modification, as well as modification by recombinase fusion molecules, provides for a bi-allelic alteration to be accomplished in a single generation. For example, an animal homozygous for a knocked-out gene may be made by SCNT and without inbreeding to produce homozygosity. Gestation length and maturation to reproduction age for livestock such as pigs and cattle is a significant barrier to research and to production. For example, generation of a homozygous knockout from heterozygous mutant cells (both sexes) by cloning and breeding would require 16 and 30 months for pigs and cattle respectively. Some have allegedly reduced this burden with sequential cycles of genetic modification and SCNT (Kuroiwa et al., 2004) however, this is both technically challenging and cost prohibitive, moreover, there are many reasons for avoiding serial cloning for making F0 animals that are to be actually useful for large vertebrate laboratory models or livestock. The ability to routinely generate bi-allelic KO cells prior to SCNT is a significant advancement in large animal genetic engineering. Bi-allelic knockout has been achieved in immortal cells lines using other processes such as ZFN and dilution cloning (Liu et al., 2010). Another group recently demonstrated bi-allelic KO of porcine GGTA1 using commercial ZFN reagents (Hauschild et al., 2011) where bi-allelic null cells could be enriched by FACS for the absence of a GGTA1-dependent surface epitope. While these studies demonstrate certain useful concepts, they do not show that animals or livestock could be modified because simple clonal dilution is generally not feasible for primary fibroblast isolates (fibroblasts grow poorly at low density) and biological enrichment for null cells is not available for the majority of genes.

Targeted nuclease-induced homologous recombination can be used so as to eliminate the need for linked selection markers. TALENs may be used to precisely transfer specific alleles into a livestock genome by homology dependent repair (HDR). In a pilot study, a specific 11bp deletion (the Belgian Blue allele) (Grobet et al., 1997; Kambadur et al., 1997) was introduced into the bovine GDF8 locus (see U.S. 2012/0222143). When transfected alone, the btGDF8.1 TALEN pair cleaved up to 16% of chromosomes at the target locus. Co-transfection with a supercoiled homologous DNA repair template harboring the 11bp deletion resulted in a gene conversion frequency (HDR) of up to 5% at day 3 without selection for the desired event. Gene conversion was identified in 1.4 % of isolated colonies that were screened. These results demonstrated that TALENs can be used to effectively induce HDR without the aid of a linked selection marker.

### Homology directed repair (HDR)

Homology directed repair (HDR) is a mechanism in cells to repair ssDNA and double stranded DNA (dsDNA) lesions. This repair mechanism can be used by the cell when there is an HDR template present that has a sequence with significant homology to the lesion site. Specific binding, as that term is commonly used in the biological arts, refers to a molecule that binds to a target with a relatively high affinity compared to non-target tissues, and generally involves a plurality of non-covalent interactions, such as electrostatic interactions, van der Waals interactions, hydrogen bonding, and the like. Specific hybridization is a form of specific binding between nucleic acids that have complementary sequences. Proteins can also specifically bind to DNA, for instance, in TALENs or CRISPR/Cas9 systems or by Gal4 motifs. Introgression of an allele refers to a process of copying an exogenous allele over an endogenous allele with a template-guided process. The endogenous allele might actually be excised and replaced by an exogenous nucleic acid allele in some situations but present theory is that the process is a copying mechanism. Since alleles are gene pairs, there is significant homology between them. The allele might be a gene that encodes a protein, or it could have other functions such as encoding a bioactive RNA chain or providing a site for receiving a regulatory protein or RNA.

The HDR template is a nucleic acid that comprises the allele that is being introgressed. The template may be a dsDNA or a single-stranded DNA (ssDNA). ssDNA templates are preferably from about 20 to about 5000 residues although other lengths can be used. Artisans will immediately appreciate that all ranges and values within the explicitly stated range are contemplated; e.g., from 500 to 1500 residues, from 20 to 100 residues, and so forth. The template may further comprise flanking sequences that provide homology to DNA adjacent to the endogenous allele or the DNA that is to be replaced. The template may also comprise a sequence that is bound to a targeted nuclease system, and is thus the cognate binding site for the system's DNA-binding member. The term cognate refers to two biomolecules that typically interact, for example, a receptor and its ligand. In the context of HDR processes, one of the biomolecules may be designed with a sequence to bind with an intended, i.e., cognate, DNA site or protein site.

### Targeted Endonuclease Systems

Genome editing tools such as transcription activator-like effector nucleases (TALENs) and zinc finger nucleases (ZFNs) have impacted the fields of biotechnology, gene therapy and functional genomic studies in many organisms. More recently, RNA-guided endonucleases (RGENs) are directed to their target sites by a complementary RNA molecule. The Cas9/CRISPR system is a REGEN. tracrRNA is another such tool. These are examples of targeted nuclease systems: these system have a DNA-binding member that localizes the nuclease to a target site. The site is then cut by the nuclease. TALENs and ZFNs have the nuclease fused to the DNA-binding member. Cas9/CRISPR are cognates that find each other on the target DNA. The DNA-binding member has a cognate sequence in the chromosomal DNA. The DNA-binding member is typically designed in light of the intended cognate sequence so as to obtain a nucleolytic action at nor near an intended site. Certain embodiments are applicable to all such systems without limitation; including, embodiments that minimize nuclease re-cleavage, embodiments for making SNPs with precision at an intended residue, and placement of the allele that is being introgressed at the DNA-binding site.

### TALENs

The term TALEN, as used herein, is broad and includes a monomeric TALEN that can cleave double stranded DNA without assistance from another TALEN. The term TALEN is also used to refer to one or both members of a pair of TALENs that are engineered to work together to cleave DNA at the same site. TALENs that work together may be referred to as a left-TALEN and a right-TALEN, which references the handedness of DNA or a TALEN-pair.

The cipher for TALs has been reported (PCT Publication WO 2011/072246) wherein each DNA binding repeat is responsible for recognizing one base pair in the target DNA sequence. The residues may be assembled to target a DNA sequence. In brief, a target site for binding of a TALEN is determined and a fusion molecule comprising a nuclease and a series of RVDs that recognize the target site is created. Upon binding, the nuclease cleaves the DNA so that cellular repair machinery can operate to make a genetic modification at the cut ends. The term TALEN means a protein comprising a Transcription Activator-like (TAL) effector binding domain and a nuclease domain and includes monomeric TALENs that are functional *per se* as well as others that require dimerization with another monomeric TALEN. The dimerization can result in a homodimeric TALEN when both monomeric TALEN are identical or can result in a heterodimeric TALEN when monomeric TALEN are different. TALENs have been shown to induce gene modification in immortalized human cells by means of the two major eukaryotic DNA repair pathways, non-homologous end joining (NHEJ) and homology directed repair. TALENs are often used in pairs but monomeric TALENs are known. Cells for treatment by TALENs (and other genetic tools) include a cultured cell, an immortalized cell, a primary cell, a primary somatic cell, a zygote, a germ cell, a primordial germ cell, a blastocyst, or a stem cell. In some embodiments, a TAL effector can be used to target other protein domains (e.g., non-nuclease protein domains) to specific nucleotide sequences. For example, a TAL effector can be linked to a protein domain from, without limitation, a DNA 20 interacting enzyme (e.g., a methylase, a topoisomerase, an integrase, a transposase, or a ligase), a transcription activators or repressor, or a protein that interacts with or modifies other proteins such as histones. Applications of such TAL effector fusions include, for example, creating or modifying epigenetic regulatory elements, making site-specific insertions, deletions, or repairs in DNA, controlling gene expression, and modifying chromatin structure.

The term nuclease includes exonucleases and endonucleases. The term endonuclease refers to any wild-type or variant enzyme capable of catalyzing the hydrolysis (cleavage) of bonds between nucleic acids within a DNA or RNA molecule, preferably a DNA molecule. Non-limiting examples of endonucleases include type II restriction endonucleases such as *Fok*I, *Hha*I*, Hind*lII*, Not*I*, Bbv*Cl*, Eco*RI*, Bgl*II*,* and *Alw*I. Endonucleases comprise also rare-cutting endonucleases when having typically a polynucleotide recognition site of about 12-45 basepairs (bp) in length, more preferably of 14-45 bp. Rare-cutting endonucleases induce DNA double-strand breaks (DSBs) at a defined locus. Rare-cutting endonucleases can for example be a targeted endonuclease, a chimeric Zinc-Finger nuclease (ZFN) resulting from the fusion of engineered zinc-finger domains with the catalytic domain of a restriction enzyme such as FokI or a chemical endonuclease. In chemical endonucleases, a chemical or peptidic cleaver is conjugated either to a polymer of nucleic acids or to another DNA recognizing a specific target sequence, thereby targeting the cleavage activity to a specific sequence. Chemical endonucleases also encompass synthetic nucleases like conjugates of orthophenanthroline, a DNA cleaving molecule, and triplex-forming oligonucleotides (TFOs), known to bind specific DNA sequences. Such chemical endonucleases are comprised in the term "endonuclease" according to the present invention. Examples of such endonuclease include *I-See I, I-Chu L I-Cre I, I-Csm I, PI-See L PI-Tti L PI-Mtu I, I-Ceu I, I-See IL 1- See III, HO, PI-Civ I, PI-Ctr L PI-Aae I, PI-Bsu I, PI-Dha I, PI-Dra L PI-Mav L PI-Meh I, PI-Mfu L PI-Mfl I, PI-Mga L PI-Mgo I, PI-Min L PI-Mka L PI-Mle I, PI-Mma I, PI- 30 Msh L PI-Msm I, PI-Mth I, PI-Mtu I, PI-Mxe I, PI-Npu I, PI-Pfu L PI-Rma I, PI-Spb I, PI-Ssp L PI-Fae L PI-Mja I, PI-Pho L PI-Tag L PI-Thy I, PI-Tko I, PI-Tsp I, I-MsoI.*

A genetic modification made by TALENs or other tools may be, for example, chosen from the list consisting of an insertion, a deletion, insertion of an exogenous nucleic acid fragment, and a substitution. The term insertion is used broadly to mean either literal insertion into the chromosome or use of the exogenous sequence as a template for repair. In general, a target DNA site is identified and a TALEN-pair is created that will specifically bind to the site. The TALEN is delivered to the cell or embryo, e.g., as a protein, mRNA or by a vector that encodes the TALEN. The TALEN cleaves the DNA to make a double-strand break that is then repaired, often resulting in the creation of an indel, or incorporating sequences or polymorphisms contained in an accompanying exogenous nucleic acid that is either inserted into the chromosome or serves as a template for repair of the break with a modified sequence. This template-driven repair is a useful process for changing a chromosome, and provides for effective changes to cellular chromosomes.

The term exogenous nucleic acid means a nucleic acid that is added to the cell or embryo, regardless of whether the nucleic acid is the same or distinct from nucleic acid sequences naturally in the cell. The term nucleic acid fragment is broad and includes a chromosome, expression cassette, gene, DNA, RNA, mRNA, or portion thereof. The cell or embryo may be, for instance, chosen from the group consisting non-human vertebrates, non-human primates, cattle, horse, swine, sheep, chicken, avian, rabbit, goats, dog, cat, laboratory animal, and fish.

Some embodiments involve a composition or a method of making a genetically modified livestock and/or artiodactyl comprising introducing a TALEN-pair into livestock and/or an artiodactyl cell or embryo that makes a genetic modification to DNA of the cell or embryo at a site that is specifically bound by the TALEN-pair, and producing the livestock animal/artiodactyl from the cell. Direct injection may be used for the cell or embryo, e.g., into a zygote, blastocyst, or embryo. Alternatively, the TALEN and/or other factors may be introduced into a cell using any of many known techniques for introduction of proteins, RNA, mRNA, DNA, or vectors. Genetically modified animals may be made from the embryos or cells according to known processes, e.g., implantation of the embryo into a gestational host, or various cloning methods. The phrase "a genetic modification to DNA of the cell at a site that is specifically bound by the TALEN", or the like, means that the genetic modification is made at the site cut by the nuclease on the TALEN when the TALEN is specifically bound to its target site. The nuclease does not cut exactly where the TALEN-pair binds, but rather at a defined site between the two binding sites.

Some embodiments involve a composition or a treatment of a cell that is used for cloning the animal. The cell may be a livestock and/or artiodactyl cell, a cultured cell, a primary cell, a primary somatic cell, a zygote, a germ cell, a primordial germ cell, or a stem cell. For example, an embodiment is a composition or a method of creating a genetic modification comprising exposing a plurality of primary cells in a culture to TALEN proteins or a nucleic acid encoding a TALEN or TALENs. The TALENs may be introduced as proteins or as nucleic acid fragments, e.g., encoded by mRNA or a DNA sequence in a vector.

### Zinc Finger Nucleases

Zinc-finger nucleases (ZFNs) are artificial restriction enzymes generated by fusing a zinc finger DNA-binding domain to a DNA-cleavage domain. Zinc finger domains can be engineered to target desired DNA sequences and this enables zinc-finger nucleases to target unique sequences within complex genomes. By taking advantage of endogenous DNA repair machinery, these reagents can be used to alter the genomes of higher organisms. ZFNs may be used in method of inactivating genes.

A zinc finger DNA-binding domain has about 30 amino acids and folds into a stable structure. Each finger primarily binds to a triplet within the DNA substrate. Amino acid residues at key positions contribute to most of the sequence-specific interactions with the DNA site. These amino acids can be changed while maintaining the remaining amino acids to preserve the necessary structure. Binding to longer DNA sequences is achieved by linking several domains in tandem. Other functionalities like non-specific FokI cleavage domain (N), transcription activator domains (A), transcription repressor domains (R) and methylases (M) can be fused to a ZFPs to form ZFNs respectively, zinc finger transcription activators (ZFA), zinc finger transcription repressors (ZFR, and zinc finger methylases (ZFM). Materials and methods for using zinc fingers and zinc finger nucleases for making genetically modified animals are disclosed in, e.g., U.S. 8,106,255; U.S. 2012/0192298; U.S. 2011/0023159; and U.S. 2011/0281306.

"Meganuclease" as used herein are another technology useful for gene editing and are endodeoxyribonucleases characterized by a large recognition site (double-stranded DNA sequences of 12 to 40 base pairs); as a result this site generally occurs only once in any given genome. For example, the 18-base pair sequence recognized by the I-SceI meganuclease would on average require a genome twenty times the size of the human genome to be found once by chance (although sequences with a single mismatch occur about three times per human-sized genome). Meganucleases are therefore considered to be the most specific naturally occurring restriction enzymes.

### Vectors and Nucleic acids

A variety of nucleic acids may be introduced into cells, for knockout purposes, for inactivation of a gene, to obtain expression of a gene, or for other purposes. As used herein, the term nucleic acid includes DNA, RNA, and nucleic acid analogs, and nucleic acids that are double-stranded or single-stranded (i.e., a sense or an antisense single strand). Nucleic acid analogs can be modified at the base moiety, sugar moiety, or phosphate backbone to improve, for example, stability, hybridization, or solubility of the nucleic acid. The deoxyribose phosphate backbone can be modified to produce morpholino nucleic acids, in which each base moiety is linked to a six membered, morpholino ring, or peptide nucleic acids, in which the deoxyphosphate backbone is replaced by a pseudopeptide backbone and the four bases are retained.

The target nucleic acid sequence can be operably linked to a regulatory region such as a promoter. Regulatory regions can be porcine regulatory regions or can be from other species. As used herein, operably linked refers to positioning of a regulatory region relative to a nucleic acid sequence in such a way as to permit or facilitate transcription of the target nucleic acid.

In general, type of promoter can be operably linked to a target nucleic acid sequence. Examples of promoters include, without limitation, tissue-specific promoters, constitutive promoters, inducible promoters, and promoters responsive or unresponsive to a particular stimulus. In some embodiments, a promoter that facilitates the expression of a nucleic acid molecule without significant tissue- or temporal-specificity can be used (i.e., a constitutive promoter). For example, a beta-actin promoter such as the chicken beta-actin gene promoter, ubiquitin promoter, miniCAGs promoter, glyceraldehyde-3-phosphate dehydrogenase (GAPDH) promoter, or 3-phosphoglycerate kinase (PGK) promoter can be used, as well as viral promoters such as the herpes simplex virus thymidine kinase (HSV-TK) promoter, the SV40 promoter, or a cytomegalovirus (CMV) promoter. In some embodiments, a fusion of the chicken beta actin gene promoter and the CMV enhancer is used as a promoter. See, for example, Xu et al., Hum. Gene Ther. 12:563, 2001; and Kiwaki et al., Hum. Gene Ther. 7:821, 1996.

Additional regulatory regions that may be useful in nucleic acid constructs, include, but are not limited to, polyadenylation sequences, translation control sequences (e.g., an internal ribosome entry segment, IRES), enhancers, inducible elements, or introns. Such regulatory regions may not be necessary, although they may increase expression by affecting transcription, stability of the mRNA, translational efficiency, or the like. Such regulatory regions can be included in a nucleic acid construct as desired to obtain optimal expression of the nucleic acids in the cell(s). Sufficient expression, however, can sometimes be obtained without such additional elements.

A nucleic acid construct may be used that encodes signal peptides or selectable expressed markers. Signal peptides can be used such that an encoded polypeptide is directed to a particular cellular location (e.g., the cell surface). Non-limiting examples of selectable markers include puromycin, ganciclovir, adenosine deaminase (ADA), aminoglycoside phosphotransferase (neo, G418, APH), dihydrofolate reductase (DHFR), hygromycin-B-phosphtransferase, thymidine kinase (TK), and xanthin-guanine phosphoribosyltransferase (XGPRT). Such markers are useful for selecting stable transformants in culture. Other selectable markers include fluorescent polypeptides, such as green fluorescent protein or yellow fluorescent protein.

In some embodiments, a sequence encoding a selectable marker can be flanked by recognition sequences for a recombinase such as, e.g., Cre or Flp. For example, the selectable marker can be flanked by *loxP* recognition sites (34-bp recognition sites recognized by the Cre recombinase) or FRT recognition sites such that the selectable marker can be excised from the construct. See, Orban et al., Proc. Natl. Acad. Sci., 89:6861, 1992, for a review of Cre/lox technology, and Brand and Dymecki, Dev. Cell, 6:7, 2004. A transposon containing a Cre- or Flp-activatable transgene interrupted by a selectable marker gene also can be used to obtain transgenic animals with conditional expression of a transgene. For example, a promoter driving expression of the marker/transgene can be either ubiquitous or tissue-specific, which would result in the ubiquitous or tissue-specific expression of the marker in F0 animals (e.g., pigs). Tissue specific activation of the transgene can be accomplished, for example, by crossing a pig that ubiquitously expresses a marker-interrupted transgene to a pig expressing Cre or Flp in a tissue-specific manner, or by crossing a pig that expresses a marker-interrupted transgene in a tissue-specific manner to a pig that ubiquitously expresses Cre or Flp recombinase. Controlled expression of the transgene or controlled excision of the marker allows expression of the transgene.

In some embodiments, the exogenous nucleic acid encodes a polypeptide. A nucleic acid sequence encoding a polypeptide can include a tag sequence that encodes a "tag" designed to facilitate subsequent manipulation of the encoded polypeptide (e.g., to facilitate localization or detection). Tag sequences can be inserted in the nucleic acid sequence encoding the polypeptide such that the encoded tag is located at either the carboxyl or amino terminus of the polypeptide. Non-limiting examples of encoded tags include glutathione S-transferase (GST) and FLAG™ tag (Kodak, New Haven, CT).

Nucleic acid constructs can be introduced into embryonic, fetal, or adult artiodactyl/livestock cells of any type, including, for example, germ cells such as an oocyte or an egg, a progenitor cell, an adult or embryonic stem cell, a primordial germ cell, a kidney cell such as a PK-15 cell, an islet cell, a beta cell, a liver cell, or a fibroblast such as a dermal fibroblast, using a variety of techniques. Non-limiting examples of techniques include the use of transposon systems, recombinant viruses that can infect cells, or liposomes or other non-viral methods such as electroporation, microinjection, or calcium phosphate precipitation, that are capable of delivering nucleic acids to cells.

In transposon systems, the transcriptional unit of a nucleic acid construct, i.e., the regulatory region operably linked to an exogenous nucleic acid sequence, is flanked by an inverted repeat of a transposon. Several transposon systems, including, for example, Sleeping Beauty (see, U.S. 6,613,752 and U.S. 2005/0003542); Frog Prince (Miskey et al., Nucleic Acids Res. 31:6873, 2003); Tol2 (Kawakami, Genome Biology 8(Suppl.1):S7, 2007); Minos (Pavlopoulos et al., Genome Biology, 8(Suppl.1):S2, 2007); Hsmar1 (Miskey et al., Mol Cell Biol., 27:4589, 2007); and Passport have been developed to introduce nucleic acids into cells, including mice, human, and pig cells. The Sleeping Beauty transposon is particularly useful. A transposase can be delivered as a protein, encoded on the same nucleic acid construct as the exogenous nucleic acid, can be introduced on a separate nucleic acid construct, or provided as an mRNA (e.g., an *in vitro*-transcribed and capped mRNA).

Nucleic acids can be incorporated into vectors. A vector is a broad term that includes any specific DNA segment that is designed to move from a carrier into a target DNA. A vector may be referred to as an expression vector, or a vector system, which is a set of components needed to bring about DNA insertion into a genome or other targeted DNA sequence such as an episome, plasmid, or even virus/phage DNA segment. Vector systems such as viral vectors (e.g., retroviruses, adeno-associated virus and integrating phage viruses), and non-viral vectors (e.g., transposons) used for gene delivery in animals have two basic components: 1) a vector comprised of DNA (or RNA that is reverse transcribed into a cDNA) and 2) a transposase, recombinase, or other integrase enzyme that recognizes both the vector and a DNA target sequence and inserts the vector into the target DNA sequence. Vectors most often contain one or more expression cassettes that comprise one or more expression control sequences, wherein an expression control sequence is a DNA sequence that controls and regulates the transcription and/or translation of another DNA sequence or mRNA, respectively.

Many different types of vectors are known. For example, plasmids and viral vectors, e.g., retroviral vectors, are known. Mammalian expression plasmids typically have an origin of replication, a suitable promoter and optional enhancer, and also any necessary ribosome binding sites, a polyadenylation site, splice donor and acceptor sites, transcriptional termination sequences, and 5' flanking non-transcribed sequences. Examples of vectors include: plasmids (which may also be a *carrier* of another type of vector), adenovirus, adeno-associated virus (AAV), lentivirus (e.g., modified HIV-1, SIV or FIV), retrovirus (e.g., ASV, ALV or MoMLV), and transposons (e.g., Sleeping Beauty, P-elements, *Tol-2,* Frog Prince, piggyBac).

As used herein, the term nucleic acid refers to both RNA and DNA, including, for example, cDNA, genomic DNA, synthetic (e.g., chemically synthesized) DNA, as well as naturally occurring and chemically modified nucleic acids, e.g., synthetic bases or alternative backbones. A nucleic acid molecule can be double-stranded or single-stranded (i.e., a sense or an antisense single strand). The term transgenic is used broadly herein and refers to a genetically modified organism or genetically engineered organism whose genetic material has been altered using genetic engineering techniques. A knockout artiodactyl is thus transgenic regardless of whether or not exogenous genes or nucleic acids are expressed in the animal or its progeny.

### Genetically modified animals

Animals may be modified using TALENs or other genetic engineering tools, including recombinase fusion proteins, or various vectors that are known. A genetic modification made by such tools may comprise disruption of a gene. The term disruption of a gene refers to preventing the formation of a functional gene product. A gene product is functional only if it fulfills its normal (wild-type) functions. Disruption of the gene prevents expression of a functional factor encoded by the gene and comprises an insertion, deletion, or substitution of one or more bases in a sequence encoded by the gene and/or a promoter and/or an operator that is necessary for expression of the gene in the animal. The disrupted gene may be disrupted by, e.g., removal of at least a portion of the gene from a genome of the animal, alteration of the gene to prevent expression of a functional factor encoded by the gene, an interfering RNA, or expression of a dominant negative factor by an exogenous gene. Materials and methods of genetically modifying animals are further detailed in U.S. 8,518,701; U.S. 2010/0251395; and U.S. 2012/0222143 which are hereby incorporated herein by reference for all purposes; in case of conflict, the instant specification is controlling. The term trans-acting refers to processes acting on a target gene from a different molecule (i.e., intermolecular). A trans-acting element is usually a DNA sequence that contains a gene. This gene codes for a protein (or microRNA or other diffusible molecule) that is used in the regulation the target gene. The trans-acting gene may be on the same chromosome as the target gene, but the activity is via the intermediary protein or RNA that it encodes. Embodiments of trans-acting gene are, e.g., genes that encode targeting endonucleases. Inactivation of a gene using a dominant negative generally involves a trans-acting element. The term cis-regulatory or cis-acting means an action without coding for protein or RNA; in the context of gene inactivation, this generally means inactivation of the coding portion of a gene, or a promoter and/or operator that is necessary for expression of the functional gene.

Various techniques known in the art can be used to inactivate genes to make knock-out animals and/or to introduce nucleic acid constructs into animals to produce founder animals and to make animal lines, in which the knockout or nucleic acid construct is integrated into the genome. Such techniques include, without limitation, pronuclear microinjection (U.S. 4,873,191), retrovirus mediated gene transfer into germ lines (Van der Putten et al., Proc. Natl. Acad. Sci. USA, 82:6148-6152, 1985), gene targeting into embryonic stem cells (Thompson et al., Cell, 56:313-321, 1989), electroporation of embryos (Lo, Mol. Cell. Biol., 3:1803-1814, 1983), sperm-mediated gene transfer (Lavitrano et al., Proc. Natl. Acad. Sci. USA, 99:14230-14235, 2002; Lavitrano et al., Reprod. Fert. Develop., 18:19-23, 2006), and *in vitro* transformation of somatic cells, such as cumulus or mammary cells, or adult, fetal, or embryonic stem cells, followed by nuclear transplantation (Wilmut et al., Nature, 385:810-813, 1997; and Wakayama et al., Nature, 394:369-374, 1998). Pronuclear microinjection, sperm mediated gene transfer, and somatic cell nuclear transfer are particularly useful techniques. An animal that is genomically modified is an animal wherein all of its cells have the genetic modification, including its germ line cells. When methods are used that produce an animal that is mosaic in its genetic modification, the animals may be inbred and progeny that are genomically modified may be selected. Cloning, for instance, may be used to make a mosaic animal if its cells are modified at the blastocyst state, or genomic modification can take place when a single-cell is modified. Animals that are modified so they do not sexually mature can be homozygous or heterozygous for the modification, depending on the specific approach that is used. If a particular gene is inactivated by a knock out modification, homozygousity would normally be required. If a particular gene is inactivated by an RNA interference or dominant negative strategy, then heterozygosity is often adequate.

Typically, in pronuclear microinjection, a nucleic acid construct is introduced into a fertilized egg; 1 or 2 cell fertilized eggs are used as the pronuclei containing the genetic material from the sperm head and the egg are visible within the protoplasm. Pronuclear staged fertilized eggs can be obtained *in vitro* or *in vivo* (i.e., surgically recovered from the oviduct of donor animals). *In vitro* fertilized eggs can be produced as follows. For example, swine ovaries can be collected at an abattoir, and maintained at 22-28°C during transport. Ovaries can be washed and isolated for follicular aspiration, and follicles ranging from 4-8 mm can be aspirated into 50 mL conical centrifuge tubes using 18 gauge needles and under vacuum. Follicular fluid and aspirated oocytes can be rinsed through pre-filters with commercial TL-HEPES (Minitube, Verona, WI). Oocytes surrounded by a compact cumulus mass can be selected and placed into TCM-199 OOCYTE MATURATION MEDIUM (Minitube, Verona, WI) supplemented with 0.1 mg/mL cysteine, 10 ng/mL epidermal growth factor, 10% porcine follicular fluid, 50 µM 2-mercaptoethanol, 0.5 mg/ml cAMP, 10 IU/mL each of pregnant mare serum gonadotropin (PMSG) and human chorionic gonadotropin (hCG) for approximately 22 hours in humidified air at 38.7°C and 5% CO₂. Subsequently, the oocytes can be moved to fresh TCM-199 maturation medium, which will not contain cAMP, PMSG or hCG and incubated for an additional 22 hours. Matured oocytes can be stripped of their cumulus cells by vortexing in 0.1% hyaluronidase for 1 minute.
For swine, mature oocytes can be fertilized in 500 µl Minitube PORCPRO IVF MEDIUM SYSTEM (Minitube, Verona, WI) in Minitube 5-well fertilization dishes. In preparation for *in vitro* fertilization (IVF), freshly-collected or frozen boar semen can be washed and resuspended in PORCPRO IVF Medium to 4 x 10⁵ sperm. Sperm concentrations can be analyzed by computer assisted semen analysis (SPERMVISION, Minitube, Verona, WI). Final *in vitro* insemination can be performed in a 10µl volume at a final concentration of approximately 40 motile sperm/oocyte, depending on boar. Incubate all fertilizing oocytes at 38.7°C in 5.0% CO₂ atmosphere for 6 hours. Six hours post-insemination, presumptive zygotes can be washed twice in NCSU-23 and moved to 0.5 mL of the same medium. This system can produce 20-30% blastocysts routinely across most boars with a 10-30% polyspermic insemination rate.

Linearized nucleic acid constructs can be injected into one of the pronuclei. Then the injected eggs can be transferred to a recipient female (e.g., into the oviducts of a recipient female) and allowed to develop in the recipient female to produce the transgenic animals. In particular, *in vitro* fertilized embryos can be centrifuged at 15,000 X g for 5 minutes to sediment lipids allowing visualization of the pronucleus. The embryos can be injected with using an Eppendorf FEMTOJET injector and can be cultured until blastocyst formation. Rates of embryo cleavage and blastocyst formation and quality can be recorded.

Embryos can be surgically transferred into uteri of asynchronous recipients. Typically, 100-200 (e.g., 150-200) embryos can be deposited into the ampulla-isthmus junction of the oviduct using a 5.5-inch TOMCAT® catheter. After surgery, real-time ultrasound examination of pregnancy can be performed.

In somatic cell nuclear transfer, a transgenic artiodactyl cell (e.g., a transgenic pig cell or bovine cell) such as an embryonic blastomere, fetal fibroblast, adult ear fibroblast, or granulosa cell that includes a nucleic acid construct described above, can be introduced into an enucleated oocyte to establish a combined cell. Oocytes can be enucleated by partial zona dissection near the polar body and then pressing out cytoplasm at the dissection area. Typically, an injection pipette with a sharp beveled tip is used to inject the transgenic cell into an enucleated oocyte arrested at meiosis 2. In some conventions, oocytes arrested at meiosis-2 are termed eggs. After producing a porcine or bovine embryo (e.g., by fusing and activating the oocyte), the embryo is transferred to the oviducts of a recipient female, about 20 to 24 hours after activation. See, for example, Cibelli et al., Science 280:1256-1258, 1998, and U.S. 6,548,741. For pigs, recipient females can be checked for pregnancy approximately 20-21 days after transfer of the embryos.

Standard breeding techniques can be used to create animals that are homozygous for the exogenous nucleic acid from the initial heterozygous founder animals. Homozygosity may not be required, however. Transgenic pigs described herein can be bred with other pigs of interest.

In some embodiments, a nucleic acid of interest and a selectable marker can be provided on separate transposons and provided to either embryos or cells in unequal amount, where the amount of transposon containing the selectable marker far exceeds (5-10 fold excess) the transposon containing the nucleic acid of interest. Transgenic cells or animals expressing the nucleic acid of interest can be isolated based on presence and expression of the selectable marker. Because the transposons will integrate into the genome in a precise and unlinked way (independent transposition events), the nucleic acid of interest and the selectable marker are not genetically linked and can easily be separated by genetic segregation through standard breeding. Thus, transgenic animals can be produced that are not constrained to retain selectable markers in subsequent generations, an issue of some concern from a public safety perspective.

Once transgenic animal have been generated, expression of an exogenous nucleic acid can be assessed using standard techniques. Initial screening can be accomplished by Southern blot analysis to determine whether or not integration of the construct has taken place. For a description of Southern analysis, see sections 9.37-9.52 of Sambrook et al., Molecular Cloning, A Laboratory Manual, second edition, Cold Spring Harbor Press, Plainview; NY., 1989. Polymerase chain reaction (PCR) techniques also can be used in the initial screening. PCR refers to a procedure or technique in which target nucleic acids are amplified. Generally, sequence information from the ends of the region of interest or beyond is employed to design oligonucleotide primers that are identical or similar in sequence to opposite strands of the template to be amplified. PCR can be used to amplify specific sequences from DNA as well as RNA, including sequences from total genomic DNA or total cellular RNA. Primers typically are 14 to 40 nucleotides in length, but can range from 10 nucleotides to hundreds of nucleotides in length. PCR is described in, for example PCR Primer: A Laboratory Manual, ed. Dieffenbach and Dveksler, Cold Spring Harbor Laboratory Press, 1995. Nucleic acids also can be amplified by ligase chain reaction, strand displacement amplification, self-sustained sequence replication, or nucleic acid sequence-based amplified. See, for example, Lewis, Genetic Engineering News 12:1, 1992; Guatelli et al., Proc. Natl. Acad. Sci. USA, 87:1874, 1990; and Weiss, Science 254:1292, 1991. At the blastocyst stage, embryos can be individually processed for analysis by PCR, Southern hybridization and splinkerette PCR (see, e.g., Dupuy et al. Proc Natl Acad Sci USA, 99:4495, 2002).

Expression of a nucleic acid sequence encoding a polypeptide in the tissues of transgenic pigs can be assessed using techniques that include, for example, Northern blot analysis of tissue samples obtained from the animal, *in situ* hybridization analysis, Western analysis, immunoassays such as enzyme-linked immunosorbent assays, and reverse-transcriptase PCR (RT-PCR).

### Interfering RNAs

A variety of interfering RNA (RNAi) are known. Double-stranded RNA (dsRNA) induces sequence-specific degradation of homologous gene transcripts. RNA-induced silencing complex (RISC) metabolizes dsRNA to small 21-23-nucleotide small interfering RNAs (siRNAs). RISC contains a double stranded RNAse (dsRNase, e.g., Dicer) and ssRNase (e.g., Argonaut 2 or Ago2). RISC utilizes antisense strand as a guide to find a cleavable target. Both siRNAs and microRNAs (miRNAs) are known. A method of disrupting a gene in a genetically modified animal comprises inducing RNA interference against a target gene and/or nucleic acid such that expression of the target gene and/or nucleic acid is reduced.

For example the exogenous nucleic acid sequence can induce RNA interference against a nucleic acid encoding a polypeptide. For example, double-stranded small interfering RNA (siRNA) or small hairpin RNA (shRNA) homologous to a target DNA can be used to reduce expression of that DNA. Constructs for siRNA can be produced as described, for example, in Fire et al., Nature 391:806, 1998; Romano and Masino, Mol. Microbiol. 6:3343, 1992; Cogoni et al., EMBO J. 15:3153, 1996; Cogoni and Masino, Nature, 399:166, 1999; Misquitta and Paterson Proc. Natl. Acad. Sci. USA, 96:1451, 1999; and Kennerdell and Carthew, Cell, 95:1017, 1998. Constructs for shRNA can be produced as described by McIntyre and Fanning (2006) BMC Biotechnology 6:1. In general, shRNAs are transcribed as a single-stranded RNA molecule containing complementary regions, which can anneal and form short hairpins.

The probability of finding a single, individual functional siRNA or miRNA directed to a specific gene is high. The predictability of a specific sequence of siRNA, for instance, is about 50% but a number of interfering RNAs may be made with good confidence that at least one of them will be effective.

Embodiments include an in vitro cell, an in vivo cell, and a genetically modified animal such as a livestock animal that express an RNAi directed against a gene, e.g., a gene selective for a developmental stage. The RNAi may be, for instance, selected from the group consisting of siRNA, shRNA, dsRNA, RISC and miRNA.

### Inducible systems

An inducible system may be used to control expression of a gene. Various inducible systems are known that allow spatiotemporal control of expression of a gene. Several have been proven to be functional in vivo in transgenic animals. The term inducible system includes traditional promoters and inducible gene expression elements.

An example of an inducible system is the tetracycline (tet)-on promoter system, which can be used to regulate transcription of the nucleic acid. In this system, a mutated Tet repressor (TetR) is fused to the activation domain of herpes simplex virus VP16 trans-activator protein to create a tetracycline-controlled transcriptional activator (tTA), which is regulated by tet or doxycycline (dox). In the absence of antibiotic, transcription is minimal, while in the presence of tet or dox, transcription is induced. Alternative inducible systems include the ecdysone or rapamycin systems. Ecdysone is an insect molting hormone whose production is controlled by a heterodimer of the ecdysone receptor and the product of the *ultraspiracle* gene (USP). Expression is induced by treatment with ecdysone or an analog of ecdysone such as muristerone A. The agent that is administered to the animal to trigger the inducible system is referred to as an induction agent.

The tetracycline-inducible system and the Cre/loxP recombinase system (either constitutive or inducible) are among the more commonly used inducible systems. The tetracycline-inducible system involves a tetracycline-controlled transactivator (tTA)/ reverse tTA (rtTA). A method to use these systems in vivo involves generating two lines of genetically modified animals. One animal line expresses the activator (tTA, rtTA, or Cre recombinase) under the control of a selected promoter. Another set of transgenic animals express the acceptor, in which the expression of the gene of interest (or the gene to be modified) is under the control of the target sequence for the tTA/rtTA transactivators (or is flanked by loxP sequences). Mating the two strains of mice provides control of gene expression.

The tetracycline-dependent regulatory systems (tet systems) rely on two components, i.e., a tetracycline-controlled transactivator (tTA or rtTA) and a tTA/rtTA-dependent promoter that controls expression of a downstream cDNA, in a tetracycline-dependent manner. In the absence of tetracycline or its derivatives (such as doxycycline), tTA binds to tetO sequences, allowing transcriptional activation of the tTA-dependent promoter. However, in the presence of doxycycline, tTA cannot interact with its target and transcription does not occur. The tet system that uses tTA is termed tet-OFF, because tetracycline or doxycycline allows transcriptional down-regulation. Administration of tetracycline or its derivatives allows temporal control of transgene expression in vivo. rtTA is a variant of tTA that is not functional in the absence of doxycycline but requires the presence of the ligand for transactivation. This tet system is therefore termed tet-ON. The tet systems have been used in vivo for the inducible expression of several transgenes, encoding, e.g., reporter genes, oncogenes, or proteins involved in a signaling cascade.

The Cre/lox system uses the Cre recombinase, which catalyzes site-specific recombination by crossover between two distant Cre recognition sequences, i.e., loxP sites. A DNA sequence introduced between the two loxP sequences (termed floxed DNA) is excised by Cre-mediated recombination. Control of Cre expression in a transgenic animal, using either spatial control (with a tissue- or cell-specific promoter) or temporal control (with an inducible system), results in control of DNA excision between the two loxP sites. One application is for conditional gene inactivation (conditional knockout). Another approach is for protein overexpression, wherein a floxed stop codon is inserted between the promoter sequence and the DNA of interest. Genetically modified animals do not express the transgene until Cre is expressed, leading to excision of the floxed stop codon. This system has been applied to tissue-specific oncogenesis and controlled antigene receptor expression in B lymphocytes. Inducible Cre recombinases have also been developed. The inducible Cre recombinase is activated only by administration of an exogenous ligand. The inducible Cre recombinases are fusion proteins containing the original Cre recombinase and a specific ligand-binding domain. The functional activity of the Cre recombinase is dependent on an external ligand that is able to bind to this specific domain in the fusion protein.

Embodiments include an in vitro cell, an in vivo cell, and a genetically modified animal such as a livestock animal that comprise a gene under control of an inducible system. The genetic modification of an animal may be genomic or mosaic. The inducible system may be, for instance, selected from the group consisting of Tet-On, Tet-Off, Cre-lox, and Hiflalpha. An embodiment is a gene set forth herein.

### Dominant Negatives

Genes may thus be disrupted not only by removal or RNAi suppression but also by creation/expression of a dominant negative variant of a protein which has inhibitory effects on the normal function of that gene product. The expression of a dominant negative (DN) gene can result in an altered phenotype, exerted by a) a titration effect; the DN PASSIVELY competes with an endogenous gene product for either a cooperative factor or the normal target of the endogenous gene without elaborating the same activity, b) a poison pill (or monkey wrench) effect wherein the dominant negative gene product ACTIVELY interferes with a process required for normal gene function, c) a feedback effect, wherein the DN ACTIVELY stimulates a negative regulator of the gene function.

### Founder animals, animal lines, traits, and reproduction

Founder animals (F0 generation) may be produced by cloning and other methods described herein. The founders can be homozygous for a genetic modification, as in the case where a zygote or a primary cell undergoes a homozygous modification. Similarly, founders can also be made that are heterozygous. The founders may be genomically modified, meaning that the cells in their genome have undergone modification. Founders can be mosaic for a modification, as may happen when vectors are introduced into one of a plurality of cells in an embryo, typically at a blastocyst stage. Progeny of mosaic animals may be tested to identify progeny that are genomically modified. An animal line is established when a pool of animals has been created that can be reproduced sexually or by assisted reproductive techniques, with heterogeneous or homozygous progeny consistently expressing the modification.

In livestock, many alleles are known to be linked to various traits such as production traits, type traits, workability traits, and other functional traits. Artisans are accustomed to monitoring and quantifying these traits, e.g., Visscher et al., Livestock Production Science, 40:123-137, 1994, U.S. 7,709,206, U.S. 2001/0016315, U.S. 2011/0023140, and U.S. 2005/0153317. An animal line may include a trait chosen from a trait in the group consisting of a production trait, a type trait, a workability trait, a fertility trait, a mothering trait, and a disease resistance trait. Further traits include expression of a recombinant gene product.

### Recombinases

Embodiments of the invention include administration of a targeted nuclease system with a recombinase (e.g., a RecA protein, a Rad51) or other DNA-binding protein associated with DNA recombination. A recombinase forms a filament with a nucleic acid fragment and, in effect, searches cellular DNA to find a DNA sequence substantially homologous to the sequence. For instance a recombinase may be combined with a nucleic acid sequence that serves as a template for HDR. The recombinase is then combined with the HDR template to form a filament and placed into the cell. The recombinase and/or HDR template that combines with the recombinase may be placed in the cell or embryo as a protein, an mRNA, or with a vector that encodes the recombinase. The disclosure of U.S. 2011/0059160 (U.S. Patent Application No. 12/869,232) is hereby incorporated herein by reference for all purposes; in case of conflict, the specification is controlling. The term recombinase refers to a genetic recombination enzyme that enzymatically catalyzes, in a cell, the joining of relatively short pieces of DNA between two relatively longer DNA strands. Recombinases include Cre recombinase, Hin recombinase, RecA, RAD51, Cre, and FLP. Cre recombinase is a Type I topoisomerase from P1 bacteriophage that catalyzes site-specific recombination of DNA between loxP sites. Hin recombinase is a 21kD protein composed of 198 amino acids that is found in the bacteria Salmonella. Hin belongs to the serine recombinase family of DNA invertases in which it relies on the active site serine to initiate DNA cleavage and recombination. RAD51 is a human gene. The protein encoded by this gene is a member of the RAD51 protein family which assists in repair of DNA double strand breaks. RAD51 family members are homologous to the bacterial RecA and yeast Rad51. *Cre* recombinase is an enzyme that is used in experiments to delete specific sequences that are flanked by loxP sites. FLP refers to Flippase recombination enzyme (FLP or Flp) derived from the 2µ plasmid of the baker's yeast *Saccharomyces cerevisiae.*

Herein, "RecA" or "RecA protein" refers to a family of RecA-like recombination proteins having essentially all or most of the same functions, particularly: (i) the ability to position properly oligonucleotides or polynucleotides on their homologous targets for subsequent extension by DNA polymerases; (ii) the ability topologically to prepare duplex nucleic acid for DNA synthesis; and, (iii) the ability of RecA/oligonucleotide or RecA/polynucleotide complexes efficiently to find and bind to complementary sequences. The best characterized RecA protein is from *E. coli;* in addition to the original allelic form of the protein a number of mutant RecA-like proteins have been identified, for example, RecA803. Further, many organisms have RecA-like strand-transfer proteins including, for example, yeast, *Drosophila,* mammals including humans, and plants. These proteins include, for example, Reel, Rec2, Rad51, Rad51B, Rad51C, Rad51D, Rad51E, XRCC2 and DMC1. An embodiment of the recombination protein is the RecA protein of *E. coli.* Alternatively, the RecA protein can be the mutant RecA-803 protein of *E. coli,* a RecA protein from another bacterial source or a homologous recombination protein from another organism.

### Compositions and kits

The present invention also provides compositions and kits containing, for example, nucleic acid molecules encoding site-specific endonucleases, CRISPR, Cas9, ZNFs, TALENs, RecA-gal4 fusions, polypeptides of the same, compositions containing such nucleic acid molecules or polypeptides, or engineered cell lines. An HDR may also be provided that is effective for introgression of an indicated allele. Such items can be used, for example, as research tools, or therapeutically.

### EXAMPLES

Methods are as follows unless otherwise noted.

### Tissue culture and transfection.

Pig were maintained at 37 at 5% CO₂ in DMEM supplemented with 10% fetal bovine serum, 100 I.U./ml penicillin and streptomycin, and 2mM L-Glutamine. For transfection, all TALENs and HDR templates were delivered through transfection using the NEON Transfection system (Life Technologies). Briefly, low passage Ossabaw, Landrace reaching 100% confluence were split 1:2 and harvested the next day at 70-80% confluence. Each transfection was comprised of 500,000-600,000 cells resuspended in buffer "R" mixed with TALEN mRNA and oligos and electroporated using the 100µl tips that provide a 100 µl working volume by the following parameters: input Voltage; 1800V; Pulse Width; 20ms; and Pulse Number; 1. Typically, 1-2 µg of TALEN mRNA and 1-4 µM of HDR templates (single stranded oligonucleotides) specific for the gene of interest were included in each transfection. Deviation from those amounts is indicated in the figures and legends. After transfection, cells were plated in a well of a 6-well dish for three days and cultured at either 30°C. After three days, cell populations were plated for colony analysis and/or expanded and at 37°C until at least day 10 to assess stability of edits.

### Surveyor mutation detection and RFLP analysis.

PCR flanking the intended sites was conducted using PLATINUM Taq DNA polymerase HiFi (Life Technologies) with 1 µl of the cell lysate according to the manufacturer's recommendations. The frequency of mutation in a population was analysed with the SURVEYOR Mutation Detection Kit (Transgenomic) according to the manufacturer's recommendations using 10 µl of the PCR product as described above. RFLP analysis was performed on 10 µl of the above PCR reaction using the indicated restriction enzyme. Surveyor and RFLP reactions were resolved on a 10% TBE polyacrylamide gels and visualized by ethidium bromide staining. Densitometry measurements of the bands were performed using IMAGEJ; and mutation rate of Surveyor reactions was calculated as described in Guschin et al., 2010(1). Percent homology directed repair (HDR) was calculated by dividing the sum intensity of RFLP fragments by the sum intensity of the parental band + RFLP fragments. RFLP analysis of colonies was treated similarly except that the PCR products were amplified by 1X MYTAQ RED MIX (Bioline) and resolved on 2.5% agarose gels.

### Dilution cloning:

Three days post transfection, 50 to 250 cells were seeded onto 10 cm dishes and cultured until individual colonies reached circa 5mm in diameter. At this point, 6 ml of TRYPLE (Life Technologies) 1:5 (vol/vol) diluted in PBS was added and colonies were aspirated, transferred into wells of a 24-well dish well and cultured under the same conditions. Colonies reaching confluence were collected and divided for cryopreservation and genotyping.

### Sample preparation:

Transfected cells populations at day 3 and 10 were collected from a well of a 6-well dish and 10-30% were resuspended in 50 µl of 1X PCR compatible lysis buffer: 10 mM Tris-Cl pH 8.0, 2 mM EDTA, 0.45% TRYTON X-100(vol/vol), 0.45% TWEEN-20(vol/vol) freshly supplemented with 200 µg/ml Proteinase K. The lysates were processed in a thermal cycler using the following program: 55°C for 60 minutes, 95°C for 15 minutes. Colony samples from dilution cloning were treated as above using 20-30 µl of lysis buffer.

| Table D: Listing of Endonuclease binding sequences and HDR templates. | | | |
|---|---|---|---|
| Gene | Example | Endonuclease | HDR Template |
| *Example* | | L: repeat-variable diresidue (RVD) code for left TALEN monomer | ssDNA oligo sequence, 5' to 3' |
| | | R: RVD code for right TALEN monomer | |
| | | OR | |
| | | Cas9/CRISPR, sgRNA: gRNA sequence, 5'to 3' | |
| *IL2Rγ* | 1, 3 | L: HD HD HD NI NI NI NN NN NG NG HD NI NN NG NN NG NG NG (SEQ ID NO:4) | TTCCACTCTACCCCCCCCAAAGG TTCAGTGTTTTGTGTAAGCTTCAA TGTTGAGTACATGAATTGCACTT GGGACAGCAGCTCTGAGCTC (SEQ ID NO:26) |
| | | R: HD HD NI NI NN NG NN HD NI NI NG NG HD NI NG NN NG NI HD NG (SEQ ID NO:5) | |
| *RAG2* | 1, 3 | L: NI HD HD NG NG HD HD NG HD HD NG HD NG HD HD NN HD NG (SEQ ID NO:6) | CTCTAAGGATTCCTGCCACCTTCC TCCTCTCCGCTACCCAGACTAAG CTTTGCACATTCAAAAGCAGCTT AGGGTCTGAAAAACATCAGT (SEQ ID NO:27) |
| | | R: HD NG NI NI NN HD NG NN HD NG NG NG NG NN NI NI NG (SEQ ID NO:7) | |
| *APC* | 2, 3 | L: NN NN NI NI NN NI NI NN NG NI NG HD NI NN HD HD NI NG (SEQ ID NO:8) | CCAGATCGCCAAAGTCACGGAAG AAGTATCAGCCATTCATCCCTCC CAGTGAAGCTTACAGAAATTCTG GGTCGACCACGGAGTTGCACT (SEQ ID NO:28) |
| | | R: NN NI HD HD HD NI NN NI NI NG NG NG HD NG NN NG (SEQ ID NO:9) | |
| *P53* | 2, 3 | L: NN NN HD NI HD HD HD NN NG NN NG HD HD NN HD NN HD (SEQ ID NO:10) | AGCTCGCCACCCCCGCCGGGCAC CCGTGTCCGCGCCATGGCCATCT AAGCTTAAAGAAGTCAGAGTACA TGCCCGAGGTGGTGAGGCGCT (SEQ ID NO:29) |
| | | R: HD NI NG NN NG NI HD NG HD NG NN NI HD NG NG(SEQ ID NO:11) | |
| *KISSR* | 3 | L: NN HD NG HD NG NI HD NG HD NG NI HD HD HD HD (SEQ ID NO:12) | GTGCTGCGTGCCCTTTACTGCTCT ACTCTACCCCCTACCAGCCTAAG CTTGTGCTGGGCGACTTCATGTG CAAGTTCCTCAACTACATCC (SEQ ID NO:30) |
| | | R: NN HD NI HD NI NG NN NI NI NN NG HD NN HD HD HD NI (SEQ ID NO:13) | |
| *EIF4GI* | 3, 7 | L: HD HD NN NG HD HD NG NG NG NN HD HD NI NI HD HD NG NG (SEQ ID NO:14) | CCCAGACTTCACTCCGTCCTTTGC CGACTTCGGCCGACCAGCCCTTA GCAACCGTGGGCCCCCAAGGGGT GGGCCAGGTGGGGAGCTGCC (SEQ ID NO:31) |
| | | R: NG NN NN NN NN NN HD HD HD NI HD NN NN NG NG NN HD NG (SEQ ID NO:15) | |
| *LDLR* | 3 | L: HD NG HD HD NG NI HD NI NI NN NG NN NN NI NG NG NG (SEQ ID NO:16) | CCGAGACGGGAAATGCACCTCCT ACAAGTGGATTTGTGATGGATCC GAACACCGAGTGCAAGGACGGG TCCGCTGAGTCCCTGGAGACGT (SEQ ID NO:32) |
| | | R: HD NN NN NI HD HD HD NN NG HD HD NG NG NN HD NI HD NG (SEQ ID NO:17) | |
| *DMD* | 3 | L: NN NN NI HD NG NN NI HD HD NI HD NG NI NG NG (SEQ ID NO:18) | AAAGTGGCCTGGCCCAACCCCTG GACTGACCACTCGAGTATTGAAG CACGTAAGTATGCTGGACCACAT TCTCTATGGCTGTAGACATTC (SEQ ID NO:33) |
| | | R: NI NN NI NN NI NI NG NN NG NN NN NG HD HD NI NN HD (SEQ ID NO:19) | |
| *NKX2-5* | 6 | L: HD NN HD NI NN NN HD NI HD NI NN NN NG HD NG NI HD (SEQ ID NO:20) | CTCTTTTCGCAGGCACAGGTCTA CGAGCTGGAGCGACGCTTCTAAG CTTGCAGCAGCGGTACCTGTCGG CTCCCGAGCGTGACCAGTTGG (SEQ ID NO:34) |
| | | R: NI HD HD NN HD NG NN HD NG NN HD NG NG NN NI (SEQ ID NO:21) | |
| *MESP1* | 6 | L: NN HD NN NN NG NG NN HD NG HD HD HD HD HD NN HD HD (SEQ ID NO:22) | TGCGGTTGCTCCCCCGCCTCGTCC CCGTAAGCTTGACTCCTGGTGCA GCGCCCCGGCCAG (SEQ ID NO:35) |
| | | R: NN NN HD HD NN NN NN NN HD NN HD NG NN HD NI HD HD (SEQ ID NO:23) | |
| *GATA4* | 6 | L: NI NG NN NG NG NG NN NI NG NN NI HD NG NG HD (SEQ ID NO:24) | AACCCTGTGTCGTTTCCCACCCA GTAGATATGTTTGATGACTAAGC TTCTCGGAAGGCAGAGAGTGTGT CAACTGCGGGCCATGTCCAC (SEQ ID NO:36) |
| | | R: NN NN HD HD HD HD NN HD NI NN NG NG NN NI HD NI HD (SEQ ID NO:25) | |
| *P65* | 7 | Cas9/CRISPR, sgRNA: CGTCACCAACGGTCTCCTC TCGG (SEQ ID NO:38) | GCTCCCACTCCCCTGGGGGCCTC TGGGCTCACCAACGGTCTCCTCC CGGGGGACGAAGACTTCTCCTCC ATTGCGGACATGGACTTCTCA (SEQ ID NO:37) |

### Example 1: Multiplex Gene Editing

Six conditions of TALEN mRNA and HDR templates directed to pig *RAG2* and *IL2Rγ* were co-transfected into pig fibroblasts. A fixed quantity of *RAG2* mRNA and template were used for each transfection whereas the quantity of *IL2Rg* TALEN mRNA and HDR template is altered for each condition as indicated. The dosage of TALEN mRNA and HDR template has both on and off target effects. An increase in TALEN mRNA for *IL2Rγ* led to an increase in both NHEJ and HDR for *IL2Rγ* while NHEJ levels for *RAG2* were unchanged. An increase in *IL2Rγ* HDR template reduced HDR at the *RAG2* locus suggesting a nonspecific inhibition of homology directed repair by escalation of the concentration of oligonucleotide. Colonies with bi-allelic HDR at *RAG2* and *IL2Rγ* were obtained at four and two percent from two conditions (FIG. 4 panel, c, panel d) which is at and above the expected frequency of two percent. The expected frequency is calculated by multiplication of day 3 HDR levels which treats each HDR allele as an independent event. Referring to FIG. 4, Multiplex gene editing of swine *RAG2* and *IL2Rγ* 4A) SURVEYOR and RFLP analysis to determine the efficiency of non-homologous end joining (NHEJ) and homology depended repair HDR on cell populations 3 days post transfection. 4B) RFLP analysis for homology dependent repair on cell populations 11 days post transfection. 4C) Percentage of colonies positive for HDR at *IL2Rγ*, *RAG2* or both. Cells were plated from the population indicated by a "4C" in panel a. Distribution of colony genotypes is shown below. 4D) Colony analysis from cells transfected with TALEN mRNA quantities of 2 and 1 µg for *IL2Rγ* and *RAG2* and HDR template at 1 µM for each. Distribution of colony genotypes is shown below.

### Example 2: Multiplex Gene Editing

Four conditions of TALEN mRNA and HDR templates directed to pig APC and *p53* were co-transfected into pig fibroblasts. The quantity of *APC* mRNA was sequentially reduced from left to right (FIG.5 panel b); the remaining of the quantities remained constant as indicated. Percent HDR reduced in a linear manor with reduction of *APC* mRNA. There was little effect on *p53* HDR with altered dosage of *APC* TALENs. Genotyping of colonies revealed a higher than expected union of clones with HDR allele in both *APC* and *p53* relative to the day 11 values; 18 and 20 percent versus 13.7 and 7.1 percent for FIG. 5C and Fig. 5D, respectively. Referring to Fig. 5 Multiplex gene editing of swine *APC* and *p53.* 5A) Surveyor and RFLP analysis to determine the efficiency of non-homologous end joining (NHEJ) and homology depended repair HDR on cell populations 3 days post transfection. 5B) RFLP analysis for homology dependent repair on cell populations 11 days post transfection. 5C) and 5D). Percentage of colonies positive derived from the indicated cell population (indicated in 5A, "5C" and "5D") for HDR at *APC, p53* or both. Colonies with 3 or more HDR alleles are listed below.

### Example 3: Multiplex with at least three genes

In Example 1, a non-specific reduction in HDR was observed at high concentration of HDR oligo, thus it was unknown whether 2+ HDR oligos could be effective without non-specific inhibition of HDR. Two concentrations were tested, 1 uM and 2 uM for each target site. While TALEN activity was not significantly altered between the two conditions, HDR was blunted significantly at 2 uM concentration for each template. Clones derived from the 1 uM condition had a variety of genotypes, some of those with edits in each gene and up to 7 alleles (FIG. 7). If treated as independent events, the expected frequency of the genotype denoted by an "a", with 7 alleles edited, is 0.001 percent. Binomial distribution predicts the likelihood of identifying 2+ colonies of such a genotype in a sample size of 72, as was done here, is less than 0.000026 percent. This high rate of success could not be predicted and is unexpected and surprising. This result was replicated with two addition combinations of TALENs/HDR template (FIGs. 8 and 9). As with the results the first trial, colonies were obtained with HDR edits in up to seven alleles and up to four genes (Table A). Several genotypes were recovered at a frequency far greater than anticipated by chance. Although a concern regarding simultaneous double strand break at several loci is induction of unintended chromosomal rearrangements, 50 of 50 karyotypes tested from trial 3 cells were normal (data not shown).

Referring to FIG. 6: Effect of Oligonucleotide HDR template concentration on 5-gene multiplex HDR efficiency. Indicated amounts of TALEN mRNA directed to swine *RAG2, IL2Rg, p53, APC* and *LDLR* were co-transfected into pig fibroblasts along with 2 uM (6A) or 1 uM (6B) of each cognate HDR template. Percent NHEJ and HDR were measured by Surveyor and RFLP assay.

Referring to FIG. 7: Colony genotypes from 5-gene multiplex HDR. Colony genotypes were evaluated by RFLP analysis. 7A) Each line represents the genotype of one colony at each specified locus. Three genotypes could be identified; those with the expected RFLP genotype of heterozygous or homozygous HDR as well as those with an RFLP positive fragment, plus a second allele that has a visible shift in size indicative of an insertion or deletion (indel) allele. The percentage of colonies with an edit at the specified locus is indicated below each column. 7B) A tally of the number of colonies edited at 0-5 loci.

Referring to FIG. 8: Colony genotypes of a second 5-gene multiplex trial. 8A) Each line represents the genotype of one colony at each specified locus. Three genotypes could be identified; those with the expected RFLP genotype of heterozygous or homozygous HDR as well as those with an RFLP positive fragment, plus a second allele that has a visible shift in size indicative of an insertion or deletion (indel) allele. The percentage of colonies with an edit at the specified locus is indicated below each column. 8B) A tally of the number of colonies edited at 0-5 loci.

Referring to FIG. 9: Colony genotypes a third 5-gene multiplex trial. 9A) Each line represents the genotype of one colony at each specified locus. Three genotypes could be identified; those with the expected RFLP genotype of heterozygous or homozygous HDR as well as those with an RFLP positive fragment, plus a second allele that has a visible shift in size indicative of an insertion or deletion (indel) allele. The percentage of colonies with an edit at the specified locus is indicated below each column. 9B) A tally of the number of colonies edited at 0-5 loci.

### Examples 4A-4D

Example 4A: Develop RAG2/IL2Rg null (RG-KO) pig fibroblasts by multiplex gene editing.

Male pig fetal fibroblasts will be transfected with TALENs and oligonucleotide templates for disruption of *RAG2* and *IL2Rg* using the inventors' previously defined methods (Tan, W., et al., Efficient nonmeiotic allele introgression in livestock using custom endonucleases. PNAS, 110(41):16526-16531, 2013.) RG-KO candidates will be identified by, e.g., restriction length polymorphism (RFLP) as confirmed by sequencing. At least about 5 validated RG-KO colonies will be pooled as a resource for cloning and chimera production.

### Example 4B: Production of chimeric embryos using RG-KO host blastocysts.

Host RG-KO embryos and female EGFP-labeled donor cells will be produced using chromatin transfer technology followed by in vitro culture to the blastocyst stage. RG-KO cells from Example 1 may be used. Day-7 inter cell mass clumps from EGFP blastocysts will be injected into day-6 RG-KO embryos prior to embryo transfer to a synchronized sow. Using this approach, Nagashima and colleagues observed chimerism in >50 percent of liveborn piglets (Nagashima H. et al., Sex differentiation and germ cell production in chimeric pigs produced by inner cell mass injection into blastocysts. Biol Reprod, 70(3):702-707, 2004). The male phenotype is dominant in injection chimeras for both mice and pigs. Therefore, XY RG-KO hosts injected with female donor cells will exclusively transmit male host genetics. Pregnancy checks will be conducted at appropriate times, e.g., days 25, 50, and 100. Pregnant sows at about 100 days of gestation will be monitored 4 times daily prior to C-section derivation of piglets by about day 114.

### Example 4C: Determine if non-chimeric offspring are deficient for T, B and NK cells.

Non-chimeric offspring will be tested to determine if they deficient for T, B and NK cells. The following process is one technique for the same. C-section derivation will be conducted on each sow carrying presumptive chimeras and one bred sow carrying wild-type piglets. Umbilical cord blood will be isolated from each piglet immediately after C-section derivation. Cord blood leukocytes will be evaluated by fluorescence-activated cell sorting (FACS) for T, B and NK cell populations as well as donor derived EGFP expression. In addition, chimerism will be evaluated by PCR from cord blood, ear and tail biopsy. This initial analysis will be completed within 6 hours of birth, such that non-chimeric piglets can be monitored closely and humanely euthanized with signs of infection. A portion of non-chimeric animals, or those lacking immune cells, will be euthanized for necropsy.

### Example 4D: Identify chimeric pigs and determine origin of T, B and NK cells.

Chimeric pigs will be tested to determine origin of T, B and NK cells. The following process is one technique for the same. Chimeric piglets will be identified using the methods above. Weekly evaluation of circulating lymphocytes and serum immunoglobulin will be compared between chimeric, non-chimeric and wild-type piglets over a 2 month period. Populations of sorted T, B and NK cells will be evaluated for EGFP expression and microsatellite analysis to confirm donor origin. The maintenance of samples and semen collections from chimeric pigs will be supported by RCI until Phase II funding is available.

### Sample Procedures for Examples A-D:

### Cord and peripheral blood FA CS.

Evaluation of blood lymphocytes and EGFP chimerism will be performed as previously described (2) with adaptations for porcine specimens. Cord blood will be collected from each piglet immediately after C-section delivery. A portion of the cord blood will be processed and cryopreserved for potential allograft treatments while the remainder will be used for FACS analysis of lymphocytes. Peripheral blood samples will be collected at 2, 4, 6 and 8 weeks of age by standard methods. RBCs will be removed and approximately 1-2E+5 cells will be distributed into tubes. Aliquots will be labeled with anti-pig antibodies for identification of T cells (CD4 and CD8), B cells (CD45RA ad CD3), NK cells (CD16 and CD3) and myeloid cells (CD3). Antigen expression will be quantified on the LS RII Flow Cytometer (BD Biosciences). Fluorophores will be carefully selected to enable multiplex evaluation of donor derived EGFP cells along with surface antigens. Single cell suspensions from the spleen will be analyzed by the same methods.

### Examinations

All major organs and tissues will be grossly examined for appropriate anatomic development and appropriate samples from all major organs and tissues including pancreas, liver, heart, kidneys, lungs, gastrointestinal, immune system (peripheral and mucosal lymph nodes and spleen), and CNS will be collected for DNA isolation. Single cell suspensions will be prepared from the spleen for FACS analysis. Tissues will be prepared for histological examination to further assess chimerism and for any alterations that may be associated with the chimeric state and for the presence of any underlying illness.

### Assessment of chimerism.

Quantitative PCR will be conducted on cord blood, ear, and tail biopsy using primers specific to the EGFP transgene and compared to a standard curve with known ratios of EGFP to wild type-cells. Specimens will also be evaluated for RG-KO alleles via the RFLP assay previously described. Engraftment of EGFP+ cells will be evaluated macroscopically on whole animals and organs during necropsy. Tissues from the major organs will be sectioned for EGFP immunohistochemistry and counterstained with DAPI (4', 6-diamidino-2-phenylindole) to determine the ratio of donor to host cells.

### Microsatellite analysis.

Animals are screened for informative microsatellites for host and donor genetics from those routinely used in the lab. Samples from tissues and blood (sorted lymphocytes or myeloid lineages, EGFP positive and negative) are evaluated. Relative quantities of donor versus host cells will be evaluated by multiplexed amplicon sequencing on the MISEQ instrument (Illumina).

### Animals

Non-chimeric pigs are made having an absence of T, B and NK cells in cord and peripheral blood. Chimeric pigs will have levels substantially similar to nearly wild-type levels. Moreover, T, B and NK cell positive chimeras will have substantially normal immune functions and remain healthy when reared in standard conditions.

### Example 5: CRISPR/Cas9 design and production.

Gene specific gRNA sequences were cloned into the Church lab gRNA vector (Addgene ID: 41824) according their methods. The Cas9 nuclease was provided either by co-transfection of the hCas9 plasmid (Addgene ID: 41815) or mRNA synthesized from RCIScript-hCas9. This RCIScript-hCas9 was constructed by sub-cloning the XbaI-AgeI fragment from the hCas9 plasmid (encompassing the hCas9 cDNA) into the RCIScript plasmid. Synthesis of mRNA was conducted as above except that linearization was performed using KpnI.

### Example 6: Multiplex gene editing with targeted endonucleases and HDR.

13A is a schematic of each gene in the multiplex experiment (depicted as a cDNA-exons denoted by alternating shades) and the site targeted by TALENS is indicated. The sequence coding the DNA binding domain for each gene is indicated below. Swine fibroblasts were co-transfected with 1 ug of each TALEN mRNA and 0.1 nMol of each HDR oligo (FIG. 13B), targeting each gene, designed to insert a premature termination codon as well as a novel HindIII RFLP site for genotyping. A total of 384 colonies were isolated for genotyping. The GATA4 and Nkx2-5 RFLP assays were performed (FIG. 13C) and MESP1 was evaluated by sequencing (not shown). Two colonies (2/384, 0.52%) were homozygous HDR knockouts for all three genes. The triple knockouts are labeled with asterisks (Fig. 13C). Additional genotypes can be observed in 13C, example colony 49 with no HDR edits; colony 52 and 63 with heterozygous edits to NKX2-5; colony 59 with heterozygous edits to both NKX2-5 and GATA4 and so on.

### Example 7: Multiplex gene-editing using a combination of TALENs and RGENs.

See FIG. 14. Swine fibroblasts were co-transfected with TALENS (1 ug EIF4G 14.1 mRNA) + Cas9/CRISPR components (2 ug Cas9 mRNA + 2 ug p65 G1s guide RNA) and 02 nMol of HDR oligo for each gene. Transfected cells were evaluated by RFLP assay revealing HDR at both sites. Cells from this population will be plated for colony isolation and isolates with edits in both genes are identified.

### Example 8: Human-porcine chimeric blastocysts.

A critical first step in creating human organs/cells in the pig using blastocyst complementation is to determine whether human stem cells can be incorporated into the inner cell mass as opposed to the trophectoderm and blastocoele cavity. To determine if human stem cells can become incorporated into the inner cell mass the inventors developed an assay system using parthenogenetic blastocysts. The parthenotes are created by the electrical activation of pig oocytes resulting in the formation of a diploid cell from the combination of DNA from the maternal pronucleus and the polar body. The single diploid cell then divides and the 6th day after activation becomes a well-formed blastocyst suitable for injection of human stem cells. Ten human umbilical cord blood stem cells (hUCBSC) were injected into individual porcine parthenogenetic blastocysts at day 6 post electrical activation. The inventors then examined the distribution of the hUCBSCs at day 7 and day 8, and also quantified the number of human stem cells at each time point using antibodies that recognize human nuclear antigen (HNA) to visualize individual hUCBSCs. It was found that the vast majority of the hUCBSC were incorporated into the inner cell mass (FIGs. 15A-15E, and 15F). Moreover, the hUCBSCs continued to proliferate during the two days post-injection into the blastocysts (FIG. 15g).

### Example 9: Human-porcine chimeric fetus.

Another critical step in creating human organs/cells via blastocyst complementation is the demonstration that porcine blastocysts injected with human stem cells can give rise to porcine fetuses containing human cells. To address this issue, hUCBSCs were injected into parthenogenetic blastocysts and transferred the chimeric blastocysts to hormonally synchronized sows. Fetuses were harvested at a gestational age of 28 days (FIG. 16A). Histological analysis of tissue sections revealed HNA-positive cells within internal organs of the chimeric fetus (FIG. 16B). These results demonstrate the ability of hUCBSCs to contribute to the developing porcine fetus.

Human-porcine chimeric fetus derived from complemented PITX3 knockout blastocysts. Porcine nigral dopamine neurons in pig-pig chimeras are also created and characterized; and human nigral dopamine neurons in human-pig chimeras. NURR1, LMX1A, and PITX3 knockout blastocysts will be generated using TALEN technology in fibroblasts and cloning. It will be determined whether the knockout blastocysts are capable of generating complementation based nigral dopamine neurons by using labeled porcine blastomeres as a source of stem cells. This approach has previously been used to generate exogenic pig-pig pancreas (Matsunari et al, 2013). Fetal pigs will be sacrificed at embryonic day 34-35 when the fetuses reach a crown-rump length of about 17 mm. At this stage of development the VM and other brain structures are comparable to the size of fetal rats at day E15 and the human fetus at mid first trimester and used for cellular transplantation. Confirmation of pig-pig exogenic dopamine neurons in the fetal VM from either NURR1, LMX1A, or PITX3 blastocysts will be a milestone that allows us to proceed with the generation of human-pig chimeras.

TALEN-knockout of LMX1A, PITX3, and NURR1 in pig fibroblasts. TALENs were developed to cleave in exons 1, 2 and 3 respectively for LMXA1, PITX3, and also NURR1, another gene that plays a major role in dopamine neuron development (see FIG. 17A, black triangle). TALENs were co-transfected with a homology dependent repair template designed to introduce a novel stop codon, HindIII site, and a frame-shift after the novel stop codon to ensure disruption of the targeted allele. Populations of transfected cells were analyzed for HindIII dependent cleavage produced by a PCR-restriction fragment polymorphism assay (FIG. 17B). The proportion of chromosomes with the novel HindIII-knockout allele (indicated by cleavage products, open triangles) is indicated on the gel. Individual clones were derived from the populations, and those verified as bi-allelic knockout by RFLP and sequencing were cryopreserved for complementation experiments.

### Example 10: Complementation of PITX3 knockout porcine blastocysts with human stem cells rescues ocular phenotype.

To determine if human stem cells are capable of complementing *PITX3* deficiency in the pig, hUCBSCs were injected into *PITX3* knockout porcine blastocysts and transferred blastocysts to hormonally synchronized gilts. Chimeric fetuses were harvested at 62 days in gestation and examined for the status of the eyelids (FIG. 18). A portion of the chimeric fetuses displayed open eyelids similar to wild-type pig fetuses while others exhibited closed eyelids. These results suggest that the *PITX3* knockout in porcine blastocysts is a suitable model for interrogating human stem cell contribution to ectodermal lineages.

### Example 11: ETV2 knockout pig embryos

Etv2 is a master regulatory gene for vascular and hematopoietic lineages, and is an ideal candidate for gene editing studies. The *Etv2* gene locus was mutated to generate vascular and hematopoietic deficient pig embryos for several reasons. First, the inventors have comprehensively demonstrated that *Etv2* is a master regulatory gene for vascular and hematopoietic development in mice (Ferdous 2009, Rasmussen 2011, Koyano-Nakagawa 2012, Rasmussen 2012, Chan 2013, Rasmussen 2013, Behrens 2014, Shi 2014). Using genetic lineage tracing strategies, the inventors demonstrated that Etv2 expressing cells give rise to vascular/endothelial and hematopoietic lineages (Rasmussen 2011, Koyano-Nakagawa 2012, Rasmussen 2012). Second, a global gene deletional strategy was undertaken and demonstrated that *Etv2* mutant mouse embryos were nonviable as they lacked vascular and hematopoietic lineages (Ferdous 2009, Koyano-Nakagawa 2012, Rasmussen 2012, Rasmussen 2013). Using transcriptome analysis, it was determined that *Tie2* was markedly dysregulated in the absence of Etv2 (Ferdous 2009, Koyano-Nakagawa 2012). Moreover, using transgenic technologies and molecular biological techniques (transcriptional assays, EMSA, ChIP and mutagenesis), it was verified that *Spil, Tie2* and *Lmo2* were direct downstream targets of Etv2 (Ferdous 2009, Koyano-Nakagawa 2012, Shi 2014). Third, forced overexpression of Etv2 in the differentiating ES/EB system significantly increased the populations of endothelial and hematopoietic lineages, demonstrating that Etv2 is a single factor that has the capacity to govern molecular cascades that will induce both lineages (Koyano-Nakagawa 2012).

### Example 12: ETV2 knockout pig embryos lack vascular and hematopoietic lineages.

Previous studies by the inventors have demonstrated that *Etv2* is essential for vasculogenesis and hematopoiesis in the mouse as embryos lacking *Etv2* are lethal at approximately E9.5 with an absence of vasculature and blood (Ferdous 2009, Rasmussen 2011, Koyano-Nakagawa 2012). It was hypothesized that ETV2 is the key regulator of the vasculature and blood in mammals, and thus, the ETV2 knockout in the pig will phenocopy the mouse. To examine the role of ETV2 in the pig, the inventors removed the entire *ETV2* coding sequence using two TALEN pairs flanking the gene in porcine fibroblasts (FIG. 19). The process was 15% efficient at complete gene removal; 79/528 of the genotyped clones were homozygous for the deletion of the *ETV2* gene. *ETV2* homozygous knockout fibroblast clones were used for nuclear cloning (Somatic Cell Nuclear Transfer; SCNT) to generate *ETV2* null embryos which were transferred to surrogate sows. The cloning efficiency was 29%, which was higher than the average success rate of 20%.

Embryos were harvested and analyzed at E18.0 (FIG. 20). At E18.0, wild-type (Wt) embryos were vascularized with a well-developed vascular plexus in the allantois (FIG. 20A) and had evidence of blood development (FIG. 20C). In contrast, *ETV2* KO embryos showed clear developmental defects. Growth was retarded in *ETV2* KOs relative to the Wt embryo, though both embryos were at the 24-somite stage (FIG 20B), and lacked both blood and vascular lineages (FIGs. 20C-H). *ETV2* KO embryos lacked cardinal veins, dorsal aortae, and the endocardium, that are clearly developed in the Wt embryos (FIG. 20E-20H). These results reflect a similar phenotype and suggest that the function of ETV2 is conserved between mice and pigs. Further, these data strongly support the hypothesis that multiple mutations can be directed into the porcine genome to support growth of chimeric organs that will be humanized in more than one cell type.

### Example 13: Complementation of ETV2 knockout porcine blastocysts with human iPSCs.

The inventors have further undertaken studies to determine whether hiPSCs are capable of complementing *ETV2* deficiency in the pig. hiPSCs were injected into *ETV2* knockout porcine blastocysts and transferred these blastocysts to hormonally synchronized gilts. Chimeric fetuses were harvested at 18 days of gestational age and immunohistochemically examined for the status of the hiPSCs (FIG. 21). Human cells were identified by genomic in situ hybridization using the probe to *Alu* repetitive sequence, as well as staining against human nuclear antigen (HNA). The presence of human cells were observed that expressed human CD31 and human vWF (vascular/endothelial marker) supporting the notion that the *ETV2* knockout in porcine blastocysts is an excellent model for interrogating human stem cell contributions to vascular and hematopoietic lineages.

### Example 14: Nkx2-5 and HandII as essential regulators of cardiogenesis.

Cardiac development is a complex highly-orchestrated event that includes the specification, proliferation, migration and differentiation of cardiac progenitors that become electrically coupled and ultimately form a functional syncytium. These stages of cardiogenesis are governed by transcriptional networks, which have been shown, using gene disruption technology, to be absolutely essential for heart formation and viability (Lyons 1995, Srivastava 1997, Tanaka 1999, Bruneau 2001, Yamagishi 2001, Garry 2006, Ferdous 2009, Caprioli 2011) (Table1). Nkx2-5 is the vertebrate homolog of the *Drosophila* homeodomain protein, Tinman (Csx). The Tinman mutation results in the absence of heart formation in the fly (Bodmer 1993). Nkx2-5 is one of the earliest transcription factors expressed in the cardiac lineage. Targeted disruption of *Nkx2-5* results in perturbed heart morphogenesis, severe growth retardation and embryonic lethality at approximately E9.5 (Lyons 1995, Tanaka 1999). HandII (dHand) is a bHLH transcription factor that has also been shown to be essential for cardiac morphogenesis. *HandII* mutant embryos are lethal during early embryogenesis and have severe right ventricular hypoplasia and aortic arch defects (Srivastava 1997). Moreover, mice lacking both *Nkx2-5 and HandII* demonstrate ventricular agenesis and have only a single atrial chamber (FIG. 22) (Yamagishi 2001). These gene disruption studies in the mouse model illustrate the effectiveness of using a gene editing strategy in the pig model.

### Example 15: Multiplex knockout of porcine NKX2-5 and HANDII genes.

A combination of TALEN stimulated HDR were used to generate *NKX2-5*/*HANDII* mutant porcine fibroblasts. Each gene was targeted either within or immediately prior to their conserved transcription factor/DNA binding domains (FIG. 23A). This strategy was favored over targeting the gene near the transcription start site to reduce the chance of producing a functional peptide by initiation at a downstream AUG. For *NKX2-5,* a homology template was provided to generate a novel in-frame stop codon, restriction site for RFLP screening, and an additional five base insertion after the stop codon to prevent a functional read-through protein. Double mutants were identified (FIG. 23B). The ability to reliably produce double null pig fibroblast cell lines in a single shot is unique and a transformative technology required for complementation.

### Example 16: Perturbed cardiogenesis in triple knockout pig embryos.

Preliminary studies have targeted a number of critical transcription factors (i.e. MESP1, GATA4, NKX2-5, HANDII, TBX5, etc.) that result in perturbed cardiogenesis and would provide important new models for the study and potential treatment of congenital heart disease in the pig. Here the inventors demonstrate, as proof-of-concept successful targeting and generation of clones homozygous for the deletion of *NKX2-5*/*HANDII*/*TBX5* genes. Triple knockout fibroblast clones were used for nuclear cloning (SCNT) to generate *NKX2-5*/*HANDII*/*TBX5* null porcine embryos, which were transferred to surrogate sows. Embryos were harvested and analyzed at E18, which is equivalent to E11 of the mouse. At E18, the triple knockout porcine embryos have vasculature, skeletal muscle and blood but essentially lack a heart (minimal GATA4 immunohisto-chemically positive cardiomyo-cytes) (FIG. 24) compared to the wildtype control porcine embryo. These data support the rationale and feasibility of utilizing *NKX2-5*/*HANDII* double knockout porcine model to limit the involvement of other lineages (i.e. neuronal lineage in the *TBX5* KO) and be more reflective of congenital heart disease models (i.e. hypoplastic right and left heart defects). This approach will result in the engineering of humanized biventricular hearts in the porcine model.

### Example 17: Myf5, Myod and Mrf4 as essential regulators of myogenesis.

The discovery of the Myod family including Myod, Myf5, Mrf4, and Myog, provided the fundamental platform for understanding the regulatory mechanisms of skeletal muscle myogenesis [5-7] (FIG. 25).

Multiple strategies have been employed to investigate the regulatory network of the Myod family during myogenesis, such as transcriptome analysis, promoter analysis and ChIP-seq [5-6,9]. Myod family members are master myogenic regulators as they transactivate a broad spectrum of gene families, including muscle specific genes, transcription factors, cell cycle genes, etc. to promote a myogenic cell fate [5-6,9-10]. Previous gene disruption studies have demonstrated that mice lacking Myf5/Myod/MRF4 lack skeletal muscle and are lethal early following birth presumably due to their inability for respiration (due to the absence of a diaphragm). These gene disruption studies in the mouse illustrate the effectiveness of using gene editing strategies in the pig.

Utilizing TALENs and homology-dependent repair (HDR) to knockout *MYOD, MYF5,* and *MRF4.* To examine the role of *MYF5*/*MYOD*/*MRF4* (aka MYF6) in the pig, disrupted each coding sequence using TALEN stimulated HDR (FIG. 26).

*MYF5*/*MYOD*/*MRF4* knockout pig embryos lack skeletal muscle lineages. Embryos were harvested and analyzed at E18.0 (FIG. 27). The results in the mouse and pig reflect a similar phenotype and support the notion that the function of *MYF5*/*MYOD*/*MRF4* are conserved between mice and pigs as mutant embryos lack skeletal muscle. Further, these data strongly support the hypothesis that direct multiple mutations into the porcine genome to support growth of chimeric organs that will be humanized in more than one cell type.

### Example 18: Complementation of MYF5/MYOD/MRF4 knockout phenotype with GFP WT pig blastomeres.

Porcine *MYF5*/*MYOD*/*MRF4* null blastocysts were generated using SCNT, and injected with GFP-labeled porcine blastomeres (since no validated porcine ES cells are available, blastomeres were utilized for this experiment). The resulting chimeras were implanted in pseudopregnant sows and examined at E20. *The feasibility of complementation was demonstrated as liver and yolk sac were GFP positive.* Additionally, the inventors estimate that approximately 10% of porcine *MYF5*/*MYOD*/*MRF4* null blastocysts were GFP labeled (FIG. 28). These data support pig;pig complementation in this porcine mutant host.

These data further support creating a triple knockout in the porcine model devoid of skeletal muscle that will ultimately create a niche for the formation of complemented tissues. This is used throughout these studies for creating humanized skeletal muscle in the pig.

### Example 19: PDX1 knockout results in apancreatic fetal pigs.

*Pdx1*^{*-*/*-*} mice are apancreatic and die shortly after birth due to the inability of the pancreatic bud to develop into the mature organ (Offield *et al.,* 1996). Rescue of the mouse Pdx1^{-/-} phenotype by blastocyst complementation has been demonstrated by injecting wild-type mouse or rat iPSCs into *Pdx1*^{*-*/*-*} mouse blastocysts, producing mice that had normal functioning pancreases, derived from the donor cells (Kobayashi *et al.,* 2010). Blastocyst complementation of Pdx1 deficiency was also recently described in the pig where a functional pancreas was produced in a trans-genic apancreatic pig following the injection of labeled WT blastomere cells into pig blastocysts expressing the dominant *Pdx1:hes1* transgene (Matsunaria *et al.,* 2013). Cloned Pdx1 knockout pigs are not susceptible to the unpredictable nature of position effects or expression levels seen when using transgenes and offer a more consistent platform for the production of pancreas ablated pigs. The inventors have used exclusive TALEN technology to biallelically knockout the *PDX1* gene in pig fibroblasts (FIG. 29A) using a TALEN pair that targets the essential homeobox domain of the *PDX1* gene, and an HDR construct to introduce a STOP codon, frameshift, and novel restriction enzyme site. Homozygous *PDX1* knockouts were obtained at a rate of 41% (76/184 clones) (FIG. 29B). The inventors have used these *PDX1-*/*-* fibroblasts and chromatin transfer cloning techniques to generate *PDX1-*/*-* blastocysts and demonstrated pancreas ablation in PDX1-/- pig embryos harvested at E30 (FIGs. 29C and 29D). Nascent β cells expressing Pdx1 and insulin are present in the pig pancreas in wild-type embryos harvested at E32 (FIG. 29E).

### Example 20: HHEX knockout results in loss of liver in fetal pigs

Generation of *HHEX* KO clones. In the initial studies, *HHEX* KO clones were generated to test the efficiency of this gene-editing method. Constructs were developed to cleave exon 2 of *HHEX* gene (see FIG. 30a black triangle) within the N-terminus of the homeo-domain-like region essential for DNA binding. Fibroblasts were transfected with vector constructs and a homology dependent repair template designed to introduce a novel stop codon, a HindIII site, and a frame-shift mutation after the novel stop codon to ensure disruption of the targeted allele. Over 50% the transfected population was positive for the HindIII KO allele by PCR-restriction fragment polymorphism assay (FIG. 30B) and several individual clones derived from the population were either heterozygous or homozygous for the KO allele (FIG. 30C). In total, 22 clones with sequence validated KO alleles were cryopreserved. The same vector constructs are used to generate both *HHEX* and *Ubc* KO blastocysts.

*HHEX* KO is embryonic lethal in pigs. To determine the effect of *HHEX* KO in pigs, *HHEX-1-* fibroblasts were cloned SCNT and transferred to a synchronized recipient. At 30-32 days in gestation the embryos were harvested and assessed for the development of the liver. All embryos were genotyped and confirmed for knockout of HHEX. All specimens exhibited delayed development with a clear absence of the liver (FIG. 31, right panel). Samples were taken from each specimen to grow fibroblasts as a source of *HHEX* knockout cells for future experiments to combine this knockout with editing of other targeted genes such as ETV2 to create human liver with human vasculature.

### Example 21: Summary of preliminary studies on porcine gene knockouts and incorporation of human stem cells in the fetal pig.

Preliminary studies demonstrated the ability of targeted gene knockouts in the pig to disrupt the development of the eye, heart, lung, liver, skeletal muscle, pancreas, vasculature, hematopoietic cells, and dopamine neurons. The inventors also demonstrated that human stem cells injected into the porcine morula/blastocysts can result in their integration within the inner cell mass and contribute to developing fetal pigs. Importantly, the inventors also observed the contribution of human stem cells in fetal pigs within the context of blastocyst complementation. These results provide strong evidence of the feasibility to engineer human organs and cells within swine.

### Example 22: MR imaging of fetal porcine organs at 16.4T.

The imaging of organs generated in the pig via blastocyst complementation will be facilitated using high field MRI. The 16.4T magnet at the UMN Center for Magnetic Resonance Research is currently the most powerful magnet in the world for imaging. FIG. 39 shows a fetal pig 30 days in gestation (20 mm crown-rump length) where all of the internal organs are quite visible in great detail. The pulse sequence used in this figure was optimized for visualizing the liver. Other pulse sequences will be developed to optimize contrast for other organs for quantitation of parameters such as organ volume in addition to 3D morphology to provide important information regarding the anatomical features of complemented organs. This will provide a rapid quantitative approach for determine the success of complementation following the knockout of target genes to generate specific organs.

### Example 23: Engineer Off-the-Shelf Platelet Cells for Hemostasis and Therapy

More than 1.5 million allogeneic platelet products are transfused in the USA each year to restore the hemostasis in thrombocytopenic patients. However, platelet transfusion can transmit infections, trigger serious immune reactions, and can be rendered ineffective by alloimmunisation. Indeed, the chronic shortage of donated platelets and the refractoriness to platelet transfusion due to antibody in recipients that are specific for HLA class I compromise patient safety. We will generate off-the-shelf HLA class I^{null} platelets from iPSC to eliminate β2-microglobulin (B2M) and undertake blastocyst complementation. This is the only method to generate sufficient numbers of platelets as *ex vivo* differentiation of genetically modified iPSC into megakaryocytes is inefficient and results in insufficient numbers of platelets. Indeed, the production of HLA^{null} platelets in the swine reprogrammed as a bioreactor will solve the current issues in platelet transfusions with major implications for the civilian population as well as warfighters. The reprogramming of iPSC will also be used to generate "weaponized" platelets that are derived from genetically modified megakaryocytes to express derivatives of CARs to bind to and target tumor cells (direct cell- kill) and tumor-associated vasculature (indirect cell- kill). The same technology to genetically insert and edit genes will also be used generate platelets to deliver drug to tumor cells and metabolize drug in the tumor microenvironment. The ability to generate large numbers of off-the-shelf and programmed platelets generates a new class of therapies with implications for improving human health in multiple disciplines.

*Knockout mice for evaluating the gene(s) to be eliminated for xenogeneic platelet production.* The inventors evaluate the platelet production in c-MPL, G6bB, and SHP1/SHP2 knockout mice, which have been shown to decrease in platelet number (Gurney et al., 1994; Mazharian et al., 2013; Mazhalian et al., 2012). *Generation of target gene knockout pig* - Next step will be to validate the candidate gene(s) knockout in the pig. According to the result from mice experiments, we will knockout target gene(s) from pig fibroblasts by TALEN. As described in other studies, edited pig fibroblasts will be then used to generate blastocysts via SCNT (Tan et al., 2013).

*Evaluation of genes related to rapid clearance of human platelet in pig liver* - One challenging area of producing human platelet in pig will be the rapid clearance of human platelet in pig liver. Previous papers suggested the role of ASGR1 (Paris et al.. 2011) or MAC-1α (Peng et al., 2012) expressed on pig liver sinusoidal endothelial cells on the clearance of platelets. The role of these genes will be evaluated in human platelet clearance by generating knockout pigs. Injection of human platelets into these pigs and evaluating the clearance of platelets will provide us the insight regarding those gene(s) that should be knocked out in addition to the genes necessary for platelet differentiation. *Gene editing and modification in iPS cells* -We will use TALEN targeting b-2 -microglobulion (B2M) to eliminate HLA class I expression from human iPS cells. To introduce specific expression cassettes into iPSC genomes and express therapeutic genes (scFvs,cytokines etc.) the *SB* system will be used, which the inventors have successfully adopted to express exogenous genes in iPSCs. For instance, scFv targeting VEGFR2 (Chinnasamy et al., 2010) to redirect platelet- binding to tumor vascular and expression of cytokines (Zhang et al., 2015) and/or enzymes that convert pro-drug (Chen et al., 2011) to active form that assist the destruction of tumor cells.

The following genes knockout blastocysts for complementation with B2M^{neg}iPSC to produce HLA^{null} platelets in the engineered pig. Platelets number obtained and function will be assessed *in vitro.* HLA^{null} platelets that express therapeutic genes (e.g., targeting scFvs, cytokines, and enzymes to metaboloize prodrug) will be evaluated *in vitro* and *in vivo* model to assess their potential for cancer therapy.

### Host genes edited will include:

c-MPL^{-/-}, G6bB^{-/-}, SHP1^{-/-}, HSP2^{-/-}; c-MPL^{-/-}/G6bB^{-/-}/SHP1^{-/-}/HSP2^{-/-}; c-MPL^{-/-}/G6bB^{-/-}/SHP1^{-/-}; c-MPL^{-/-}/G6bB^{-/-}/HSP2^{-/-}; c-MPL^{-/-}/SHP1^{-/-}/HSP2^{-/-}; G6bB^{-/-}/SHP1^{-/-}/HSP2^{-/-}; c-MPL^{-/-}/G6bB^{-/-}; c-MPL^{-/-}/SHP1^{-/-}; c-MPL^{-/-}/HSP2^{-/-}; G6bB^{-/-}/SHP1^{-/-}; G6bB^{-/-}/HSP2^{-/-}; c-MPL^{-/-}; G6bB^{-/-}; SHP1^{-/-}; HSP2^{-/-}.

Table E provides a list of the genotype of edited carriers (host), their genotype of the donor used to complement (rescue) the animal.

| TABLE E: Carrier and Host Genotype | | |
|---|---|---|
| HOST (Blastocyst, Embryo, Zygote, cell | DONOR (Blastocyst, Embryo, Zygote, cell) | FUNCTION |
| c-MPL^{-/-}, G6bB^{-/-}, SHP1^{-/-}, HSP2^{-/-} | HLA classI^{neg} | Platelet Production |
| c-MPL^{-/-}/G6bB^{-/-}/SHP1^{-/-} /HSP2^{-/-} | iPSC WT | |
| c-MPL^{-/-}/G6bB^{-/-}/SHP1^{-/-} | HLA | |
| c-MPL^{-/-}/G6bB^{-/-}/HSP2^{-/-} | classI^{neg} | |
| c-MPL^{-/-}/SHP1^{-/-}/HSP2^{-/-} | | |
| G6bB^{-/-}/SHP1^{-/-}/HSP2^{-/-} | | |
| c-MPL^{-/-}/G6bB^{-/-} | | |
| c-MPL^{-/-}/SHP1^{-/-} | | |
| c-MPL^{-/-}/HSP2^{-/-} | | |
| G6bB^{-/-}/SHP1^{-/-} | | |
| G6bB^{-/-}/HSP2^{-/-} | | |
| c-MPL^{-/-} | | |
| G6bB^{-/-} | | |
| SHP1^{-/-} | | |
| HSP2^{-/-} | | |

### Example 24: Engineer Off-the-Shelf CAR T Cells to Target Cancer

Immunotherapy has emerged as an effective approach to target cancer cells. Autologous T cells that have been genetically modified to express chimeric antigen receptor (CAR) have been shown to eradicate tumors refractory to chemotherapy. Despite this clinical success, broad application of CAR⁺ T-cell therapy is hampered by the current manufacturing process, wherein each product is infused for a single patient. CAR⁺ T-cell therapy administering allogeneic T cells that are generated in advance of need is the most promising approach to solve this problem. We have shown that CAR⁺ T cells genetically edited with artificial nuclease(s) to eliminate expression of endogenous T-cell receptor (TCR) exhibit redirected specificity for CD19 on malignant B cells and yet apparently do not participate in graft-versus-host disease. The inventors have shown that genetic elimination of HLA-A increase the chance for finding HLA-matched 3^{rd} party donors, which has advantage over administering mismatched HLA donors to achieve long-term survival of infused T cells in patients. This advanced genetic engineering (insertion of CAR using the *Sleeping Beauty* system and elimination of TCR and HLA-A using artificial nucleases) will be undertaken in iPSC as derived clones can be sequenced to validate safe harbor insertion of the CAR and on-target genetic editing. HLA-A^{neg}TCR^{neg}CAR⁺ iPSC (homozygous at HLA B and DR enabling matching with recipients) will be generated and subsequent blastocyst complementation will be used to generate T cells in swine. The *ex vivo* generation of T cells from engineered iPSC is currently not feasible, thus the generation of swine as bioreactors to produce off-the-shelf HLA-A^{neg}TCR^{neg}CAR⁺ T cells will broaden immunotherapy and represent a paradigm shift for the field as therapeutic T cells can be infused at the time of need, rather than when they are available.

Efficient genetic modification of iPSCs are key for generating CAR+ iPSC. To attain this objective, *Sleeping Beauty* (*SB*) transposons are used, which the inventors have successfully adopted in genetic modification of T cells and are tested in the clinical trial (Singh et al., 2014). A single SB plasmid that drives expression of both CD19 target CAR and iCasp 9 has been prepared. iCasp 9 has been successfully applied in the clinic as a suicide gene, which relies on dimerization of iCasp9 protein via a with chemical dimerizer (AP) to induce apoptosis of cells expressing iCasp 9 (Di Stasi et al., 2011). This is particularly important when genetically modified iPSC derived T cells cause unexpected adverse events (e.g. formation of tumor or uncontrolled proliferation). We will use this plasmid along with hyperactive *SB11* transposase from in vitro-transcribed mRNA to modify iPSCs. We have introduced plasmids and/or mRNA by electroporation into iPSCs. In this experiment, the inventors have demonstrated that the SB system can be successfully adopted to introduce CAR genes into iPSCs (FIG. 34). HLAAnegTCRnegCAR+ iPSC clones are isolated and cryopreserved for complementation experiment.

Genotypes of host and donor cells includes: HLA^{+/+}/TCR^{-/-}/HLA-A^{-/-}IL2Rγ^{-/-}/RAG1^{-/-} /RAG2^{-/-} (RAG1/2); IL2Rγ^{-/-}/RAG1^{-/-}/; IL2Rγ^{-/-}/RAG2^{-/-}. See, FIG 34.

Table F provides a list of the genotype of edited carriers (host), their genotype of the donor used to

| TABLE F: Carrier and Host Genotype | | |
|---|---|---|
| HOST (Blastocyst, Embryo, Zygote, cell | DONOR (Blastocyst, Embryo, Zygote, cell) | FUNCTION |
| HLA^{+/+}/TCR^{-/-}/HLA-A^{-/-} | CAR^{+/+} T; | TARGET CANCER |
| IL2Rγ^{-/-}/RAG1^{-/-}/RAG2^{-/-} (RAG1/2) | HLA^{+/+}/TCR^{-/-} /HLA-A^{-/-}, | |
| IL2Rγ^{-/-}/RAG1^{-/-}/ | | |
| *IL2Rγ^{-/-}/RAG2^{-/-} | | |

| | | |
|---|---|---|
| *Phenotype validated | | |

### FURTHER DISCLOSURE

Patents, patent applications, publications, and articles mentioned herein are hereby incorporated by reference; in the case of conflict, the instant specification is controlling. The embodiments have various features; these features may be mixed and matched as guided by the need to make a functional embodiment. The headings and subheadings are provided for convenience but are not substantive and do not limit the scope of what is described. The following numbered paragraphs 1-56 present embodiments of the invention wherein in a first paragraph:
1. A method of producing human and/or humanized T cells and/or platelets in a non-human animal comprising:
   i) disrupting one or more endogenous genes responsible for T cell and/or platelet growth and/or development in a host cell or embryo;
   ii) complementing the host's lost genetic information by introducing at least one human donor cell into the host to create a chimeric embryo;
   wherein the one or more human cells occupy a niche created by the disabled gene or genes upon development of the embryo;
   wherein the niche comprises a human or humanized cells, tissue or organ.
2. The method of paragraph 1, wherein, the host comprises a non-human embryo, zygote or blastocyst.
3. The method of any of paragraphs 1-2, wherein the donor is the recipient of the organ or tissue produced.
4. The method of any of paragraphs 1-3, wherein the donor is not the recipient.
5. The method of any of paragraphs 1-4, wherein the host is an atryodactyl.
6. The method of any of paragraphs 1-5, wherein the host is a pig, a cow or a goat.
7. The method of any of paragraphs 1-6, wherein disrupting is accomplished using targeted endonucleases.
8. The method of paragraph 7, wherein the targeted endonucleases comprise CRISPR/CAS, zinc finger nuclease, meganuclease, TALENs or combinations thereof.
9. The method of any of paragraphs 7-8, wherein of one or more of the endonucleases are provided as mRNAs and are introduced into the cell or embryo from a solution having a concentration from 0.1 ng/ml to 100 ng/ml; artisans will immediately appreciate that all values and ranges within the expressly stated limits are contemplated, e.g., about 20, from about 1 to about 20, from about 0.5 to about 50, and so forth; and/or
   of one or more (e.g., each of) of the HDR templates are provided as mRNAs and are introduced into the cell or embryo from a solution having a concentration from about 0.2 µM to about 20 µM.
10. The method of any of paragraphs 1-9, with the embryo being zygote, blastocyst, morula, or having a number of cells from 1-200.
11. The method of any of paragraphs 1-10, wherein the donor cells are embryonic stem cells, tissue-specific stem cells, mesenchymal stem cells, pluripotent stem cells, umbilical cord blood stem cells (hUCBSC) or induced pluripotent stem cells.
12. The method of any of paragraphs 1-11 wherein the host animal is heterozygous for one or more gene edits.
13. The method of any of paragraphs 1-11, wherein the host animal is homozygous for one or more gene edits.
14. The method of any of paragraphs 1-13, wherein the disrupted genes edits include: c-MPL, G6bB, SHP1, HSP2, HLA, TCR, HLA-A, IL2Rγ, RAG1, and/or RAG2
15. The method of any of paragraphs 1-14, wherein:
   when the one or more endogenous genes comprise c-MPL, G6bB, SHP1 and/or HSP2 than the tissue or organ comprises platelets;
   when the one or more endogenous genes comprise HLA, TCR, HLA-A, IL2Rγ, RAG1, and/or RAG2 than the tissue or organ comprises T-cells.
16. The method of any of paragraphs 1-15, wherein the T-cells are chimeric antigen receptor (CAR) T cells.
17. The method of any of paragraphs 1-16, further comprising introducing a homology directed repair (HDR) template having a template sequence with homology to one of the endogenous genes, with the template sequence replacing at least a portion of the endogenous gene sequence to disrupt the endogenous gene.
18. The method of any of paragraphs 1-17, further comprising introducing a plurality of homology directed repair (HDR) template, each having a template sequence with homology to one of the endogenous genes, with each the template sequences replacing at least a portion of one of the endogenous gene sequences to disrupt the endogenous gene.
19. The method of any of paragraphs 1-18, wherein the disruption comprises a substitution of one or more DNA residues of the endogenous gene.
20. The method of any of paragraphs 1-19, wherein the disruption consists of a substitution of one or more DNA residues of the endogenous gene.
21. The method of any of paragraphs 1-20, wherein the disruptions are gene knockouts.
22. An animal made by a method of any of paragraphs 1-21.
23. A non-human chimeric embryo or animal made by the method of any of paragraphs 1-22 further comprising cloning the host cell, making a host embryo from the cell, and adding a donor cell to the host embryo to form the chimeric embryo.
24. A non-human chimeric embryo having at least one human donor cell wherein the non-human embryo has one or more endogenous genes responsible for the development of one or more tissues or organs disrupted;
   wherein the at least one human donor cells develop into tissues or organs for which the disrupted genes were responsible;
   wherein:
   when the one or more endogenous genes comprise c-MPL, G6bB, SHP1 and/or HSP2 than the tissue or organ comprises platelets;
   when the one or more endogenous genes comprise HLA, TCR, HLA-A, IL2Ry, RAG1, and/or RAG2 than the tissue or organ comprises T-cells.
25. An animal grown from the chimeric embryo of paragraph 24.
26. The chimeric embryo of paragraph 24, wherein the developed tissues or organs are human or humanized.
27. A non-human embryo host comprising an embryo with a plurality of genetic edits disrupting one or more genes, providing a niche for complementation by donor cells.
28. The non-human embryo host of paragraph 27, wherein the plurality of gene edits are made using gene editing technology without the use of maker genes or selection markers.
29. The non-human embryo host of any of paragraphs 27-28, wherein the gene editing technology comprises CRISPR/CAS, zinc finger nuclease, meganuclease, TALENs or combinations thereof.
30. The non-human embryo host of any of paragraphs 27-29, wherein the plurality of genetic edits is made simultaneously.
31. The non-human embryo host of any of paragraphs 27-30, wherein the donor cells are embryonic stem cells, tissue-specific stem cells, mesenchymal stem cells, pluripotent stem cells or induced pluripotent stem cells.
32. The non-human embryo host of any of paragraphs 27-31, wherein the donor cells come from the recipient of an organ or tissue derived from the donor cells.
33. The non-human embryo host according to any of paragraphs 27-32, wherein the disruption comprises a gene edit, a knockout, an insertion of one or more DNA residues, a deletion of one or more bases, or both an insertion and a deletion of one or more DNA residues.
34. The non-human embryo host according to any of paragraphs 27-32, wherein the disruption comprises a substitution of one or more DNA residues.
35. The non-human embryo host of any of paragraphs 27-34 wherein the disruption consists of a substitution of one or more DNA residues.
36. A non-human chimeric animal comprising one or more endogenous edited genes, and human donor cells integrated with the host cells to form the chimeric animal; wherein the human cells comprise a tissue or organ occupying a niche created by the endogenous edited genes.
37. A non-human chimeric embryo comprising a non-human embryo having at least one human cell, wherein one or more endogenous genes of the non-human embryo responsible for the development of one or more endogenous organs or tissues have been disrupted and wherein the one or more human cells complement the function of the one or more disrupted genes providing one or more human or humanized tissues or organs wherein the chimeric embryo develops into an animal wherein
   when the one or more endogenous genes comprise c-MPL, G6bB, SHP1 and/or HSP2 than the tissue or organ comprises platelets;
   when the one or more endogenous genes comprise HLA, TCR, HLA-A, IL2Rγ, RAG1, and/or RAG2 than the tissue or organ comprises thymus cells or T-cells.
38. The non-human chimeric embryo of paragraph 37, wherein the T cells are selected from: Effector T cells, Helper T cells, Cytotoxic T cells, Memory T cells, Regulatory T cells, Natural killer T cells, mucosal T cells or Gamma delta T cells.
39. The non-human chimeric embryo of any of paragraphs 37-38, wherein the T cells are chimeric antigen receptor (CAR) T cells.
40. The non-human chimeric embryo of any of paragraphs 37-39, wherein the embryo is heterozygous for the disrupted genes.
41. The non-human chimeric embryo of any of paragraphs 37-40, wherein the embryo is homozygous for the disrupted gene.
42. The non-human chimeric embryo according to any of paragraphs 37-41, wherein the disruption comprises a gene edit, a knockout, an insertion of one or more DNA residues, a deletion of one or more bases, or both an insertion and a deletion of one or more DNA residues.
43. The non-human chimeric embryo according to any of paragraphs 37-42, wherein the disruption comprises a substitution of one or more DNA residues.
44. The non-human chimeric embryo of any of paragraphs 37-43 wherein the disruption consists of a substitution of one or more DNA residues.
45. A non-human chimeric animal developed from any of paragraphs 37-44.
46. Cells, tissues or organs developed from the chimeric embryo of paragraphs 37-45.
47. A method of making a chimeric, non-human embryo comprising:
   disrupting one or more endogenous genes of a non-human host embryo:
   introducing a human cell into the host embryo
   wherein the one or more disrupted genes are responsible for the development of one or more tissues or organs; and
   wherein the human cell complements the host embryo for the disrupted genes.
48. The method of paragraph 47, further comprising developing the embryo into a chimeric animal.
49. The method of any of paragraphs 47-48, wherein the complementation results in the human cell differentiating into the tissues or organs for which the disrupted genes were responsible.
50. The method of any of paragraphs 47-49, wherein:
   when the one or more disrupted genes comprise c-MPL, G6bB, SHP1 and/or HSP2 than the tissue or organ comprises platelets;
   when the one or more disrupted genes comprise HLA, TCR, HLA-A, IL2Rγ, RAG1, and/or RAG2 than the tissue or organ comprises T-cells.
51. The method of any of paragraphs 47-50, wherein the T cells are selected from: Effector T cells, Helper T cells, Cytotoxic T cells, Memory T cells, Regulatory T cells, Natural killer T cells, mucosal T cells or Gamma delta T cells.
52. The method of any of paragraphs 47-51, wherein the T cells are chimeric antigen receptor (CAR) T cells
53. The method of any of paragraphs 47-52, wherein the disruption comprises a gene edit, a knockout, an insertion of one or more DNA residues, a deletion of one or more bases, or both an insertion and a deletion of one or more DNA residues.
54. The method according to any of paragraphs 47-53 wherein the disruption comprises a substitution of one or more DNA residues.
56. The method of any of paragraphs 47-54wherein the disruption consists of a substitution of one or more DNA residues.

While this invention has been described in conjunction with the various exemplary embodiments outlined above, various alternatives, modifications, variations, improvements, and/or substantial equivalents, whether known or that are or may be presently unforeseen, may become apparent to those having at least ordinary skill in the art. Accordingly, the exemplary embodiments according to this invention, as set forth above, are intended to be illustrative, not limiting. Various changes may be made without departing from the spirit and scope of the invention. Therefore, the invention is intended to embrace all known or later-developed alternatives, modifications, variations, improvements, and/or substantial equivalents of these exemplary embodiments.

### NUMBERED SENTENCES

The following numbered sentences form part of the description and not the claims and are not intended to define the scope of the claimed subject matter.
1. A method of producing human and/or humanized T cells and/or platelets in a non-human animal comprising:
   i) disrupting one or more endogenous genes responsible for T cell and/or platelet growth and/or development in a host cell or embryo;
   ii) complementing the host's lost genetic information by introducing at least one human donor cell into the host to create a chimeric embryo;
   wherein the one or more human cells occupy a niche created by the disabled gene or genes upon development of the embryo;
   wherein the disrupted genes edits include: c-MPL, G6bB, SHP1, HSP2, HLA, TCR, HLA-A, IL2Rγ, RAG1, and/or RAG2
   wherein the niche comprises a human or humanized T cells and/or platelets.
2. The method of sentence 1, wherein, the host comprises a non-human embryo, zygote or blastocyst.
3. The method of any of sentences 1-2, wherein the donor is the recipient of the organ or tissue produced.
4. The method of any of sentences 1-3, wherein the donor is not the recipient.
5. The method of any of sentences 1-4, wherein the host is an atryodactyl.
6. The method of any of sentences 1-5, wherein the host is a pig, a cow or a goat.
7. The method of any of sentences 1-6, wherein disrupting is accomplished using targeted endonucleases.
8. The method of sentence 7, wherein the targeted endonucleases comprise CRISPR/CAS, zinc finger nuclease, meganuclease, TALENs or combinations thereof.
9. The method of any of sentences 7-8, wherein of one or more of the endonucleases are provided as mRNAs and are introduced into the cell or embryo from a solution having a concentration from 0.1 ng/ml to 100 ng/ml; artisans will immediately appreciate that all values and ranges within the expressly stated limits are contemplated, e.g., about 20, from about 1 to about 20, from about 0.5 to about 50, and so forth; and/or
   of one or more (e.g., each of) of the HDR templates are provided as mRNAs and are introduced into the cell or embryo from a solution having a concentration from about 0.2 µM to about 20 µM.
10. The method of any of sentences 1-9, with the embryo being zygote, blastocyst, morula, or having a number of cells from 1-200.
11. The method of any of sentences 1-10, wherein the donor cells are embryonic stem cells, tissue-specific stem cells, mesenchymal stem cells, pluripotent stem cells, umbilical cord blood stem cells (hUCBSC) or induced pluripotent stem cells.
12. The method of any of sentences 1-11 wherein the host animal is heterozygous for one or more gene edits.
13. The method of any of sentences 1-11, wherein the host animal is homozygous for one or more gene edits.
14. The method of any of sentences 1-13, wherein the disrupted genes edits include one or more of: c-MPL, G6bB, SHP1, HSP2, HLA, TCR, HLA-A, IL2Rγ, RAG1, and/or RAG2
15. The method of any of sentences 1-14, wherein:
   when the one or more endogenous genes comprise c-MPL, G6bB, SHP1 and/or HSP2 than the tissue or organ comprises platelets;
   when the one or more endogenous genes comprise HLA, TCR, HLA-A, IL2Ry, RAG1, and/or RAG2 than the tissue or organ comprises T-cells.
16. The method of any of sentences 1-15, wherein the T-cells are chimeric antigen receptor (CAR) T cells.
17. The method of any of sentences 1-16, further comprising introducing a homology directed repair (HDR) template having a template sequence with homology to one of the endogenous genes, with the template sequence replacing at least a portion of the endogenous gene sequence to disrupt the endogenous gene.
18. The method of any of sentences 1-17, further comprising introducing a plurality of homology directed repair (HDR) template, each having a template sequence with homology to one of the endogenous genes, with each the template sequences replacing at least a portion of one of the endogenous gene sequences to disrupt the endogenous gene.
19. The method of any of sentences 1-18, wherein the disruption comprises a substitution of one or more DNA residues of the endogenous gene.
20. The method of any of sentences 1-19, wherein the disruption consists of a substitution of one or more DNA residues of the endogenous gene.
21. The method of any of sentences 1-20, wherein the disruptions are gene knockouts.
22. An animal made by a method of any of sentences 1-21.
23. A non-human chimeric embryo or animal made by the method of any of sentences 1-22 further comprising cloning the host cell, making a host embryo from the cell, and adding a donor cell to the host embryo to form the chimeric embryo.
24. A non-human chimeric embryo having at least one human donor cell wherein the non-human embryo has one or more endogenous genes responsible for the development of one or more tissues or organs disrupted;
   wherein the at least one human donor cells develop into tissues or organs for which the disrupted genes were responsible;
   wherein:
   when the one or more endogenous genes comprise c-MPL, G6bB, SHP1 and/or HSP2 than the tissue or organ comprises platelets;
   when the one or more endogenous genes comprise HLA, TCR, HLA-A, IL2Ry, RAG1, and/or RAG2 than the tissue or organ comprises T-cells.
25. An animal grown from the chimeric embryo of sentence 24.
26. The chimeric embryo of sentence 24, wherein the developed tissues or organs are human or humanized.
27. A non-human chimeric embryo comprising a non-human embryo having at least one human cell, wherein one or more endogenous genes of the non-human embryo responsible for the development of one or more endogenous organs or tissues have been disrupted and wherein the one or more human cells complement the function of the one or more disrupted genes providing one or more human or humanized tissues or organs wherein the chimeric embryo develops into an animal wherein
   when the one or more endogenous genes comprise c-MPL, G6bB, SHP1 and/or HSP2 than the tissue or organ comprises platelets;
   when the one or more endogenous genes comprise HLA, TCR, HLA-A, IL2Ry, RAG1, and/or RAG2 than the tissue or organ comprises thymus cells or T-cells.
28. The non-human chimeric embryo of sentence 27, wherein the T cells are selected from: Effector T cells, Helper T cells, Cytotoxic T cells, Memory T cells, Regulatory T cells, Natural killer T cells, mucosal T cells or Gamma delta T cells.
29. The non-human chimeric embryo of any of sentences 27-28, wherein the T cells are chimeric antigen receptor (CAR) T cells.
30. The non-human chimeric embryo of any of sentences 27-29, wherein the embryo is heterozygous for the disrupted genes.
31. The non-human chimeric embryo of any of sentences 27-30, wherein the embryo is homozygous for the disrupted gene.
32. The non-human chimeric embryo according to any of sentences 27-31, wherein the disruption comprises a gene edit, a knockout, an insertion of one or more DNA residues, a deletion of one or more bases, or both an insertion and a deletion of one or more DNA residues.
33. The non-human chimeric embryo according to any of sentences 27-32, wherein the disruption comprises a substitution of one or more DNA residues.
34. The non-human chimeric embryo of any of sentences 27-33 wherein the disruption consists of a substitution of one or more DNA residues.
35. A non-human chimeric animal developed from any of sentences 27-34.
36. Cells, tissues or organs developed from the chimeric embryo of sentences 27-35.
37. A method of making a chimeric, non-human embryo comprising:
   disrupting one or more endogenous genes of a non-human host embryo:
   introducing a human cell into the host embryo
   wherein the one or more disrupted genes are responsible for the development of one or more tissues or organs; and
   wherein the human cell complements the host embryo for the disrupted genes;
   wherein:
      when the one or more disrupted genes comprise c-MPL, G6bB, SHP1 and/or HSP2 than the tissue or organ comprises platelets;
      when the one or more disrupted genes comprise HLA, TCR, HLA-A, IL2Ry, RAG1, and/or RAG2 than the tissue or organ comprises T-cells.
38. The method of sentence 37, further comprising developing the embryo into a chimeric animal.
39. The method of any of sentences 37-38, wherein the complementation results in the human cell differentiating into the tissues or organs for which the disrupted genes were responsible.
40. The method of any of sentences 37-39, wherein the T cells are selected from: Effector T cells, Helper T cells, Cytotoxic T cells, Memory T cells, Regulatory T cells, Natural killer T cells, mucosal T cells or Gamma delta T cells.
41. The method of any of sentences 37-40, wherein the T cells are chimeric antigen receptor (CAR) T cells.
42. The method of any of sentences 37-41, wherein the disruption comprises a gene edit, a knockout, an insertion of one or more DNA residues, a deletion of one or more bases, or both an insertion and a deletion of one or more DNA residues.
43. The method according to any of sentences 37-42 wherein the disruption comprises a substitution of one or more DNA residues.
44. The method of any of sentences 37-43 wherein the disruption consists of a substitution of one or more DNA residues.

## Claims

1. A method of producing human or humanized T cells in a non-human animal comprising:
i) disrupting one or more endogenous genes responsible for T cell growth or development in a host cell or embryo;
ii) complementing the host's lost genetic information by introducing at least one human donor cell into the host to create a chimeric embryo;
wherein the one or more human cells occupy a niche created by the disrupted gene or genes upon development of the embryo;
wherein the disrupted endogenous genes include one or more of: HLA, TCR, HLA-A, IL2Ry, RAG1, or RAG2
wherein the niche comprises a human or humanized T cells.

2. The method of claim 1, wherein:
i) the host comprises a non-human embryo, zygote or blastocyst; or
ii) the host is an artiodactyl, a pig, a cow or a goat
optionally wherein the host animal is:
i) heterozygous for one or more gene edits; or
ii) homozygous for one or more gene edits.

3. The method of either of claims 1-2, wherein the donor is the recipient of the organ or tissue produced or wherein the donor is not the recipient.

4. The method of any of claims 1-3, wherein disrupting is accomplished using targeted endonucleases, optionally wherein the targeted endonucleases comprise CRISPR/CAS, zinc finger nuclease, meganuclease, TALENs or combinations thereof,
preferably wherein:
i) one or more of the endonucleases are provided as mRNAs and are introduced into the cell or embryo from a solution having a concentration from 0.1 ng/ml to 100 ng/ml; or
ii) one or more of the homology directed repair (HDR) templates are provided as mRNAs and are introduced into the cell or embryo from a solution having a concentration from about 0.2 µM to about 20 µM.

5. The method of any of claims 1-4, with the embryo being zygote, blastocyst, morula, or having a number of cells from 1-200, or wherein the donor cells are embryonic stem cells, tissue-specific stem cells, mesenchymal stem cells, pluripotent stem cells, umbilical cord blood stem cells (hUCBSC) or induced pluripotent stem cells.

6. The method of any of claims 1-5, wherein the T-cells are chimeric antigen receptor (CAR) T cells.

7. The method of any of claims 1-6, further comprising:
i) introducing a homology directed repair (HDR) template having a template sequence with homology to one of the endogenous genes, with the template sequence replacing at least a portion of the endogenous gene sequence to disrupt the endogenous gene; or
ii) introducing a plurality of homology directed repair (HDR) templates, each having a template sequence with homology to one of the endogenous genes, with each the template sequences replacing at least a portion of one of the endogenous gene sequences to disrupt the endogenous gene.

8. A non-human chimeric embryo or animal made by a method as claimed in any of claims 1-7, wherein the method further comprises cloning the host cell, making a host embryo from the cell, and adding a donor cell to the host embryo to form the chimeric embryo.

9. A non-human chimeric embryo having at least one human donor cell wherein the non-human embryo has one or more endogenous genes responsible for the development of one or more tissues or organs disrupted;
wherein the at least one human donor cells develop into tissues or organs for which the disrupted genes were responsible;
wherein the one or more endogenous genes comprise HLA, TCR, HLA-A, IL2Ry, RAG1, or RAG2, and the tissue or organ comprises T-cells,
optionally wherein the developed tissues or organs are human or humanized.

10. A non-human chimeric embryo comprising a non-human embryo having at least one human cell,
wherein one or more endogenous genes of the non-human embryo responsible for the development of one or more endogenous organs or tissues have been disrupted, and
wherein the one or more human cells complement the function of the one or more disrupted genes providing one or more human or humanized tissues or organs,
wherein the chimeric embryo develops into an animal wherein the one or more endogenous genes comprise HLA, TCR, HLA-A, IL2Ry, RAG1, or RAG2, and the tissue or organ comprises thymus cells or T-cells.

11. A non-human chimeric animal made by a method of any of claims 1-7 or developed from the non-human chimeric embryo of claims 9 or 10.

12. Cells, tissues or organs developed from the chimeric embryo of claims 10 or 11.

13. A method of making a chimeric, non-human embryo comprising:
disrupting one or more endogenous genes of a non-human host embryo:
introducing a human cell into the host embryo
wherein the one or more disrupted genes are responsible for the development of one or more tissues or organs; and
wherein the human cell complements the host embryo for the disrupted genes;
wherein the one or more disrupted genes comprise HLA, TCR, HLA-A, IL2Ry, RAG1, or RAG2, and the tissue or organ comprises T-cells,
optionally further comprising developing the embryo into a chimeric animal, optionally wherein the complementation results in the human cell differentiating into the tissues or organs for which the disrupted genes were responsible.

14. The non-human chimeric embryo of claim 10, or the method of claim 13, wherein the T cells are selected from: Effector T cells, Helper T cells, Cytotoxic T cells, Memory T cells, Regulatory T cells, Natural killer T cells, mucosal T cells, Gamma delta T cells, or chimeric antigen receptor (CAR) T cells

15. The non-human chimeric embryo of claim 10, or the method of claim 13 or 14, wherein the disruption comprises a gene edit, a knockout, an insertion of one or more DNA residues, a deletion of one or more bases, or both an insertion and a deletion of one or more DNA residues, optionally, wherein the disruption comprises or consists of a substitution of one or more DNA residues of the endogenous gene.
